# EUROPEAN PATENT APPLICATION

(11) **EP 1 905 826 A1**
(43) Date of publication of application: **02.04.2008**
(21) Application number: 06450140.6
(22) Date of filing: 29.09.2006
(51) Int. Cl.: C12N 9/10, C12N 15/82

(54) **Galactosyltransferase**

(71) Applicant: Universität für Bodenkultur Wien, 1180 Wien (AT)
(72) Inventor: Strasser, Richard, 1170 Wien (AT); Steinkellner, Herta, 1190 Wien (AT); Mach, Lukas, 2103 Langenzersdorf (AT); Glössl, Josef, 1140 Wien (AT); Altmann, Friedrich, 1140 Wien (AT); Bondili, Jayakumar Singh, Islampet, Vijayawada - 520001 (IN)
(74) Representative: Sonn & Partner Patentanwälte

(57) **Abstract**

The present invention provides a method of expressing a plant Lewis-type β1,3-galactosyltransferase in an organism comprising the step of providing the organism with a nucleic acid molecule comprising a sequence A which is defined as being selected from
a) a sequence according to SEQ ID NO: 1 with an open reading frame from base pair 1-1932,
j) a sequence which is at least 50% identical with SEQ ID NO: 1,
k) a sequence which hybridizes with SEQ ID NO: 1 under stringent conditions, or
l) a sequence which has degenerated to SEQ ID NO: 1 due to the genetic code,
wherein the sequences a) to d) encode a plant protein having Lewis-type β1,3-galactosyltransferase activity, or
m) a sequence which is complementary to one of the sequences a) to d),

and expressing a protein encoded by sequence A or if the organism comprises a sequence A in its wildtype form overexpressing a protein encoded by sequence A.

SEQUENCE LISTING

<110> Universität für Bodenkultur Wien

<120> Galactosyltransferase

<130> r48115

<140> EP06450140.6
<141> 2006-09-29

<160> 66

<170> PatentIn version 3.3

<210> 1
<211> 1932
<212> DNA
<213> Arabidopsis thaliana

<400> 1
atgaagagat tttatggagg gcttcttgtt gtatcaatgt gtatgttctt gacggtgtat 60

agatatgtag acttgaacac tcctgttgaa aagccttata ttactgctgc tgcttctgtt 120

gttgttactc ctaacaccac tcttcctatg gaatggctgc ggattactct ccctgacttt 180

atgaaagaag cgaggaatac tcaagaagcg atatctggtg atgatatcgc tgttgtctcg 240

ggtttgtttg ttgagcagaa tgtgtctaaa gaagagagag agcctttgct tacttggaac 300

cgtttggaaa gtcttgttga taatgcacag agtttagtta atggagttga tgctattaag 360

gaagctggca ttgtttggga gagtcttgtg tctgctgttg aagctaagaa actagttgat 420

gttaatgaaa atcagacgag gaaaggaaaa gaggagcttt gtcctcagtt tctaagcaaa 480

atgaatgcta ctgaagctga tgggagtagt ctgaagttgc aaattccttg tggtttgact 540

cagggttcct ccatcacagt tattggcatc ccagatggtc ttgttggtag ttttcggatt 600

gatctaacgg gacaaccgct tccaggggag cctgatccac ccatcattgt gcattataat 660

gttaggcttc ttggtgacaa atcgacggaa gaccctgtga ttgttcaaaa cagctggacg 720

gcatctcagg actggggagc tgaggaacgc tgtccaaaat ttgatcctga tatgaataag 780

aaagtggatg acttggatga atgcaacaag atggttggtg gagaaattaa ccgaacttct 840

tcaactagct tgcagtccaa cacttcaagg ggagttccag tagccaggga agcatctaaa 900

catgaaaaat actttccttt caagcagggt ttcttatcgg ttgctacact tagggtggga 960

acagagggaa tgcagatgac agttgatggg aaacatataa cttcatttgc tttccgcgat 1020

acactggaac cgtggcttgt tagtgaaata cggattacag gtgactttag gttaatatcc 1080

attcttgcca gcggtttgcc cacatcagaa gaatcagagc acgttgttga tctagaggca 1140

ctaaaatcac ctaccctttc accattaagg ccactggatc tcgttattgg tgttttctcc 1200

actgcaaaca attttaaaag acggatggct gtgaggagaa catggatgca gtatgatgat 1260

gtaagatctg gaagagttgc agtacgcttt tttgttggcc ttcacaaaag tcctcttgtt 1320

aacttggaac tctggaacga ggctcggact tacggtgatg ttcagctaat gccctttgtt 1380

gattattaca gtctcatcag ttggaaaaca ctagccatct gcatcttcgg gacagaggtt 1440

gactcagcca agttcatcat gaaaacggat gatgacgcct ttgttcgtgt agatgaagtg 1500

ttactttctt tatcaatgac caacaacact cgcgggttaa tatacggact gatcaattcc 1560

gactctcaac ctattcgaaa ccctgatagc aaatggtaca tcagttatga ggaatggcct 1620

gaagagaaat atccaccatg ggcgcatggt ccaggctaca ttgtatctcg tgacatagca 1680

gaatcggttg gtaagctttt caaagaagga aacctaaaga tgtttaagct agaagatgtg 1740

gcaatgggga tatggatagc tgagctgaca aaacatggac tcgagcctca ttacgaaaac 1800

gatggaagga tcattagtga tggatgcaag gatggttatg tggttgctca ttaccaaagc 1860

cctgccgaaa tgacttgcct ttggcgtaaa taccaagaaa ccaaacgctc tctttgctgc 1920

cgcgaatggt aa 1932

<210> 2
<211> 643
<212> PRT
<213> Arabidopsis thaliana

<400> 2

Met Lys Arg Phe Tyr Gly Gly Leu Leu Val Val Ser Met Cys Met Phe
1               5                   10                  15

Leu Thr Val Tyr Arg Tyr Val Asp Leu Asn Thr Pro Val Glu Lys Pro
            20                  25                  30

Tyr Ile Thr Ala Ala Ala Ser Val Val Val Thr Pro Asn Thr Thr Leu
        35                  40                  45

Pro Met Glu Trp Leu Arg Ile Thr Leu Pro Asp Phe Met Lys Glu Ala
    50                  55                  60

Arg Asn Thr Gln Glu Ala Ile Ser Gly Asp Asp Ile Ala Val Val Ser
65                  70                  75                  80

Gly Leu Phe Val Glu Gln Asn Val Ser Lys Glu Glu Arg Glu Pro Leu
                85                  90                  95

Leu Thr Trp Asn Arg Leu Glu Ser Leu Val Asp Asn Ala Gln Ser Leu
            100                 105                 110

Val Asn Gly Val Asp Ala Ile Lys Glu Ala Gly Ile Val Trp Glu Ser
        115                 120                 125

Leu Val Ser Ala Val Glu Ala Lys Lys Leu Val Asp Val Asn Glu Asn
    130                 135                 140

Gln Thr Arg Lys Gly Lys Glu Glu Leu Cys Pro Gln Phe Leu Ser Lys
145                 150                 155                 160

Met Asn Ala Thr Glu Ala Asp Gly Ser Ser Leu Lys Leu Gln Ile Pro
                165                 170                 175

Cys Gly Leu Thr Gln Gly Ser Ser Ile Thr Val Ile Gly Ile Pro Asp

            180                 185                 190

Gly Leu Val Gly Ser Phe Arg Ile Asp Leu Thr Gly Gln Pro Leu Pro
        195                 200                 205

Gly Glu Pro Asp Pro Pro Ile Ile Val His Tyr Asn Val Arg Leu Leu
    210                 215                 220

Gly Asp Lys Ser Thr Glu Asp Pro Val Ile Val Gln Asn Ser Trp Thr
225                 230                 235                 240

Ala Ser Gln Asp Trp Gly Ala Glu Glu Arg Cys Pro Lys Phe Asp Pro
                245                 250                 255

Asp Met Asn Lys Lys Val Asp Asp Leu Asp Glu Cys Asn Lys Met Val
            260                 265                 270

Gly Gly Glu Ile Asn Arg Thr Ser Ser Thr Ser Leu Gln Ser Asn Thr
        275                 280                 285

Ser Arg Gly Val Pro Val Ala Arg Glu Ala Ser Lys His Glu Lys Tyr
    290                 295                 300

Phe Pro Phe Lys Gln Gly Phe Leu Ser Val Ala Thr Leu Arg Val Gly
305                 310                 315                 320

Thr Glu Gly Met Gln Met Thr Val Asp Gly Lys His Ile Thr Ser Phe
                325                 330                 335

Ala Phe Arg Asp Thr Leu Glu Pro Trp Leu Val Ser Glu Ile Arg Ile
            340                 345                 350

Thr Gly Asp Phe Arg Leu Ile Ser Ile Leu Ala Ser Gly Leu Pro Thr
        355                 360                 365

Ser Glu Glu Ser Glu His Val Val Asp Leu Glu Ala Leu Lys Ser Pro
    370                 375                 380

Thr Leu Ser Pro Leu Arg Pro Leu Asp Leu Val Ile Gly Val Phe Ser
385                 390                 395                 400

Thr Ala Asn Asn Phe Lys Arg Arg Met Ala Val Arg Arg Thr Trp Met
                405                 410                 415

Gln Tyr Asp Asp Val Arg Ser Gly Arg Val Ala Val Arg Phe Phe Val
            420                 425                 430

Gly Leu His Lys Ser Pro Leu Val Asn Leu Glu Leu Trp Asn Glu Ala
        435                 440                 445

Arg Thr Tyr Gly Asp Val Gln Leu Met Pro Phe Val Asp Tyr Tyr Ser
    450                 455                 460

Leu Ile Ser Trp Lys Thr Leu Ala Ile Cys Ile Phe Gly Thr Glu Val
465                 470                 475                 480

Asp Ser Ala Lys Phe Ile Met Lys Thr Asp Asp Asp Ala Phe Val Arg
                485                 490                 495

Val Asp Glu Val Leu Leu Ser Leu Ser Met Thr Asn Asn Thr Arg Gly
            500                 505                 510

Leu Ile Tyr Gly Leu Ile Asn Ser Asp Ser Gln Pro Ile Arg Asn Pro
        515                 520                 525

Asp Ser Lys Trp Tyr Ile Ser Tyr Glu Glu Trp Pro Glu Glu Lys Tyr
    530                 535                 540

Pro Pro Trp Ala His Gly Pro Gly Tyr Ile Val Ser Arg Asp Ile Ala
545                 550                 555                 560

Glu Ser Val Gly Lys Leu Phe Lys Glu Gly Asn Leu Lys Met Phe Lys
                565                 570                 575

Leu Glu Asp Val Ala Met Gly Ile Trp Ile Ala Glu Leu Thr Lys His
            580                 585                 590

Gly Leu Glu Pro His Tyr Glu Asn Asp Gly Arg Ile Ile Ser Asp Gly
        595                 600                 605

Cys Lys Asp Gly Tyr Val Val Ala His Tyr Gln Ser Pro Ala Glu Met
    610                 615                 620

Thr Cys Leu Trp Arg Lys Tyr Gln Glu Thr Lys Arg Ser Leu Cys Cys
625                 630                 635                 640

Arg Glu Trp

<210>  3
<211>  2467
<212>  DNA
<213>  Arabidopsis thaliana

<400>  3
atgaagagat tttatggagg gcttcttgtt gtatcaatgt gtatgttctt gacggtgtat      60

agatatgtag acttgaacac tcctgttgaa aagccttata ttactgctgc tgcttctgtt     120

gttgttactc ctaacaccac tcttcctatg gaatggctgc ggattactct ccctgacttt     180

atgaaagaag cgaggaatac tcaagaagcg atatctggtg atgatatcgc tgttgtctcg     240

ggtttgtttg ttgagcagaa tgtgtctaaa gaagagagag agcctttgct tacttggaac     300

cgtttggaaa gtcttgttga taatgcacag agtttagtta atggagttga tgctattaag     360

gaagctggca ttgtttggga gagtcttgtg tctgctgttg aagctaagaa actagttgat     420

gttaatgaaa atcagacgag gaaaggaaaa gaggagcttt gtcctcagtt tctaagcaaa     480

atgaatgcta ctgaagctga tgggagtagt ctgaagttgc aaattccttg tggtttgact 540
cagggttcct ccatcacagt tattggcatc ccagatggtc ttgttggtag ttttcggatt 600
gatctaacgg gacaaccgct tccaggggag cctgatccac ccatcattgt gcattataat 660
gttaggcttc ttggtgacaa atcgacggaa gaccctgtga ttgttcaaaa cagctggacg 720
gcatctcagg actggggagc tgaggaacgc tgtccaaaat ttgatcctga tatgaataag 780
aaaggtgaat gcaagtatag tttcacccgc ttacataaat gtagtcactt gatcctccat 840
gaataagtct gtgttgacat gattataata cacagtggat gacttggatg aatgcaacaa 900
gatggttggt ggagaaatta accgaacttc ttcaactagc ttgcagtcca acacttcaag 960
gggagttcca gtagccaggg aagcatctaa acatgaaaaa tactttcctt tcaagcaggg 1020
tttcttatcg gttgctacac ttagggtggg aacagaggga atgcagatga cagttgatgg 1080
gaaacatata acttcatttg ctttccgcga tgtaatatca tcactcttct tatatatttt 1140
tctactgctc ttagcaattt tccatcaaat gtttagtgtc tcatatgggt tcttcgtctt 1200
tcagacactg gaaccgtggc ttgttagtga aatacggatt acaggtgact ttaggttaat 1260
atccattctt gccagcggtt tgcccacatc agaagaatca gagcacgttg ttgatctaga 1320
ggcactaaaa tcacctaccc tttcaccatt aaggccactg gatctcgtta ttggtgtttt 1380
ctccactgca aacaatttta aaagacggat ggctgtgagg agaacatgga tgcagtatga 1440
tgatgtaaga tctggaagag ttgcagtacg ctttttgtt ggccttgtaa gctttctccc 1500
tctctgaaaa tatcaggagt atgttggaat tgcataattc taaatgatga ttttctttgt 1560
ggttggtaat gcagcacaaa agtcctcttg ttaacttgga actctggaac gaggctcgga 1620
cttacggtga tgttcagcta atgccctttg ttgattatta cagtctcatc agttggaaaa 1680
cactagccat ctgcatcttc ggggtaataa tatagttgtc tttgtcatgt ttcttcctag 1740
ccacagttga gtagtcttta ctgttttgcc tcactaacgt catattttaa acagacagag 1800
gttgactcag ccaagttcat catgaaaacg gatgatgacg cctttgttcg tgtagatgaa 1860
gtgttacttt ctttatcaat gaccaacaac actcgcgggt taatatacgg actgatcaat 1920
tccgactctc aacctattcg aaaccctgat agcaaatggt acatcagtta tgaggtacaa 1980
cacaatctga catagctatg tttgcaaata gactgatttc tgattcattt aacgaagttt 2040
ctactcaaaa aacaggaatg gcctgaagag aaatatccac catgggcgca tggtccaggc 2100
tacattgtat ctcgtgacat agcagaatcg gttggtaagc ttttcaaaga aggaaaccta 2160
aaggtattgt actactctat gctaactcca ttcaatcaca tttccggatt cacatagaaa 2220
tggattgtga aaatttttga aatttgtgaa tagatgttta agctagaaga tgtggcaatg 2280
gggatatgga tagctgagct gacaaaacat ggactcgagc ctcattacga aaacgatgga 2340
aggatcatta gtgatggatg caaggatggt tatgtggttg ctcattacca aagccctgcc 2400
gaaatgactt gcctttggcg taaataccaa gaaaccaaac gctctctttg ctgccgcgaa 2460
tggtaaa 2467

<210>  4
<211>  192
<212>  PRT
<213>  Arabidopsis thaliana

<400>  4

Lys Arg Arg Met Ala Val Arg Arg Thr Trp Met Gln Tyr Asp Asp Val
1               5                   10                  15

Arg Ser Gly Arg Val Ala Val Arg Phe Phe Val Gly Leu His Lys Ser
            20                  25                  30

Pro Leu Val Asn Leu Glu Leu Trp Asn Glu Ala Arg Thr Tyr Gly Asp
        35                  40                  45

Val Gln Leu Met Pro Phe Val Asp Tyr Tyr Ser Leu Ile Ser Trp Lys
    50                  55                  60

Thr Leu Ala Ile Cys Ile Phe Gly Thr Glu Val Asp Ser Ala Lys Phe
65                  70                  75                  80

Ile Met Lys Thr Asp Asp Asp Ala Phe Val Arg Val Asp Glu Val Leu
                85                  90                  95

Leu Ser Leu Ser Met Thr Asn Asn Thr Arg Gly Leu Ile Tyr Gly Leu
            100                 105                 110

Ile Asn Ser Asp Ser Gln Pro Ile Arg Asn Pro Asp Ser Lys Trp Tyr
        115                 120                 125

Ile Ser Tyr Glu Glu Trp Pro Glu Glu Lys Tyr Pro Pro Trp Ala His
    130                 135                 140

Gly Pro Gly Tyr Ile Val Ser Arg Asp Ile Ala Glu Ser Val Gly Lys
145                 150                 155                 160

Leu Phe Lys Glu Gly Asn Leu Lys Met Phe Lys Leu Glu Asp Val Ala
                165                 170                 175

Met Gly Ile Trp Ile Ala Glu Leu Thr Lys His Gly Leu Glu Pro His
            180                 185                 190

<210>  5
<211>  34
<212>  DNA
<213>  Artificial

<220>
<223>  primer

<400>  5
tttaggtacc atgaagagat tttatggagg gctt                                34

<210>  6
<211>  28
<212>  DNA

<213>  Artificial

<220>
<223>  primer

<400>  6
tataggatcc ttaccattcg cggcagca                                          28


<210>  7
<211>  287
<212>  DNA
<213>  Arabidopsis thaliana

<400>  7
accttcgctt tctctgctgg tggaaggtgc ttcgatacta cgacaaagag cagcgaacta      60

gtaaagcctc ggagatctga atcccatttc aatcagcaaa tctttccgtc tctacttctc     120

tgtttgccgg tgtaccggat tctgattccg gaacagacat tgcttaattt tgcttccctt     180

tggcctggat tgattgaagt taaaaaatag ctacattgta ctgtaagatt gaatattgat     240

gatctcgccg cgcgatttct cgtttgagtg attttcggct gtgaaaa                   287


<210>  8
<211>  219
<212>  DNA
<213>  Arabidopsis thaliana

<400>  8
aacgtgaacg cacatatttc aaccattgcg ttttggttca cgtgtccttc cacaggtaca      60

taagaaaaga gttcattctt tggtcacaaa atatttgatt ttgtttgttt gttgtacaag     120

tagactagat ttgcagttta gttttacttt tacgtgtgta tattacaaag atgtactaaa     180

actttcaact gataaataac aataggagca tattttatc                            219


<210>  9
<211>  2480
<212>  DNA
<213>  Arabidopsis thaliana

<400>  9
atctattgaa ggatccaatt tccttgcatc gaaatactgc gggcacaaca tgcatcattg      60

gcctagctta ttagatccat tacatattgt actattcatt ttttttcccc ggacatctta     120

ggttaaagat agcatcattt ttctaaagta actgacatta gcaacaccat gaattttgcg     180

tgaagaaaaa aaatattgta gggccaaatc tttatcattt tctatagtat gaagattcca     240

agactgaggt tctcgcccgc acgagaaggg cgagcttgag gtgtttatct gcaaggagat     300

gggtgacgac gggtctaaag ttgggtgggt ttgtagatag aaggctgagg aagcttcggt     360

aaccaccgct ccatggagac gggtcattaa aaccatgttt tgattactgt taaaagccat     420

gttttgatta agcacaatgc atttcatgca taagcggttg aacatttttt ttttactctt     480

tagcaatatc gtgagccatt tgtttttgtt ataacatgta cataagagaa tgatgttgag     540

ctatcaaaaa tcagtaaaat atagactcaa cgatatatag gataaagatg aggtaaaggt     600

tgatgaagaa gaggataatg atcggttggt gaggaagagg ataattaggt tgatcaggta     660

gaggatgatg atcattgatc acaatggatc gttggtaata gattgccctt cattaacaga     720

tgtggactta gttgacgttt gggaagacta ttgcttgata gagaatatgt cttgccttga 780
tgtggcagac actggctttg ttcctaatcc cgataccaag tttcttagat ctctttcttc 840
tgatgtatgg atttgcgctt gaccaaatca atggtagcca ttttatatat ctctctctct 900
ctcgtattcc ttgttgtttc ttcaactaat cttcgttttc ctctatcata agtcgcgtgt 960
tgtagcgcca ttaacttctc tctgctcata acgttttatt ttcaaacaca aaattccgta 1020
gactggttag aaccacttat gtttttgctt caaaattctc taaaactgaa aactcttatg 1080
atcaacgaaa acacggtaac aagcgcatcc atctaatttt gatgaactca acattataat 1140
aaactgtact gataccagga cttcgagggt ctccaacctt cctggaacca gtcgagttct 1200
attcccacat gcctgtcaac tcatctagag atctttggtt ggaaagacaa tggaagaaga 1260
gaagatgaga aacaatttag tgacatacat tctcgcaaac tcaaagtgtt taaagacagt 1320
ggaaatctcc ctcatagcag ccttcaatct taaggagagg caaaaggagt tagttaccat 1380
gcctaggatt tcaacattat ctaggcttct attccccatc caaatggaat gggagttcaa 1440
tgaaacaact ttttcattat atatattgtc atatacagta tatgctataa ctactcaagc 1500
tgtcgtttac agattgcaat ttttttgcct tattaactcg aaaactctaa ttcagccaac 1560
tttttactag tcgagatgaa ttttttttta agcatatacg tacaacaagg ctagattttt 1620
tcatcatgag taagtttaac aaagactgtg tgcttgcaaa gtcatcatga gtaggttgtt 1680
ttgagtctct tatgtgaatt aaacaaaaca aaaaaaacaa cgtgaacatt tcagtccaat 1740
tttgtttgtc tccctacgat tcaaccagca aaagaaaaaa gcacataaaa gcaaaaaatg 1800
ggaatcaata tattgagacg ttaattatga ctggcatgtg tatgtatatt gtatggtggt 1860
gcgttacaaa tgaaagcacc ctcatatctc tcgctatcac gacttgggaa cttgttttta 1920
ccccttcaag tttcgtttgc caacttttac ttactgtttt ttttttacca gttactttat 1980
tttacttttt gcaatcaaat gtaactttgt ttatttaaaa atgatgtttc tagagggtca 2040
tcaaaaatat cagtgattag gcataattgt caaatcttga taattcaaag atctcgttta 2100
tatgtgttta gtaatttagt catagactca accaaaaaac tttagtgatc aagtgatact 2160
tttttttttt ttttatcttc ttccaaagca tgcaaacaga ggaaatcaaa atataaatgt 2220
aatcttttaa aactacggtt gtaattaaaa ttactttttt tttttttttt gtcgtaaatt 2280
actaaattag tattgaatta agttagaaac tatttctgtg tattttctta gagtaccttg 2340
tgtataattt tgtatgaaaa tagctaaaga actatcggta acttttcttc actttacagt 2400
tttgtttttt acctctacag tttttacaat tttcgttctt gttttacagt taaaaaaaaa 2460
tagaaagaaa catgaaacca 2480

<210> 10
<211> 507
<212> PRT
<213> Arabidopsis thaliana

<400> 10

Gln Lys Gln Gly His Gly Asn Asp Ser Ser Arg Ala Lys Glu Lys Gln

1               5                   10                  15

Cys Pro His Phe Leu Asn Asn Met Asn Ser Thr Glu Leu Gly Asn Ser
            20                  25                  30

Ser Tyr Lys Leu Gln Leu Pro Cys Gly Leu Thr Gln Gly Ser Ser Ile
        35                  40                  45

Thr Ile Ile Gly Ile Pro Asn Gly Leu Leu Gly Asn Phe Arg Ile Asp
    50                  55                  60

Leu Thr Gly Glu Pro Leu Pro Gly Glu Pro Asp Pro Pro Ile Val Leu
65                  70                  75                  80

His Tyr Asn Val Arg Leu His Gly Asp Lys Ile Thr Glu Asp Pro Val
                85                  90                  95

Ile Val Gln Asn Ser Trp Thr Gln Ala His Asp Trp Gly Glu Glu Asp
            100                 105                 110

Arg Cys Pro Ser Pro Thr Pro Glu Lys Phe Asp Lys Val Asp Asp Leu
        115                 120                 125

Glu Gln Cys Asn Lys Ile Val Gly Lys Asn Ile Ser Gln Arg His Pro
    130                 135                 140

Ala Gly Met His Ser His Thr Ser Arg Gln Ser Ser Thr Met Asp Glu
145                 150                 155                 160

Gln Ser Val Asn Arg Lys Tyr Phe Pro Phe Lys Gln Gly Tyr Pro Phe
                165                 170                 175

Val Ala Thr Leu Arg Val Gly Ser Glu Gly Ile Gln Met Thr Val Asp
            180                 185                 190

Gly Lys His Ile Thr Ser Phe Ala Phe Arg Glu Thr Leu Glu Pro Trp
        195                 200                 205

Leu Val Ser Glu Ile Lys Ile Ser Gly Asp Leu Lys Leu Ile Ser Ile
    210                 215                 220

Leu Ala Ser Gly Leu Pro Thr Ser Glu Asp Ser Glu His Ile Ile Asp
225                 230                 235                 240

Leu Glu Ser Leu Lys Ser Ser Pro Ile Ser Ala Gln Thr Leu Leu Tyr
                245                 250                 255

Leu Phe Ile Gly Val Phe Ser Thr Ala Asn Asn Phe Lys Arg Arg Met
            260                 265                 270

Ala Val Arg Arg Thr Trp Met Gln Tyr Asn Ala Val Arg Ser Asn Thr
        275                 280                 285

Thr Ala Val Arg Phe Phe Val Gly Leu His Lys Ser Thr Val Val Asn
    290                 295                 300

Glu Glu Leu Trp Arg Glu Ala Arg Thr Tyr Gly Asp Val Gln Leu Met
305                 310                 315                 320

Pro Phe Val Asp Tyr Tyr Ser Leu Ile Thr Trp Lys Ser Leu Ala Ile
                325                 330                 335

Cys Ile Phe Gly Thr Gln Val Ser Ala Lys Phe Val Met Lys Thr Asp
            340                 345                 350

Asp Asp Ala Phe Val Arg Val Asp Glu Val Leu Asp Ser Leu His Arg
        355                 360                 365

Ile Asn Ala Asp His Gly Leu Leu Tyr Gly Leu Ile Asn Leu Asp Ser
    370                 375                 380

Arg Pro His Arg Asn Thr Asp Ser Lys Trp Tyr Ile Ser Pro Glu Glu
385                 390                 395                 400

Trp Ser Glu Gly Thr Tyr Pro Pro Trp Ala His Gly Pro Gly Tyr Val
                405                 410                 415

Val Ser His Asp Ile Ala Arg Thr Val Ser Lys Lys Phe Arg Glu Asn
            420                 425                 430

His Leu Lys Met Phe Lys Leu Glu Asp Val Ala Met Gly Ile Trp Ile
        435                 440                 445

Ala Asp Met Lys Lys Glu Gly Leu Glu Val Arg Tyr Glu Asn Glu Val
    450                 455                 460

Arg Val Tyr Pro Glu Gly Cys Lys Asp Gly Tyr Val Val Ala His Tyr
465                 470                 475                 480

Gln Gly Pro Arg Glu Met Leu Cys Leu Trp Gln Lys Leu Gln Val Asp
                485                 490                 495

Lys Arg Ala Lys Cys Cys Gly Asp Ser Arg Lys
            500                 505

<210>  11
<211>  31
<212>  DNA
<213>  Artificial

<220>
<223>  primer

<400>  11
tatactgcag aagccttata ttactgctgc t                                    31

<210>  12

<211>  33
<212>  DNA
<213>  Artificial

<220>
<223>  primer

<400>  12
taaaggtacc ttaccattcg cggcagcaaa gag                                    33


<210>  13
<211>  23
<212>  DNA
<213>  Artificial

<220>
<223>  primer

<400>  13
aaggccactg gatctcgtta ttg                                               23


<210>  14
<211>  25
<212>  DNA
<213>  Artificial

<220>
<223>  primer

<400>  14
ttaccaaccg attctgctat gtcac                                             25


<210>  15
<211>  23
<212>  DNA
<213>  Artificial

<220>
<223>  primer

<400>  15
ggtgctcgga attttcgtct aca                                               23


<210>  16
<211>  24
<212>  DNA
<213>  Artificial

<220>
<223>  primer

<400>  16
tcattctctt tgcaactggc tcta                                              24


<210>  17
<211>  22
<212>  DNA
<213>  Artificial

<220>
<223>  primer

<400>  17
attgccgtag atgaaagact ga                                                22

<210>  18
<211>  31
<212>  DNA
<213>  Artificial

<220>
<223>  primer

<400>  18
tttatctaga tggcttcaaa ggtctcctgc c                                    31


<210>  19
<211>  31
<212>  DNA
<213>  Artificial

<220>
<223>  primer

<400>  19
ttctggatcc taacatctca gatgcttctt g                                    31


<210>  20
<211>  30
<212>  DNA
<213>  Artificial

<220>
<223>  primer

<400>  20
atcagggatc cactgcacgg tatgctcctc                                      30


<210>  21
<211>  30
<212>  DNA
<213>  Artificial

<220>
<223>  primer

<400>  21
atcgtggtac ctagctgcgt ctgcaaaaag                                      30


<210>  22
<211>  34
<212>  DNA
<213>  Artificial

<220>
<223>  primer

<400>  22
tatatctaga aaagtcctct tgttaacttg gaac                                 34


<210>  23
<211>  32
<212>  DNA
<213>  Artificial

<220>
<223>  primer

<400>  23

tataagatct taaacatctt taggtttcct tc                                    32

<210>  24
<211>  33
<212>  DNA
<213>  Artificial

<220>
<223>  primer

<400>  24
tataggtacc ttaaacatct ttaggtttcc ttc                                   33

<210>  25
<211>  41
<212>  DNA
<213>  Artificial

<220>
<223>  primer

<400>  25
tatagaattc ggatccaaaa gtcctcttgt taacttggaa c                          41

<210>  26
<211>  422
<212>  DNA
<213>  Arabidopsis thaliana

<400>  26
aaaagtcctc ttgttaactt ggaactctgg aacgaggctc ggacttacgg tgatgttcag      60

ctaatgccct ttgttgatta ttacagtctc atcagttgga aaacactagc catctgcatc     120

ttcgggacag aggttgactc agccaagttc atcatgaaaa cggatgatga cgcctttgtt     180

cgtgtagatg aagtgttact ttctttatca atgaccaaca acactcgcgg gttaatatac     240

ggactgatca attccgactc tcaacctatt cgaaaccctg atagcaaatg gtacatcagt     300

tatgaggaat ggcctgaaga gaaatatcca ccatgggcgc atggtccagg ctacattgta     360

tctcgtgaca tagcagaatc ggttggtaag cttttcaaag aaggaaacct aaagatgttt     420

aa                                                                    422

<210>  27
<211>  422
<212>  DNA
<213>  Arabidopsis thaliana

<400>  27
ttaaacatct ttaggtttcc ttctttgaaa agcttaccaa ccgattctgc tatgtcacga      60

gatacaatgt agcctggacc atgcgcccat ggtggatatt tctcttcagg ccattcctca     120

taactgatgt accatttgct atcagggttt cgaataggtt gagagtcgga attgatcagt     180

ccgtatatta acccgcgagt gttgttggtc attgataaag aaagtaacac ttcatctaca     240

cgaacaaagg cgtcatcatc cgttttcatg atgaacttgg ctgagtcaac ctctgtcccg     300

aagatgcaga tggctagtgt tttccaactg atgagactgt aataatcaac aaagggcatt     360

agctgaacat caccgtaagt ccgagcctcg ttccagagtt ccaagttaac aagaggactt     420

tt 422

<210> 28
<211> 557
<212> PRT
<213> Arabidopsis thaliana

<400> 28

Val Ser Asp Leu Phe Ile Lys Arg Asn Leu Ser Ala Gln Glu Gln Gln
1               5                   10                  15

Ser Leu Ser Thr Trp Tyr Gln Leu Lys Arg Leu Val Thr His Asp Gln
            20                  25                  30

Val Leu Pro Asn Ala Ile Glu Ala Val Lys Glu Ala Ser Val Ala Trp
        35                  40                  45

Asn Asn Leu Met Ser Ala Val Glu Arg Glu Lys Leu Asp Ala Asn Asp
    50                  55                  60

Ser Ser Val Lys Thr Gly Lys Gln Lys Gln Cys Pro His Phe Leu Ser
65                  70                  75                  80

Lys Thr Asn Ala Thr Glu Ile Asp Ala Ser Gly Phe Lys Leu Arg Phe
                85                  90                  95

Pro Cys Gly Leu Thr Gln Gly Ser Ser Ile Thr Ile Ile Gly Ile Pro
            100                 105                 110

Asn Gly Leu Leu Gly Asn Phe Arg Ile Asp Leu Thr Gly Glu Pro Leu
        115                 120                 125

Pro Gly Glu Pro Asp Pro Pro Val Ile Leu His Tyr Asn Val Arg Leu
    130                 135                 140

His Gly Asp Lys Ile Thr Glu Asp Pro Val Ile Val Gln Asn Thr Trp
145                 150                 155                 160

Thr Ile Ala His Asp Trp Gly Glu Glu Glu Arg Cys Pro Leu Pro Ser
                165                 170                 175

Asp Glu Lys Ser Lys Lys Val Asp Glu Leu Glu Gln Cys Asn Glu Met
            180                 185                 190

Val Gly Asn Val Ile Thr Arg His Val Ile Ala Thr Asn Lys Ser Ser
        195                 200                 205

Leu Ala Gln Asp Gly Ser Lys Ser Arg Lys Tyr Phe Pro Phe Lys Gln
    210                 215                 220

Gly Tyr Leu Ser Val Ala Thr Leu Arg Val Gly Ser Glu Gly Ile Gln
225                 230                 235                 240

Met Ala Val Asp Gly Lys His Ile Thr Ser Phe Ala Phe Arg Glu Thr
                245                 250                 255

Leu Glu Pro Trp Leu Val Ser Glu Val Arg Ile Ser Gly Asp Ile Lys
            260                 265                 270

Leu Ile Ser Val Val Ala Ser Gly Leu Pro Thr Ser Glu Asp Ser Glu
        275                 280                 285

His Ile Ser Asp Leu Glu Ala Leu Lys Ala Val Pro Leu Pro Pro Arg
    290                 295                 300

Lys Arg Leu Asp Leu Phe Val Gly Val Phe Ser Thr Ala Asn Asn Phe
305                 310                 315                 320

Lys Arg Arg Met Ala Val Arg Arg Thr Trp Met Gln Tyr Asp Ala Val
                325                 330                 335

Arg Ser Gly Gln Val Ala Val Arg Phe Phe Val Gly Leu His Lys Asn
            340                 345                 350

Gln Met Val Asn Glu Glu Leu Trp Asn Glu Ala Arg Thr Tyr Met Asp
        355                 360                 365

Ile Gln Leu Met Pro Phe Val Asp Tyr Tyr Ser Leu Ile Ala Trp Lys
    370                 375                 380

Thr Ile Ala Ile Cys Val Phe Gly Thr Glu Val Ile Ser Ala Lys Phe
385                 390                 395                 400

Val Met Lys Thr Asp Asp Asp Ala Phe Val Arg Val Asp Glu Ile Leu
                405                 410                 415

Ser Ser Met Gln Arg Ile Asn Val Thr Arg Gly Leu Leu Tyr Gly Leu
            420                 425                 430

Ile Asn Ser Asp Ser His Pro His Arg Ser Pro Asp Ser Lys Trp Phe
        435                 440                 445

Ile Ser Pro Glu Glu Trp Pro Glu Glu Thr Tyr Pro Pro Trp Ala His
    450                 455                 460

Gly Pro Gly Tyr Val Val Ser Ser Asp Ile Ala Lys Thr Ile Ser Ser
465                 470                 475                 480

Lys Gln Arg Lys Gly Arg Leu Lys Met Phe Lys Leu Glu Asp Val Ala
                485                 490                 495

Met Gly Ile Trp Ile Ser Glu Ile Lys Lys Lys Gly Leu Glu Val Lys
            500                 505                 510

Tyr Glu Lys Glu Glu Arg Ile Phe Asn Glu Gly Cys Arg Asp Gly Tyr
        515                 520                 525

Val Ile Ala His Tyr Gln Gly Pro Arg Glu Met Leu Cys Leu Trp Gln
530                 535                 540

Lys Ile Glu Glu Lys Lys Arg Ala Leu Cys Cys Gly Glu
545                 550                 555

<210>  29
<211>  548
<212>  PRT
<213>  Arabidopsis thaliana

<400>  29

Ser Ala Gln Glu Gln Gln Ser Leu Ser Thr Trp Tyr Gln Leu Lys Arg
1               5                   10                  15

Leu Val Thr His Asp Arg Val Leu Pro Asn Ala Ile Glu Ala Val Lys
            20                  25                  30

Glu Ala Ser Ile Ala Trp Asn Asn Leu Met Ser Ala Val Glu Arg Glu
        35                  40                  45

Lys Leu Asp Ala Asn Asp Thr Ser Val Lys Thr Gly Lys Gln Lys Gln
    50                  55                  60

Cys Pro His Phe Leu Ser Lys Thr Asn Ala Thr Glu Ile Asp Ala Ser
65                  70                  75                  80

Gly Phe Lys Leu Arg Phe Pro Cys Gly Leu Thr Gln Gly Ser Ser Ile
                85                  90                  95

Thr Ile Ile Gly Ile Pro Asn Gly Leu Leu Gly Asn Phe Arg Ile Asp
            100                 105                 110

Leu Thr Gly Glu Pro Leu Pro Gly Glu Pro Asp Pro Pro Val Ile Leu
        115                 120                 125

His Tyr Asn Val Arg Leu His Gly Asp Lys Ile Thr Glu Asp Pro Val
    130                 135                 140

Ile Val Gln Asn Thr Trp Thr Ile Ala His Asp Trp Gly Glu Glu Glu
145                 150                 155                 160

Arg Cys Pro Leu Pro Ser Asp Glu Lys Ser Lys Lys Val Asp Glu Leu
                165                 170                 175

Glu Gln Cys Asn Glu Met Val Gly Asn Ile Met Ala Thr Arg His Val
            180                 185                 190

Ile Ala Thr Asn Lys Ser Ser Leu Ala Gln Asp Arg Ser Lys Ser Arg
        195                 200                 205

Lys Tyr Phe Pro Phe Lys Gln Gly Tyr Leu Ser Val Ala Thr Leu Arg

210                 215                 220

Val Gly Ser Glu Gly Ile Gln Met Thr Val Asp Gly Lys His Ile Thr
225                 230                 235                 240

Ser Phe Ser Phe Arg Glu Thr Leu Glu Pro Trp Leu Val Ser Glu Val
                245                 250                 255

Arg Ile Ser Gly Asp Ile Lys Leu Ile Ser Val Val Ala Ser Gly Leu
            260                 265                 270

Pro Thr Ser Glu Asp Ser Glu His Ile Ser Asp Leu Glu Ala Leu Lys
        275                 280                 285

Ala Ala Pro Leu Pro Pro Arg Lys Arg Leu Asp Leu Phe Val Gly Val
    290                 295                 300

Phe Ser Thr Ala Asn Asn Phe Lys Arg Arg Met Ala Val Arg Arg Thr
305                 310                 315                 320

Trp Met Gln Tyr Asp Ala Val Arg Ser Gly Gln Val Ala Val Arg Phe
                325                 330                 335

Phe Val Gly Leu His Lys Asn Gln Met Val Asn Glu Glu Leu Trp Asn
            340                 345                 350

Glu Ala Arg Thr Tyr Met Asp Ile Gln Leu Met Pro Phe Val Asp Tyr
        355                 360                 365

Tyr Ser Leu Ile Ala Trp Lys Thr Ile Ala Ile Cys Val Phe Gly Thr
    370                 375                 380

Glu Val Val Ser Ala Lys Phe Val Met Lys Thr Asp Asp Asp Ala Phe
385                 390                 395                 400

Val Arg Val Asp Glu Ile Leu Ser Ser Met Gln Arg Ile Asn Val Thr
                405                 410                 415

Arg Gly Leu Leu Tyr Gly Leu Ile Asn Ser Asp Ser His Pro His Arg
            420                 425                 430

Ser Pro Asp Ser Lys Trp Phe Ile Ser Pro Glu Glu Trp Pro Glu Glu
        435                 440                 445

Thr Tyr Pro Pro Trp Ala His Gly Pro Gly Tyr Val Val Ser Ser Asp
    450                 455                 460

Ile Ala Lys Gln Ser Ala Gln Asn Arg Lys Lys Gly Arg Leu Lys Met
465                 470                 475                 480

Phe Lys Leu Glu Asp Val Ala Met Gly Ile Trp Ile Ser Glu Met Asn
                485                 490                 495

Lys Lys Gly Leu Glu Val Lys Tyr Glu Lys Glu Glu Arg Ile Phe Asn
            500                 505                 510

Glu Gly Cys Arg Asp Gly Tyr Val Ile Ala His Tyr Gln Gly Pro Arg
        515                 520                 525

Glu Met Leu Cys Leu Trp Gln Lys Ile Glu Glu Lys Lys Arg Ala Leu
    530                 535                 540

Cys Trp Gly Glu
545

<210>  30
<211>  637
<212>  PRT
<213>  Arabidopsis thaliana

<400>  30

Met Lys Lys Trp His Gly Gly Phe Val Ile Val Ser Leu Leu Ile Ile
1               5                   10                  15

Leu Met Leu Arg Tyr Val Ile Leu Asp Arg Leu Leu Ala Glu Lys Ser
            20                  25                  30

Leu Gln Tyr Val Phe Gln Gln Asn Arg Thr Ala Glu Leu His Trp Leu
        35                  40                  45

Asp Val Pro Asn Pro Pro Ala Ile Gln Asn Pro Gln Ser Ser Ser Glu
    50                  55                  60

Val Ile Ser Thr Arg Leu Leu Ala Ser Asn Leu Ser Ile Thr Arg Asn
65                  70                  75                  80

Leu Ser Asp Thr Glu Leu Gln Ser Leu His Ser Trp Asn His Leu Lys
                85                  90                  95

His Leu Leu Thr His Ala His Ile Leu Pro Asp Gly Val Glu Ala Ile
            100                 105                 110

Lys Glu Ala Gly Val Ala Trp Arg Glu Leu Asn Thr Ala Leu Ala Tyr
        115                 120                 125

Asp Asp Ser Val Val Ser Val Asn Gly Ser Ile Gln Gln Lys Asp Lys
    130                 135                 140

Gly Lys Gln Cys Pro Tyr Ser Val Arg Arg Met Asn Ala Thr Arg Leu
145                 150                 155                 160

Gly Asp Arg Phe Ala Leu Lys Leu Pro Cys Gly Leu Ile Gln Gly Ser
                165                 170                 175

Ser Ile Thr Ile Ile Gly Thr Pro Gly Gly Leu Leu Ala Asn Phe Lys
            180                 185                 190

Ile Glu Leu Thr Gly Ala Ala Val Pro Gly Glu Pro Asp Pro Pro Ile
        195                 200                 205

Val Leu His Tyr Asn Val Arg Leu Leu Gly Asp Lys Leu Thr Glu Asp
    210                 215                 220

Pro Val Ile Val Gln Asn Thr Trp Thr Ile Ala Asp Asp Trp Gly Ser
225                 230                 235                 240

Glu Asn Arg Cys Pro Ser Pro Asp Ser Asp Ala Lys Asp Ser Ala Lys
                245                 250                 255

Val Asp Asp Leu Glu Lys Cys Ser Ser Met Val Gly Glu Asp Gln Lys
            260                 265                 270

Lys Ile Leu Pro Ser Lys Leu His Ser His Val Leu Thr Met Pro Pro
        275                 280                 285

Ala Arg Lys Lys Lys Ala Glu Ser Arg Lys Tyr Phe Pro Phe Arg Gln
    290                 295                 300

Gly Tyr Leu Ala Val Ala Ile Leu Arg Ile Gly Ala His Gly Ile His
305                 310                 315                 320

Met Thr Val Asp Gly Lys His Ile Thr Ser Phe Ala Phe Arg Glu Asp
                325                 330                 335

Leu Glu Pro Gly Phe Val Gly Glu Val Arg Ile Ala Gly Asp Ile Lys
            340                 345                 350

Leu Leu Ser Val Ile Ala Ser Gly Leu Pro Thr Thr Glu Tyr Phe Glu
        355                 360                 365

His Val Thr Asp Leu Glu Ile Leu Lys Ala Pro Pro Val Ala Met Asp
    370                 375                 380

Lys Ser Val Asp Leu Phe Ile Gly Val Phe Ser Thr Ala Asn Asn Phe
385                 390                 395                 400

Lys Arg Arg Met Ala Val Arg Arg Thr Trp Met Gln Tyr Asp Ala Val
                405                 410                 415

Arg Ser Gly Lys Val Ala Val Arg Phe Phe Val Gly Leu His Lys Asn
            420                 425                 430

Glu Val Val Asn Glu Glu Leu Trp Asn Glu Ala Arg Thr Tyr Gly Asp
        435                 440                 445

Ile Gln Leu Met Pro Phe Val Asp Tyr Tyr Ser Gln Ile Leu Trp Lys
    450                 455                 460

Thr Ile Ala Ile Cys Ile Tyr Gly Thr Asn Val Leu Ser Ala Lys Tyr
465                 470                 475                 480

Val Met Lys Thr Asp Asp Asp Ala Phe Val Arg Val Asp Glu Ile Leu
                485                 490                 495

Ser Ser Leu Asp Arg Thr Asn Ile Ser His Gly Leu Leu Tyr Gly Arg
            500                 505                 510

Val Asn Ser Asp Ser Gln Pro His Arg Asp Pro Tyr Ser Lys Trp Tyr
        515                 520                 525

Ile Thr Pro Glu Glu Trp Pro Glu Glu Asn Tyr Pro Pro Trp Ala His
    530                 535                 540

Gly Pro Gly Tyr Ile Val Ser Gln Asp Ile Ala Lys Glu Val Tyr Arg
545                 550                 555                 560

Lys His Lys Asn Gly Glu Leu Lys Met Phe Lys Leu Glu Asp Val Ala
                565                 570                 575

Met Gly Ile Trp Ile Asn Glu Met Lys Lys Asp Gly Leu Asp Val Lys
            580                 585                 590

Tyr Glu Asn Asp Gly Arg Ile Leu Val Glu Gly Cys Glu Asp Gly Tyr
        595                 600                 605

Val Ile Ala His Tyr Gln Glu Pro Arg Asp Met Met Cys Leu Trp Asp
    610                 615                 620

Lys Phe Gln Lys Thr Lys Arg Gly Thr Cys Cys Lys Glu
625                 630                 635

<210>  31
<211>  637
<212>  PRT
<213>  Oryza sativa

<400>  31

Met Lys Lys Trp His Gly Gly Phe Val Ile Val Ser Leu Phe Ile Ile
1               5                   10                  15

Leu Met Leu Arg Tyr Val Ile Leu Asp Ser Pro Leu Ala Glu Lys Ser
            20                  25                  30

Leu Gln Tyr Val Phe Gln Gln Asn Asn Thr Thr Pro Leu His Trp Leu
        35                  40                  45

Asp Val Pro Asn Pro Pro Ala Val Gln Asn Pro Gln Asn Ile Ser Gln
    50                  55                  60

Val Ile Ser Thr Glu Leu Leu Ala Ser Asn Leu Ser Ile Thr Arg Asn
65                  70                  75                  80

Phe Ser Asp Arg Glu Leu Gln Ser Leu His Ser Trp Asn His Leu Lys
                85                  90                  95

Asp Leu Val Ser His Ala His Ile Leu Pro Asp Gly Val Glu Ala Ile
            100                 105                 110

Lys Glu Ala Gly Val Ala Trp Arg Glu Leu Asn Ala Ala Leu Glu Tyr
        115                 120                 125

Asp Glu Ser Ala Val Ser Val Asn Gly Ser Thr His Gln Lys Ser Lys
    130                 135                 140

Glu Lys Gln Cys Pro Tyr Ser Ile Arg Arg Met Asn Ala Thr Arg Ser
145                 150                 155                 160

Gly Asp Arg Phe Val Leu Lys Ile Pro Cys Gly Leu Ile Gln Gly Ser
                165                 170                 175

Ser Ile Thr Ile Ile Gly Thr Pro Gly Gly Leu Leu Gly Ser Phe Lys
            180                 185                 190

Ile Glu Leu Thr Gly Ala Thr Val Pro Gly Glu Pro Asp Pro Pro Ile
        195                 200                 205

Val Leu His Tyr Asn Val Arg Leu Leu Gly Asp Lys Leu Thr Glu Asp
    210                 215                 220

Pro Val Ile Val Gln Asn Thr Trp Thr Ile Ala Asp Asp Trp Gly Ser
225                 230                 235                 240

Glu Asp Arg Cys Pro Ser Pro Gly Ser Asp Ala Lys Asp Ile Ala Lys
                245                 250                 255

Val Asp Glu Leu Glu Lys Cys Gly Ser Met Val Gly Asn Asp Gln Lys
            260                 265                 270

Gln Ala Trp Ala Thr Lys Leu Lys Ser Asn Val Ser Ser Ile Gln Pro
        275                 280                 285

Ala Trp Lys Lys Asn Thr Glu Pro Lys Lys Tyr Phe Pro Phe Arg Gln
    290                 295                 300

Gly Tyr Leu Ala Ile Ala Ile Leu Arg Val Gly Ala His Gly Ile His
305                 310                 315                 320

Met Thr Val Asp Gly Lys His Val Thr Ser Phe Ala Phe Arg Glu Asp
                325                 330                 335

Leu Glu Pro Gly Phe Val Gly Glu Val Arg Ile Ala Gly Asp Ile Lys
            340                 345                 350

Leu Leu Ser Val Ile Ala Ser Gly Leu Pro Thr Thr Glu Asp Phe Glu

        355                 360                 365

His Val Thr Asp Leu Glu Ile Leu Lys Ala Pro Pro Val Pro Met Asp
    370                 375                 380

Lys Pro Leu Asp Leu Phe Ile Gly Ile Phe Ser Thr Ala Asn Asn Phe
385                 390                 395                 400

Lys Arg Arg Met Ala Val Arg Arg Thr Trp Met Gln Tyr Asp Ala Val
                405                 410                 415

Arg Ser Gly Lys Ala Ala Val Arg Phe Phe Val Gly Leu His Lys Asn
            420                 425                 430

Glu Val Val Asn Glu Glu Leu Trp Asn Glu Ala Arg Thr Tyr Gly Asp
        435                 440                 445

Ile Gln Leu Met Pro Phe Val Asp Tyr Tyr Ser Leu Ile Leu Trp Lys
    450                 455                 460

Thr Ile Ala Ile Cys Ile Tyr Gly Thr Asn Val Leu Ser Ala Lys Tyr
465                 470                 475                 480

Val Met Lys Thr Asp Asp Asp Ala Phe Val Arg Val Asp Glu Ile Leu
                485                 490                 495

Leu Ser Leu Asp Arg Val Asn Ile Ser His Gly Leu Leu Tyr Gly Arg
            500                 505                 510

Val Asn Ser Asp Ser Gln Pro His Arg Asp Pro Tyr Ser Lys Trp Tyr
        515                 520                 525

Ile Thr Pro Glu Glu Trp Pro Glu Glu Ser Tyr Pro Pro Trp Ala His
    530                 535                 540

Gly Pro Gly Tyr Ile Val Ser Gln Asp Ile Ala Lys Glu Val Tyr Arg
545                 550                 555                 560

Lys His Lys Arg Gly Glu Leu Lys Met Phe Lys Leu Glu Asp Val Ala
                565                 570                 575

Met Gly Ile Trp Ile Asn Glu Met Lys Lys Asp Gly Leu Asp Val Lys
            580                 585                 590

Tyr Glu Asn Asp Gly Arg Ile Leu Val Glu Gly Cys Glu Glu Gly Tyr
        595                 600                 605

Val Val Ala His Tyr Gln Glu Pro Arg Asp Met Met Cys Leu Trp Asp
    610                 615                 620

Lys Phe Leu Lys Thr Lys Arg Gly Thr Cys Cys Lys Glu
625                 630                 635

<210> 32
<211> 1914
<212> DNA
<213> Oryza sativa

<400> 32
atgaagaaat ggcacggtgg ttttgtcata gtatctttgt tcattatatt gatgctaaga 60

tatgttatat tggatagtcc acttgctgaa aaatctcttc agtatgtctt ccaacagaat 120

aacacaaccc cactacattg gctggatgtt ccaaacccgc ctgcagttca gaatccacag 180

aacatctctc aagtaatatc aactgaattg ttagcttcaa acctttccat aacaagaaat 240

ttctccgata gagaactgca gtcattgcat tcttggaatc atttaaaaga cctagtaagt 300

catgcgcaca tcttaccaga tggagtagaa gcaatcaagg aagctggagt agcatggagg 360

gagctaaatg cagcccttga atacgatgaa tctgctgttt ctgtcaatgg tagcactcat 420

caaaaaagta aagagaagca atgcccatat tccattcgaa gaatgaatgc tacaagatca 480

ggtgatagat ttgtcttaaa gattccttgt ggactgattc agggctcttc aataactatt 540

atcggtactc ctggtggtct tcttggtagt tttaagatag aattaactgg ggctactgta 600

cctggtgaac cagaccctcc aattgtgctt cattacaatg tccgtcttct tggtgataaa 660

cttacagaag atcctgtaat agtccaaaat acatggacta tagctgacga ctggggttct 720

gaagatcgtt gcccatctcc tggttcagat gctaaggaca ttgcaaaagt ggatgagtta 780

gaaaaatgcg gtagcatggt gggcaatgat caaaaacaag catgggccac taagttaaag 840

tccaatgttt caagcataca acctgcctgg aaaaagaaca cagaacctaa gaaatatttt 900

cccttcagac aaggatatct tgctatagca attcttcgtg ttggagcaca tgggatccat 960

atgactgtag atgggaaaca tgtcacttca tttgcctttc gggaggattt ggagcctgga 1020

tttgttggtg aagtaagaat tgcaggagat attaaattgt tgtctgtgat agcaagtggt 1080

ttgcctacaa cagaggactt tgaacatgtg actgacttag aaattttgaa ggctccacca 1140

gttcccatgg acaaacccct ggatcttttt attgggatct tttctacagc taacaatttc 1200

aagcgcagaa tggcagttcg cagaacttgg atgcagtatg atgctgtccg ctcaggaaag 1260

gctgcagttc gattctttgt tggcctgcat aaaaatgagg tggtgaatga ggaactttgg 1320

aatgaagcac gaacatatgg agacatccaa ttgatgccct ttgttgatta ttatagcctg 1380

attctatgga agactatagc catctgcatc tatgggacaa acgtcttgtc agccaagtat 1440

gtgatgaaaa cagatgatga tgcttttgtc cgtgtggatg agatactttt atctttagat 1500

cgcgtgaaca tcagtcatgg gttactgtac ggtcgtgtca attctgattc tcagcctcat 1560

cgagacccat atagcaagtg gtacataacc ccagaggaat ggcctgagga gagttatcct 1620

ccatgggcac atggtccggg atacattgta tcacaagaca tagcaaaaga agtttacagg 1680

aaacacaaga gaggagaact taagatgttc aaactagaag atgtcgcaat gggaatatgg 1740

atcaatgaga tgaagaagga tgggttggat gtaaagtatg aaaatgatgg gaggattttg 1800

gtcgaaggct gtgaggaagg atatgtggtt gcacattacc aggagccaag ggacatgatg 1860

tgcctatggg acaaatttct gaaaacaaaa cgaggaacat gttgcaagga gtaa 1914

<210> 33
<211> 621
<212> PRT
<213> Oryza sativa

<400> 33

Met Trp Val Thr Lys Arg Leu Gly Ile Thr Val Leu Ile Val Leu Phe
1               5                   10                  15

Pro Leu Leu Ile Val His His Leu Ile Val Asn Ser Pro Val Ser Gly
            20                  25                  30

Pro Ser Arg Tyr Gln Val Ile His Ser Asn Leu Leu Gly Trp Leu Ser
        35                  40                  45

Asp Ser Leu Gly Asn Ser Val Ala Gln Asn Pro Asp Asn Thr Pro Val
    50                  55                  60

Glu Val Ile Pro Ala Asp Ala Ser Ala Ser Asn Ser Ser Asp Ser Gly
65                  70                  75                  80

Asn Ser Ser Leu Glu Gly Phe Gln Trp Leu Asn Thr Trp Asn His Met
                85                  90                  95

Lys Gln Leu Thr Asn Ile Ser Asp Gly Leu Pro His Ala Asn Glu Ala
            100                 105                 110

Ile Asp Asn Ala Arg Thr Ala Trp Glu Asn Leu Thr Ile Ser Val His
        115                 120                 125

Asn Ser Thr Ser Lys Gln Ile Lys Lys Glu Arg Gln Cys Pro Tyr Ser
    130                 135                 140

Ile His Arg Met Asn Ala Ser Lys Pro Asp Thr Gly Asp Phe Thr Ile
145                 150                 155                 160

Asp Ile Pro Cys Gly Leu Ile Val Gly Ser Ser Val Thr Ile Ile Gly
                165                 170                 175

Thr Pro Gly Ser Leu Ser Gly Asn Phe Arg Ile Asp Leu Val Gly Thr
            180                 185                 190

Glu Leu Pro Gly Gly Ser Gly Lys Pro Ile Val Leu His Tyr Asp Val
        195                 200                 205

Arg Leu Thr Ser Asp Glu Leu Thr Gly Gly Pro Val Ile Val Gln Asn
    210                 215                 220

Ala Phe Thr Ala Ser Asn Gly Trp Gly Tyr Glu Asp Arg Cys Pro Cys
225                 230                 235                 240

Ser Asn Cys Asn Asn Ala Thr Gln Val Asp Asp Leu Glu Arg Cys Asn

                245                 250                 255

Ser Met Val Gly Arg Glu Glu Lys Arg Ala Ile Asn Ser Lys Gln His
            260                 265                 270

Leu Asn Ala Lys Lys Asp Glu His Pro Ser Thr Tyr Phe Pro Phe Lys
        275                 280                 285

Gln Gly His Leu Ala Ile Ser Thr Leu Arg Ile Gly Leu Glu Gly Ile
    290                 295                 300

His Met Thr Val Asp Gly Lys His Val Thr Ser Phe Pro Tyr Lys Ala
305                 310                 315                 320

Gly Leu Glu Ala Trp Phe Val Thr Glu Val Gly Val Ser Gly Asp Phe
                325                 330                 335

Lys Leu Val Ser Ala Ile Ala Ser Gly Leu Pro Thr Ser Glu Asp Leu
            340                 345                 350

Glu Asn Ser Phe Asp Leu Ala Met Leu Lys Ser Ser Pro Ile Pro Glu
        355                 360                 365

Gly Lys Asp Val Asp Leu Leu Ile Gly Ile Phe Ser Thr Ala Asn Asn
    370                 375                 380

Phe Lys Arg Arg Met Ala Ile Arg Arg Thr Trp Met Gln Tyr Asp Ala
385                 390                 395                 400

Val Arg Glu Gly Ala Val Val Val Arg Phe Phe Val Gly Leu His Thr
                405                 410                 415

Asn Leu Ile Val Asn Lys Glu Leu Trp Asn Glu Ala Arg Thr Tyr Gly
            420                 425                 430

Asp Ile Gln Val Leu Pro Phe Val Asp Tyr Tyr Ser Leu Ile Thr Trp
        435                 440                 445

Lys Thr Leu Ala Ile Cys Ile Tyr Gly Thr Gly Ala Val Ser Ala Lys
    450                 455                 460

Tyr Leu Met Lys Thr Asp Asp Asp Ala Phe Val Arg Val Asp Glu Ile
465                 470                 475                 480

His Ser Ser Val Lys Gln Leu Asn Val Ser His Gly Leu Leu Tyr Gly
                485                 490                 495

Arg Ile Asn Ser Asp Ser Gly Pro His Arg Asn Pro Glu Ser Lys Trp
            500                 505                 510

Tyr Ile Ser Pro Glu Glu Trp Pro Glu Glu Lys Tyr Pro Pro Trp Ala
        515                 520                 525

His Gly Pro Gly Tyr Val Val Ser Gln Asp Ile Ala Lys Glu Ile Asn
    530                 535                 540

Ser Trp Tyr Glu Thr Ser His Leu Lys Met Phe Lys Leu Glu Asp Val
545                 550                 555                 560

Ala Met Gly Ile Trp Ile Ala Glu Met Lys Lys Gly Gly Leu Pro Val
                565                 570                 575

Gln Tyr Lys Thr Asp Glu Arg Ile Asn Ser Asp Gly Cys Asn Asp Gly
            580                 585                 590

Cys Ile Val Ala His Tyr Gln Glu Pro Arg His Met Leu Cys Met Trp
        595                 600                 605

Glu Lys Leu Leu Arg Thr Asn Gln Ala Thr Cys Cys Asn
    610                 615                 620

<210>  34
<211>  1866
<212>  DNA
<213>  Oryza sativa

<400>  34
atgtgggtga caaagcgact cggaattacg gttctaatcg tcctcttccc tctgctgatt      60
gtgcatcatc tgatagtgaa tagcccagtg tcagggccat ctcgatacca agtcatacat     120
tctaatctcc ttggttggtt gagcgactca ttgggaaatt cagtggctca aaatccagat     180
aatacaccag tagaagtgat accagcagat gcttcagctt caaacagctc ggattccgga     240
aattcctctc ttgaaggatt tcagtggtta aatacatgga atcacatgaa gcagttgacc     300
aacatctccg atggccttcc tcacgcaaat gaggctatag acaatgcaag aactgcatgg     360
gaaaacctga cgatatcagt tcacaattca acctctaaac agattaagaa agagaggcag     420
tgtccatatt ctatccacag aatgaatgcc tccaagccag acactggaga ttttaccatc     480
gatatacctt gtgggctgat tgtaggttct tcagtaacta ttataggcac tccaggtagc     540
ctctccggta acttcaggat tgatcttgtt gggacagaat tgccaggggg atctgggaaa     600
cccatcgtac tgcactatga tgttcgtcta acaagtgatg aacttactgg aggtccagtc     660
atagtgcaga atgccttcac tgcaagtaat ggctggggtt atgaggatag atgtccctgc     720
agtaactgta ataatgcaac tcaagtggat gatttggagc gatgtaattc catggtgggt     780
agagaagaga agagagctat aaactcaaaa cagcacctga atgccaagaa agatgaacac     840
ccaagcacat attttccatt taagcaggga caccttgcta tctcaactct tcggataggg     900
ctagaaggga ttcacatgac agttgatgga aaacatgtta cctcatttcc atataaagcg     960
ggcttggaag catggttcgt tactgaagta ggagtatctg gtgatttcaa gctagtaagt    1020
gcaattgcaa gtggcctgcc cacatcagaa gacctggaga acagcttcga tcttgcaatg    1080
ttgaagtcgt cacctattcc agagggaaaa gatgtggacc ttttgattgg tatcttttca    1140
acagcaaaca acttcaaacg aaggatggcg atccgacgga catggatgca atatgatgcg    1200

gtgcgcgaag gagcagtagt ggtccgtttt ttcgttggcc tgcatacaaa cctcattgta 1260

aacaaggaac tctggaatga agcacgcaca tatggcgaca ttcaagtgct gccctttgtt 1320

gattactaca gtctgataac atggaagaca ctggcaatat gcatctacgg gactggtgct 1380

gtatcagcga aatatctgat gaaaacagac gatgatgctt ttgtccgtgt ggacgagata 1440

cactcctcag taaaacaact gaatgtcagt cacggcctac tttatggtcg tatcaattct 1500

gactcaggtc ctcacagaaa tcctgagagc aaatggtaca taagcccaga ggaatggcct 1560

gaagagaaat acccaccatg ggcacacgga ccaggatatg tggtatcaca agacattgca 1620

aaggaaatca acagttggta tgaaacaagt catttgaaga tgttcaaact agaagatgta 1680

gcaatgggta tatggattgc tgaaatgaag aagggtgggt tacctgtcca atacaaaaca 1740

gatgaaagaa ttaacagtga tggctgcaat gatgggtgca tcgttgctca ctaccaagaa 1800

ccaaggcaca tgctatgcat gtgggagaaa ctcctaagga caaatcaagc aacgtgctgc 1860

aactaa 1866

<210> 35
<211> 632
<212> PRT
<213> Populus trichocarpa

<400> 35

Met Lys Lys Trp Tyr Gly Gly Val Leu Val Ala Ser Leu Phe Met Leu
1               5                   10                  15

Leu Ile Leu Arg Tyr Gly Leu Leu Lys Asn Pro Ile Gly Gly Asn Ile
            20                  25                  30

Leu Leu Asn Pro Ser Ser Asn Ala Ser Lys Pro Leu Glu Trp Val His
        35                  40                  45

Pro Ala Val Pro Pro Ala Val Gln Asn Pro Glu Thr Ser Ser Gln Val
    50                  55                  60

Phe Ser Thr Asp Thr Ile Val Ser Ser Leu Phe Ala Pro Arg Asn Ile
65                  70                  75                  80

Ser Asn Glu Glu His Lys Ser Leu Gln Thr Trp Asn Leu Leu Lys His
                85                  90                  95

Leu Ile Asp His Ala Gln Val Phe Ala Asn Gly Val Glu Ala Ile Lys
            100                 105                 110

Glu Ala Gly Asn Ala Trp Ser Ser Leu Met Ala Ser Ala Glu Glu Glu
        115                 120                 125

Arg Leu Ser Tyr Thr Asn Glu Ser Ser Ser Arg Lys Val Lys Glu Lys
    130                 135                 140

Gln Cys Pro His Phe Leu Asn Lys Met Asn Ala Thr Glu Leu Asp Asn

145                 150                 155                 160

Ser Gly Tyr Lys Leu Trp Leu Pro Cys Gly Leu Thr Gln Gly Ser Ser
                165                 170                 175

Ile Thr Ile Ile Ser Ile Pro Asp Gly Leu Leu Gly Asn Phe Arg Ile
            180                 185                 190

Asp Leu Thr Gly Glu Ala Leu Pro Gly Glu Pro Asp Pro Pro Ile Ile
        195                 200                 205

Leu His Tyr Asn Val Arg Leu His Gly Asp Lys Ile Thr Glu Asp Pro
    210                 215                 220

Val Ile Val Gln Asn Thr Trp Thr Val Ala His Asp Trp Gly Glu Glu
225                 230                 235                 240

Glu Arg Cys Pro Ser Pro Ser Pro Glu Lys Ile Lys Lys Val Asp Glu
                245                 250                 255

Leu Asp Gln Cys Asn Lys Met Val Gly Arg Asn Asp Thr Arg Val Thr
            260                 265                 270

Ser Met His Ser Asp His Ser Arg Arg Ser Ser Leu Gln Glu Gly Thr
        275                 280                 285

Lys Ala Arg Arg Tyr Phe Pro Phe Lys Gln Gly Gln Leu Ser Val Ala
    290                 295                 300

Thr Leu Arg Val Gly Met Glu Gly Ile Gln Met Thr Val Asp Gly Lys
305                 310                 315                 320

His Ile Thr Ser Phe Ala Tyr Arg Glu Thr Leu Glu Pro Trp Leu Val
                325                 330                 335

Ser Glu Val Arg Ile Ser Gly Asp Leu Asn Leu Ile Ser Val Val Ala
            340                 345                 350

Ser Gly Leu Pro Thr Ser Glu Asp Ser Glu His Ala Val Asp Leu Glu
        355                 360                 365

Val Leu Lys Ser Ala Pro Leu Ser Pro Lys Lys Thr Leu Asp Leu Phe
    370                 375                 380

Ile Gly Val Phe Ser Thr Ala Asn Asn Phe Lys Arg Arg Met Ala Val
385                 390                 395                 400

Arg Arg Thr Trp Met Gln Tyr Ala Ala Val Arg Ser Gly Ala Val Ala
                405                 410                 415

Val Arg Phe Phe Val Gly Leu His Lys Asn Gln Ile Val Asn Glu Glu
            420                 425                 430

Leu Trp Asn Glu Ala Arg Thr Tyr Gly Asp Ile Gln Leu Met Pro Phe
        435                 440                 445

Val Asp Tyr Tyr Asn Leu Ile Thr Phe Lys Thr Leu Ala Ile Cys Ile
    450                 455                 460

Phe Gly Thr Glu Val Ala Ser Ala Lys Tyr Val Met Lys Thr Asp Asp
465                 470                 475                 480

Asp Ala Phe Val Arg Val Asp Glu Val Leu Ala Ser Leu Lys Arg Ile
                485                 490                 495

Lys Val Ser His Gly Leu Leu Tyr Gly Leu Ile Asn Ser Asp Ser Arg
            500                 505                 510

Pro His Arg Ser Thr Glu Ser Lys Trp Tyr Ile Ser Pro Glu Glu Trp
        515                 520                 525

Ser Glu Glu Thr Tyr Pro Pro Trp Ala His Gly Pro Gly Tyr Val Val
    530                 535                 540

Ser Arg Asp Ile Ala Lys Ala Val Tyr Lys Arg Tyr Lys Glu Gly Arg
545                 550                 555                 560

Leu Lys Met Phe Lys Leu Glu Asp Val Ala Met Gly Ile Trp Ile Ala
                565                 570                 575

Glu Met Lys Lys Glu Gly Leu Glu Val Lys Tyr Glu Met Glu Gly Arg
            580                 585                 590

Val His Asn Glu Gly Cys Arg Asp Gly Tyr Val Val Ala His Tyr Gln
        595                 600                 605

Ala Pro Arg Glu Met Leu Cys Leu Trp Gln Lys Leu Gln Glu Gly Asn
    610                 615                 620

Val Ala Arg Cys Cys Gly Asp Arg
625                 630

<210>  36
<211>  1899
<212>  DNA
<213>  Populus trichocarpa

<400>  36
atgaagaagt ggtacggtgg tgttctggtt gcatccttgt tcatgttact gatccttaga      60

tatggtctcc tgaaaaatcc aatcggagga aatattctgt tgaatccctc ctccaatgca     120

agcaagcctc ttgaatgggt gcatcctgct gttccacctg cagttcaaaa tccagagact     180

tcttctcaag ttttttctac agataccata gtctccagtc tgtttgctcc gagaaatatt     240

tccaatgaag agcacaagtc tctgcagaca tggaatctac tgaaacactt gattgatcat     300

gctcaggttt tcgcaaatgg agtagaagct attaaggaag ctggaaatgc gtggagcagc     360

ctgatggctt cagcagaaga ggaaagactt agttatacaa atgaaagttc aagtaggaaa 420
gtgaaagaga aacaatgtcc tcactttctt aacaaaatga atgccacaga gcttgataat 480
agtggttata aattgtggct tccttgtggc ttgactcagg gttcgtccat cacaattatc 540
agcattccag atggtcttct cggtaatttt cggatcgact taaccgggga agcacttcca 600
ggggagcctg atccacccat cattttacat tacaatgtta ggcttcatgg tgataagatt 660
acagaggatc cagtaatagt ccaaaacacc tggactgtgg ctcatgactg gggtgaagag 720
gagcgttgtc cttctccatc tcctgaaaaa attaagaaag tggacgagtt ggatcaatgt 780
aataagatgg tgggcagaaa cgatactcgg gtgactagca tgcattctga tcattcgaga 840
aggtcatcac ttcaggaagg aactaaagca agaagatatt ttccatttaa gcaaggtcag 900
ctatctgtcg caacgcttag agtgggaatg gaaggaatcc agatgactgt tgatggaaag 960
cacataacat cctttgcata tcgtgaaact ttggagccat ggctagttag tgaagttagg 1020
atttctgggg acctaaatct aatttcagtt gtggctagtg gtctacctac atcagaggat 1080
tcagagcatg cagttgatct agaagtactc aaatcagctc ctctctctcc aaaaaaaaca 1140
ctggatctct ttattggtgt cttttctacg gcaaacaatt ttaagcggag gatggctgtt 1200
cgaagaacat ggatgcagta tgctgcagtg cgatcagggg cagttgcagt gcgctttttt 1260
gttggtctgc ataaaaatca aatagtgaat gaggaactct ggaatgaagc acggacatat 1320
ggagacatac agctgatgcc ttttgttgac tactataacc tcatcacctt taaaacttta 1380
gctatctgca tatttgggac agaggttgca tcagctaagt atgtcatgaa gacagatgac 1440
gatgcgtttg ttcgtgtgga tgaagtgtta gcttctttaa agaggatcaa agtgagtcat 1500
gggttgctct atggattaat caattcagac tctcggcctc ataggagtac tgaaagcaag 1560
tggtatatta gccctgagga atggtctgaa gagacctatc ccccctgggc acatggtcct 1620
ggttacgttg tgtctcgtga cattgccaag gcagtttaca agagatacaa agaaggccgt 1680
ttaaagatgt ttaagctaga ggatgtagca atgggtatct ggattgctga gatgaaaaag 1740
gagggtttag aagtcaaata tgagatggaa gggagggtcc ataatgaagg gtgcagggat 1800
ggctatgttg tagcccacta ccaagctcct agggaaatgc tctgtctgtg gcagaaactt 1860
caagaaggga atgttgctag atgctgcggt gatcgataa 1899

<210> 37
<211> 637
<212> PRT
<213> Zea mays

<400> 37

Met Lys Lys Trp His Gly Gly Phe Val Ile Val Ser Leu Leu Ile Ile
1               5                   10                  15

Leu Met Leu Arg Tyr Val Ile Leu Asp Arg Leu Leu Ala Glu Lys Ser
            20                  25                  30

Leu Gln Tyr Val Phe Gln Gln Asn Arg Thr Ala Glu Leu His Trp Leu

35                  40                  45

Asp Val Pro Asn Pro Pro Ala Ile Gln Asn Pro Gln Ser Ser Ser Glu
    50                  55                  60

Val Ile Ser Thr Arg Leu Leu Ala Ser Asn Leu Ser Ile Thr Arg Asn
65                  70                  75                  80

Leu Ser Asp Thr Glu Leu Gln Ser Leu His Ser Trp Asn His Leu Lys
                85                  90                  95

His Leu Leu Thr His Ala His Ile Leu Pro Asp Gly Val Glu Ala Ile
            100                 105                 110

Lys Glu Ala Gly Val Ala Trp Arg Glu Leu Asn Thr Ala Leu Ala Tyr
        115                 120                 125

Asp Asp Ser Val Val Ser Val Asn Gly Ser Ile Gln Gln Lys Asp Lys
    130                 135                 140

Gly Lys Gln Cys Pro Tyr Ser Val Arg Arg Met Asn Ala Thr Arg Leu
145                 150                 155                 160

Gly Asp Arg Phe Ala Leu Lys Leu Pro Cys Gly Leu Ile Gln Gly Ser
                165                 170                 175

Ser Ile Thr Ile Ile Gly Thr Pro Gly Gly Leu Leu Ala Asn Phe Lys
            180                 185                 190

Ile Glu Leu Thr Gly Ala Ala Val Pro Gly Glu Pro Asp Pro Pro Ile
        195                 200                 205

Val Leu His Tyr Asn Val Arg Leu Leu Gly Asp Lys Leu Thr Glu Asp
    210                 215                 220

Pro Val Ile Val Gln Asn Thr Trp Thr Ile Ala Asp Asp Trp Gly Ser
225                 230                 235                 240

Glu Asn Arg Cys Pro Ser Pro Asp Ser Asp Ala Lys Asp Ser Ala Lys
                245                 250                 255

Val Asp Asp Leu Glu Lys Cys Ser Ser Met Val Gly Glu Asp Gln Lys
            260                 265                 270

Lys Ile Leu Pro Ser Lys Leu His Ser His Val Leu Thr Met Pro Pro
        275                 280                 285

Ala Arg Lys Lys Lys Ala Glu Ser Arg Lys Tyr Phe Pro Phe Arg Gln
    290                 295                 300

Gly Tyr Leu Ala Val Ala Ile Leu Arg Ile Gly Ala His Gly Ile His
305                 310                 315                 320

Met Thr Val Asp Gly Lys His Ile Thr Ser Phe Ala Phe Arg Glu Asp
                325                 330                 335

Leu Glu Pro Gly Phe Val Gly Glu Val Arg Ile Ala Gly Asp Ile Lys
            340                 345                 350

Leu Leu Ser Val Ile Ala Ser Gly Leu Pro Thr Thr Glu Tyr Phe Glu
        355                 360                 365

His Val Thr Asp Leu Glu Ile Leu Lys Ala Pro Pro Val Ala Met Asp
    370                 375                 380

Lys Ser Val Asp Leu Phe Ile Gly Val Phe Ser Thr Ala Asn Asn Phe
385                 390                 395                 400

Lys Arg Arg Met Ala Val Arg Arg Thr Trp Met Gln Tyr Asp Ala Val
                405                 410                 415

Arg Ser Gly Lys Val Ala Val Arg Phe Phe Val Gly Leu His Lys Asn
            420                 425                 430

Glu Val Val Asn Glu Glu Leu Trp Asn Glu Ala Arg Thr Tyr Gly Asp
        435                 440                 445

Ile Gln Leu Met Pro Phe Val Asp Tyr Tyr Ser Gln Ile Leu Trp Lys
    450                 455                 460

Thr Ile Ala Ile Cys Ile Tyr Gly Thr Asn Val Leu Ser Ala Lys Tyr
465                 470                 475                 480

Val Met Lys Thr Asp Asp Asp Ala Phe Val Arg Val Asp Glu Ile Leu
                485                 490                 495

Ser Ser Leu Asp Arg Thr Asn Ile Ser His Gly Leu Leu Tyr Gly Arg
            500                 505                 510

Val Asn Ser Asp Ser Gln Pro His Arg Asp Pro Tyr Ser Lys Trp Tyr
        515                 520                 525

Ile Thr Pro Glu Glu Trp Pro Glu Glu Asn Tyr Pro Pro Trp Ala His
    530                 535                 540

Gly Pro Gly Tyr Ile Val Ser Gln Asp Ile Ala Lys Glu Val Tyr Arg
545                 550                 555                 560

Lys His Lys Asn Gly Glu Leu Lys Met Phe Lys Leu Glu Asp Val Ala
                565                 570                 575

Met Gly Ile Trp Ile Asn Glu Met Lys Lys Asp Gly Leu Asp Val Lys
            580                 585                 590

Tyr Glu Asn Asp Gly Arg Ile Leu Val Glu Gly Cys Glu Asp Gly Tyr

595                 600                 605

Val Ile Ala His Tyr Gln Glu Pro Arg Asp Met Met Cys Leu Trp Asp
    610                 615                 620

Lys Phe Gln Lys Thr Lys Arg Gly Thr Cys Cys Lys Glu
625                 630                 635

<210>  38
<211>  1914
<212>  DNA
<213>  Zea mays

<400>  38
atgaagaaat ggcatggagg ctttgtcatt gtatctttgc tcatcatctt gatgttaaga      60

tatgtgatat tggataggct acttgcagaa aaatcgcttc agtatgtttt ccagcaaaac     120

aggacagcag aattacattg gttggatgtt ccaaacccac ctgcaatcca gaatccacag     180

agttcttctg aagtaatatc aaccagactc ttggcatcca acctttccat taccagaaat     240

ctctctgata cagaacttca atctttacat tcttggaatc atttgaaaca ccttttaacg     300

catgcccata tcctaccaga tggagtagag gcaatcaagg aagctggagt tgcatggagg     360

gaactcaata cagcccttgc atatgatgat tcagttgttt ctgttaatgg cagcattcag     420

cagaaggaca aaggaaagca atgcccttat tctgtccgac ggatgaatgc cacaagatta     480

ggggataggt ttgctttaaa acttccatgt ggattgattc agggctcttc aataacaatt     540

attggtactc ctggtggtct tctggctaat tttaagatag agttaactgg agctgcagtt     600

cctggtgaac cagaccctcc aattgtcctt cattataatg tccgccttct tggtgataaa     660

ctcacagaag atcctgtaat agtccaaaat acatggacga tagctgatga ttggggttct     720

gaaaaccgtt gcccatctcc tgattcagat gctaaggaca gtgcaaaagt ggatgatcta     780

gaaaagtgta gtagcatggt aggtgaggac cagaagaaaa tccttccctc taagttacat     840

tcacatgttt tgaccatgcc acctgcaagg aagaagaaag cagaatctag aaagtatttt     900

ccattcaggc agggatatct tgctgtagca attcttcgta tcggagcaca tgggatccat     960

atgactgtag atggcaaaca tatcacttcg tttgcatttc gagaagatct ggaaccaggg    1020

tttgttggtg aagtaagaat tgcaggagac attaaattgc tatctgtgat agcaagtggc    1080

ctgccaacaa cagagtactt cgagcatgtc actgatttgg aaattttgaa ggctccacct    1140

gttgccatgg ataaatctgt tgatcttttc attggggtct tctctacagc aaataatttc    1200

aaacgccgaa tggcagttcg cagaacttgg atgcagtatg atgctgtgcg ctcaggaaaa    1260

gttgcagttc gattctttgt tggcctgcac aaaaacgagg tggtcaatga ggaactctgg    1320

aatgaagcac gaacatatgg agacatccag ttgatgccgt ttgtggatta ctatagccag    1380

attctttgga agaccatagc gatctgcatc tatgggacaa atgtcctgtc agctaagtat    1440

gtgatgaaaa ctgatgatga tgcttttgtt cgagtagatg aaatactttc ttctctcgac    1500

cgaaccaata tcagccatgg actgttgtat ggtcgtgtca attctgattc ccagcctcat    1560

cgagatccct atagcaagtg gtacataacc cctgaggaat ggcctgagga gaattatcct    1620

ccatgggcgc atggtccagg atatattgtt tcacaggaca tagctaaaga agtttacagg 1680
aaacacaaga atggggaatt aaagatgttc aagctggagg atgtcgcgat gggaatatgg 1740
atcaatgaga tgaagaagga tggcttggat gtcaagtatg agaatgatgg gaggatcttg 1800
gttgaaggat gcgaggatgg gtatgtgatt gctcactacc aagaaccaag ggatatgatg 1860
tgcctctggg ataagtttca gaaaacaaaa cgaggaacat gttgcaaaga ataa 1914

<210> 39
<211> 637
<212> PRT
<213> Zea mays

<400> 39

Met Lys Lys Trp His Gly Gly Phe Val Ile Val Ser Leu Phe Ile Ile
1               5                   10                  15

Leu Met Leu Arg Tyr Val Ile Leu Asp Arg Leu Leu Ala Glu Lys Ser
            20                  25                  30

Leu Gln Tyr Val Phe Gln Gln Asn Arg Thr Ala Glu Leu His Trp Leu
        35                  40                  45

Asp Val Pro Asn Pro Pro Ala Ile Gln Asn Pro Gln Ser Ser Ser Glu
    50                  55                  60

Val Ile Ser Thr Arg Leu Leu Ala Ser Asn Leu Ser Ile Thr Arg Asn
65                  70                  75                  80

Leu Ser Asp Thr Glu Leu Gln Ser Leu His Ser Trp Asn His Leu Lys
                85                  90                  95

His Leu Leu Thr His Ala His Ile Leu Pro Asp Gly Val Glu Ala Ile
            100                 105                 110

Lys Glu Ala Gly Val Ala Trp Arg Glu Leu Asn Thr Ala Leu Ala Tyr
        115                 120                 125

Asp Asp Ser Val Val Ser Val Asn Gly Ser Ile Gln Gln Lys Asp Lys
    130                 135                 140

Gly Lys Gln Cys Pro Tyr Ser Val Arg Arg Met Asn Ala Thr Arg Leu
145                 150                 155                 160

Gly Asp Arg Phe Ala Leu Lys Leu Pro Cys Gly Leu Ile Gln Gly Ser
                165                 170                 175

Ser Ile Thr Ile Ile Gly Thr Pro Gly Gly Leu Leu Ala Asn Phe Lys
            180                 185                 190

Ile Glu Leu Thr Gly Ala Ala Val Pro Gly Glu Pro Asp Pro Pro Ile
        195                 200                 205

Val Leu His Tyr Asn Val Arg Leu Leu Gly Asp Lys Leu Thr Glu Asp
    210                 215                 220

Pro Val Ile Val Gln Asn Thr Trp Thr Ile Ala Asp Asp Trp Gly Ser
225                 230                 235                 240

Glu Asn Arg Cys Pro Ser Pro Asp Ser Asp Ala Lys Asp Ser Ala Lys
                245                 250                 255

Val Asp Asp Leu Glu Lys Cys Ser Ser Met Val Gly Glu Asp Gln Lys
            260                 265                 270

Glu Ile Leu Pro Ser Lys Leu His Ser His Val Leu Thr Met Pro Pro
        275                 280                 285

Ala Arg Lys Lys Lys Ala Glu Ser Arg Lys Tyr Phe Pro Phe Arg Gln
    290                 295                 300

Gly Tyr Leu Ala Val Ala Ile Leu Arg Ile Gly Ala His Gly Ile His
305                 310                 315                 320

Met Thr Val Asp Gly Lys His Ile Thr Ser Phe Ala Phe Arg Glu Asp
                325                 330                 335

Leu Glu Pro Gly Phe Val Gly Glu Val Arg Ile Ala Gly Asp Ile Lys
            340                 345                 350

Leu Leu Ser Val Ile Ala Gly Gly Leu Pro Thr Thr Glu Tyr Phe Glu
        355                 360                 365

His Val Thr Asp Leu Glu Ile Leu Lys Ala Pro Pro Val Ala Met Asp
    370                 375                 380

Lys Ser Val Asp Leu Phe Ile Gly Val Phe Ser Thr Ala Asn Asn Phe
385                 390                 395                 400

Lys Arg Arg Met Ala Val Arg Arg Thr Trp Met Gln Tyr Asp Ala Val
                405                 410                 415

Arg Ser Gly Lys Val Ala Val Arg Phe Phe Val Gly Leu His Lys Asn
            420                 425                 430

Glu Val Val Asn Glu Glu Leu Trp Asn Glu Ala Arg Thr Tyr Gly Asp
        435                 440                 445

Ile Gln Leu Met Pro Phe Val Asp Tyr Tyr Ser Leu Ile Leu Trp Lys
    450                 455                 460

Thr Ile Ala Ile Cys Ile Tyr Gly Thr Asn Val Leu Ser Ala Lys Tyr
465                 470                 475                 480

Val Met Lys Thr Asp Asp Asp Ala Phe Val Arg Val Asp Glu Ile Leu

                485                 490                 495

Ser Ser Leu Asp Arg Thr Asn Ile Ser His Gly Leu Leu Tyr Gly Arg
            500                 505                 510

Val Asn Ser Asp Ser Gln Pro His Arg Asp Pro Tyr Ser Lys Trp Tyr
        515                 520                 525

Ile Thr Pro Glu Glu Trp Pro Glu Glu Asn Tyr Pro Pro Trp Ala His
    530                 535                 540

Gly Pro Gly Tyr Ile Val Ser Gln Asp Ile Ala Lys Glu Val Tyr Arg
545                 550                 555                 560

Lys His Lys Asn Gly Glu Leu Lys Met Phe Lys Leu Glu Asp Val Ala
                565                 570                 575

Met Gly Ile Trp Ile Asn Glu Met Lys Lys Asp Gly Leu Asp Val Lys
            580                 585                 590

Tyr Glu Asn Asp Gly Arg Ile Leu Val Glu Gly Cys Glu Asp Gly Tyr
        595                 600                 605

Val Ile Ala His Tyr Gln Glu Pro Arg Asp Met Met Cys Leu Trp Asp
    610                 615                 620

Lys Phe Gln Lys Thr Lys Arg Gly Thr Cys Cys Lys Glu
625                 630                 635

<210> 40
<211> 1914
<212> DNA
<213> Zea mays

<400> 40
atgaagaaat ggcatggagg ctttgtcatt gtatctttgt tcatcatctt gatgttaaga 60

tatgtgatat tggataggct acttgcagaa aaatcgcttc agtatgtttt ccagcaaaac 120

aggacagcag aattacattg gttggatgtt ccaaacccac ctgcaatcca gaatccacag 180

agttcttctg aagtaatatc aaccagactc ttggcatcca acctttccat taccagaaat 240

ctctctgata cagaacttca atctttacat tcttggaatc atttgaaaca ccttttaacg 300

catgcccata tcctaccaga tggagtagag gcaatcaagg aagctggagt tgcatggagg 360

gaactcaata cagcccttgc atatgatgat tcagttgttt ctgttaatgg cagcattcag 420

cagaaggaca aaggaaagca atgcccttat tctgtccgac ggatgaatgc cacaagattg 480

ggggataggt ttgctttaaa acttccatgt ggattgattc agggctcttc aataactatt 540

attggtactc ctggcggtct tctggctaat tttaagatag agttaactgg agctgcagtt 600

cctggtgaac cagaccctcc aattgtcctt cattataatg tccgccttct tggtgataaa 660

ctcacagaag atcctgtaat agtccaaaat acatggacga tagctgatga ttggggttct 720

gaaaatcgtt gcccatctcc tgattcagat gctaaggaca gtgcaaaagt ggatgatcta 780

gaaaagtgta gtagcatggt aggtgaggac cagaaggaaa tccttccctc taagttacat 840
tcacatgttt tgaccatgcc acctgcaagg aagaagaaag cagaatctag aaagtatttt 900
ccattcaggc agggatatct tgctgtagca attcttcgta tcggagcaca tgggatccat 960
atgactgtag atggcaaaca tatcacttcg tttgcatttc gagaagatct ggaaccaggg 1020
tttgttggtg aagtaagaat tgcaggagac attaaattgc tatctgtgat agcaggtggc 1080
ctgccaacaa cagagtactt cgagcatgtc actgatttgg aaattttgaa ggctccacct 1140
gttgccatgg ataaatctgt tgatcttttc attggggtct tctctacagc aaataatttc 1200
aaacgccgaa tggcagttcg cagaacttgg atgcagtatg atgctgtgcg ctcaggaaaa 1260
gttgcagttc gattctttgt tggcctgcac aaaaacgagg tggtcaatga ggaactctgg 1320
aatgaagcac gaacatatgg agacatccag ttgatgccat ttgtggatta ctatagcctg 1380
attctttgga agaccatagc gatctgcatc tatgggacaa atgtcctgtc agctaagtat 1440
gtgatgaaaa ctgatgatga tgcttttgtt cgagtagatg aaatactttc ttctctcgac 1500
cgaaccaata tcagccatgg actgttgtat ggtcgtgtca attctgattc ccagcctcat 1560
cgagatccct atagcaagtg gtacataacc cctgaggaat ggcctgagga gaattatcct 1620
ccatgggcgc atggtccagg atatattgtt tcacaggaca tagctaaaga agtttacagg 1680
aaacacaaga atggggaatt aaagatgttc aagctggagg atgtcgcgat gggaatatgg 1740
atcaatgaga tgaagaagga tggcttggat gtcaagtatg agaatgatgg gaggatcttg 1800
gttgaaggat gcgaggatgg gtatgtgatt gctcactacc aagaaccaag ggatatgatg 1860
tgcctctggg ataagtttca gaaaacaaaa cgaggaacat gttgcaaaga ataa 1914

<210> 41
<211> 636
<212> PRT
<213> Zea mays

<400> 41

Met Lys Lys Trp His Gly Gly Phe Val Ile Val Ser Leu Phe Ile Ile
1               5                   10                  15

Leu Met Leu Arg Tyr Val Ile Leu Asp Asn Pro Leu Val Glu Asn Ser
            20                  25                  30

Leu Gln Tyr Val Phe Gln Gln Asn Arg Thr Ala Glu Leu His Trp Leu
        35                  40                  45

Asp Val Pro Asn Pro Pro Ala Ile Gln Asn Leu Gln Ile Ser Ser Glu
    50                  55                  60

Val Ile Ser Thr Gly Leu Leu Ala Ser Asn Leu Ser Ile Thr Arg Asn
65                  70                  75                  80

Leu Ser Asp Arg Glu Leu Gln Ser Leu His Ser Trp Asn His Leu Lys
                85                  90                  95

Gln Leu Leu Ser His Val His Ile Leu Pro Asp Gly Val Glu Ala Ile
            100                 105                 110

Lys Glu Ala Gly Val Ala Trp Arg Glu Leu Asn Thr Ala Leu Ala Tyr
        115                 120                 125

Asp Asp Ala Val Ser Val Asn Gly Ser Ile Gln Gln Lys Asp Lys Gly
    130                 135                 140

Lys Gln Cys Pro Tyr Ser Ile Arg Arg Met Asn Ala Thr Arg Leu Gly
145                 150                 155                 160

Asp Arg Phe Ala Leu Lys Leu Pro Cys Gly Leu Ile Gln Gly Ser Ser
                165                 170                 175

Ile Thr Ile Ile Gly Thr Pro Gly Gly Leu Leu Gly Asn Phe Lys Ile
            180                 185                 190

Glu Leu Thr Gly Ala Ala Val Pro Gly Glu Pro Asp Pro Pro Ile Val
        195                 200                 205

Leu His Tyr Asn Val Arg Leu Leu Gly Asp Lys Leu Thr Glu Asp Pro
    210                 215                 220

Val Ile Val Gln Asn Thr Trp Thr Ile Ser Asp Asp Trp Gly Ser Glu
225                 230                 235                 240

Asn Arg Cys Pro Ser Pro Asp Ser Asp Ala Lys Asp Thr Thr Lys Val
                245                 250                 255

Asp Asp Leu Glu Lys Cys Ser Ser Met Val Gly Glu Asp Gln Lys Glu
            260                 265                 270

Ile Leu Pro Ser Lys Phe His Ser Asn Val Ser Ala Met Pro Pro Ala
        275                 280                 285

Ser Lys Lys Lys Ala Glu Pro Arg Lys Tyr Phe Pro Phe Arg Gln Gly
    290                 295                 300

Tyr Leu Ala Val Ala Ile Leu Arg Ile Gly Ala His Gly Ile His Met
305                 310                 315                 320

Thr Val Asp Gly Lys His Ile Thr Ser Phe Ala Phe Arg Glu Asp Leu
                325                 330                 335

Glu Pro Gly Phe Val Gly Glu Val Arg Ile Ala Gly Asp Ile Lys Leu
            340                 345                 350

Leu Ser Val Ile Ala Ser Gly Leu Pro Thr Thr Glu Asp Phe Glu His
        355                 360                 365

Val Thr Asp Leu Glu Ile Leu Lys Ala Pro Pro Val Pro Met Asp Lys

370                 375                 380

Pro Val Asp Leu Phe Ile Gly Val Phe Ser Thr Ala Asn Asn Phe Lys
385                 390                 395                 400

Arg Arg Met Ala Val Arg Arg Thr Trp Met Gln Tyr Asp Ser Val Arg
                405                 410                 415

Ser Gly Lys Val Thr Val Arg Phe Phe Val Gly Leu His Lys Asn Glu
            420                 425                 430

Leu Val Asn Glu Glu Leu Trp Asn Glu Ala Arg Thr Tyr Gly Asp Ile
        435                 440                 445

Gln Leu Met Pro Phe Val Asp Tyr Tyr Ser Leu Ile Leu Trp Lys Thr
    450                 455                 460

Ile Ala Ile Cys Ile Tyr Gly Thr Asn Val Leu Ser Ala Lys Tyr Val
465                 470                 475                 480

Met Lys Thr Asp Asp Asp Ala Phe Val Arg Val Asp Glu Ile Leu Ser
                485                 490                 495

Ser Leu Asp Arg Thr Asn Ile Ser His Gly Leu Leu Tyr Gly Arg Val
            500                 505                 510

Asn Ser Asp Ser Gln Pro His Arg Asp Pro Tyr Ser Lys Trp Tyr Ile
        515                 520                 525

Thr Pro Glu Glu Trp Pro Glu Glu Asn Tyr Pro Pro Trp Ala His Gly
    530                 535                 540

Pro Gly Tyr Ile Val Ser Lys Asp Ile Ala Lys Glu Val Tyr Arg Lys
545                 550                 555                 560

His Lys Ser Gly Glu Leu Lys Met Phe Lys Leu Glu Asp Val Ala Met
                565                 570                 575

Gly Ile Trp Ile Asn Glu Met Lys Lys Asp Gly Leu Asp Val Lys Tyr
            580                 585                 590

Glu Asn Asp Gly Arg Ile Leu Val Glu Gly Cys Glu Asp Gly Tyr Val
        595                 600                 605

Ile Ala His Tyr Gln Glu Pro Arg Asp Met Met Cys Leu Trp Asp Gln
    610                 615                 620

Phe Gln Lys Thr Lys Arg Gly Thr Cys Cys Lys Glu
625                 630                 635

<210>  42
<211>  1911
<212>  DNA

<213>  Zea mays

<400>  42
atgaagaaat ggcatggagg ctttgtcatt gtatctttgt tcatcatctt gatgttaaga      60

tatgtgatat tggataaccc acttgtagaa aactcgcttc agtatgtttt ccagcaaaac     120

aggacggcag aattacattg gttggatgtt ccaaacccac ctgcaatcca gaatctacag     180

atttcttctg aagtaatatc aactggactc ttggcttcca acctttccat taccagaaat     240

ctctctgata gagaacttca atctttacat tcttggaatc atttgaaaca gctattatct     300

catgttcata tcctaccaga tggagtagag gcaatcaagg aagctggagt tgcatggagg     360

gaactcaata cagcccttgc atatgatgat gcagtttctg ttaatggcag cattcagcag     420

aaggacaaag gaaagcaatg cccttattct atcagaagga tgaatgccac aagattaggg     480

gataggtttg ctttaaaact tccctgtgga ctgattcagg gctcttcaat aactattatt     540

ggtacacctg gtggtcttct tggtaatttt aagatagagt taactggggc tgcagttcct     600

ggtgaaccag accctccaat tgtccttcat tataatgtcc gccttcttgg tgataaactc     660

acagaagatc ctgtaatagt ccaaaataca tggacaatat ctgatgattg gggttctgaa     720

aaccgttgcc catctcctga ttcagatgct aaggacacta caaaagtgga tgatctagaa     780

aaatgtagta gcatggtagg tgaggaccag aaagaaatcc ttccctctaa gtttcattca     840

aatgtttcgg ccatgccacc tgcaagtaag aagaaagcag aacctagaaa gtattttcca     900

ttcagacagg gatatcttgc cgtagcaatt cttcggatcg gagcacatgg gatccatatg     960

actgtagatg gcaaacatat cacttctttt gcattccgag aggatttgga accagggttt    1020

gtaggtgaag taagaattgc aggagacatt aaattgttat ctgtgatagc aagtggcctg    1080

cctacaacag aggacttcga gcatgtcact gacttagaaa tattgaaggc tccacctgtg    1140

cccatggata aaccggttga tcttttcatt ggggtcttct ctacagcaaa taatttcaaa    1200

cgccgaatgg cagttcgcag aacttggatg cagtatgatt ctgttcgatc aggaaaagtt    1260

acagttcgat tctttgttgg cctgcacaaa aatgagttgg tcaatgagga actctggaat    1320

gaagcacgaa catatggaga catccagttg atgccatttg tggattacta tagcctgatt    1380

ctttggaaga ccatagcgat ctgcatctat gggacaaatg tcctgtcagc taagtatgtg    1440

atgaaaactg atgatgatgc ttttgttcga gtagatgaaa tactttcttc tctcgaccga    1500

accaatatca gccatggact gttgtatggt cgtgtcaatt ctgattccca gcctcatcga    1560

gatccctata gcaagtggta cataacccct gaggaatggc ctgaggagaa ttatcctccc    1620

tgggcgcatg gaccaggata tattgtttca aaggacatag ctaaagaagt ttacaggaaa    1680

cacaagagtg gggagttaaa gatgttcaag ctggaggatg tcgcaatggg aatatggatc    1740

aatgagatga agaaggatgg cttggatgtc aagtatgaga atgatgggag gattttggtt    1800

gaaggatgcg aggatggcta tgtgattgct cattaccaag aaccaaggga tatgatgtgc    1860

ctctgggatc agtttcagaa aacaaaacga ggaacatgtt gcaaagaata a             1911

<210>  43
<211>  557

<212>  PRT
<213>  Nicotiana tabacum

<400>  43

Val Ser Asp Leu Phe Ile Lys Arg Asn Leu Ser Ala Gln Glu Gln Gln
1               5                   10                  15

Ser Leu Ser Thr Trp Tyr Gln Leu Lys Arg Leu Val Thr His Asp Gln
            20                  25                  30

Val Leu Pro Asn Ala Ile Glu Ala Val Lys Glu Ala Ser Val Ala Trp
        35                  40                  45

Asn Asn Leu Met Ser Ala Val Glu Arg Glu Lys Leu Asp Ala Asn Asp
    50                  55                  60

Ser Ser Val Lys Thr Gly Lys Gln Lys Gln Cys Pro His Phe Leu Ser
65                  70                  75                  80

Lys Thr Asn Ala Thr Glu Ile Asp Ala Ser Gly Phe Lys Leu Arg Phe
                85                  90                  95

Pro Cys Gly Leu Thr Gln Gly Ser Ser Ile Thr Ile Ile Gly Ile Pro
            100                 105                 110

Asn Gly Leu Leu Gly Asn Phe Arg Ile Asp Leu Thr Gly Glu Pro Leu
        115                 120                 125

Pro Gly Glu Pro Asp Pro Pro Val Ile Leu His Tyr Asn Val Arg Leu
    130                 135                 140

His Gly Asp Lys Ile Thr Glu Asp Pro Val Ile Val Gln Asn Thr Trp
145                 150                 155                 160

Thr Ile Ala His Asp Trp Gly Glu Glu Glu Arg Cys Pro Leu Pro Ser
                165                 170                 175

Asp Glu Lys Ser Lys Lys Val Asp Glu Leu Glu Gln Cys Asn Glu Met
            180                 185                 190

Val Gly Asn Val Ile Thr Arg His Val Ile Ala Thr Asn Lys Ser Ser
        195                 200                 205

Leu Ala Gln Asp Gly Ser Lys Ser Arg Lys Tyr Phe Pro Phe Lys Gln
    210                 215                 220

Gly Tyr Leu Ser Val Ala Thr Leu Arg Val Gly Ser Glu Gly Ile Gln
225                 230                 235                 240

Met Ala Val Asp Gly Lys His Ile Thr Ser Phe Ala Phe Arg Glu Thr
                245                 250                 255

Leu Glu Pro Trp Leu Val Ser Glu Val Arg Ile Ser Gly Asp Ile Lys

            260                 265                 270

Leu Ile Ser Val Val Ala Ser Gly Leu Pro Thr Ser Glu Asp Ser Glu
        275                 280                 285

His Ile Ser Asp Leu Glu Ala Leu Lys Ala Val Pro Leu Pro Pro Arg
    290                 295                 300

Lys Arg Leu Asp Leu Phe Val Gly Val Phe Ser Thr Ala Asn Asn Phe
305                 310                 315                 320

Lys Arg Arg Met Ala Val Arg Arg Thr Trp Met Gln Tyr Asp Ala Val
                325                 330                 335

Arg Ser Gly Gln Val Ala Val Arg Phe Phe Val Gly Leu His Lys Asn
            340                 345                 350

Gln Met Val Asn Glu Glu Leu Trp Asn Glu Ala Arg Thr Tyr Met Asp
        355                 360                 365

Ile Gln Leu Met Pro Phe Val Asp Tyr Tyr Ser Leu Ile Ala Trp Lys
    370                 375                 380

Thr Ile Ala Ile Cys Val Phe Gly Thr Glu Val Ile Ser Ala Lys Phe
385                 390                 395                 400

Val Met Lys Thr Asp Asp Asp Ala Phe Val Arg Val Asp Glu Ile Leu
                405                 410                 415

Ser Ser Met Gln Arg Ile Asn Val Thr Arg Gly Leu Leu Tyr Gly Leu
            420                 425                 430

Ile Asn Ser Asp Ser His Pro His Arg Ser Pro Asp Ser Lys Trp Phe
        435                 440                 445

Ile Ser Pro Glu Glu Trp Pro Glu Glu Thr Tyr Pro Pro Trp Ala His
    450                 455                 460

Gly Pro Gly Tyr Val Val Ser Ser Asp Ile Ala Lys Thr Ile Ser Ser
465                 470                 475                 480

Lys Gln Arg Lys Gly Arg Leu Lys Met Phe Lys Leu Glu Asp Val Ala
                485                 490                 495

Met Gly Ile Trp Ile Ser Glu Ile Lys Lys Lys Gly Leu Glu Val Lys
            500                 505                 510

Tyr Glu Lys Glu Glu Arg Ile Phe Asn Glu Gly Cys Arg Asp Gly Tyr
        515                 520                 525

Val Ile Ala His Tyr Gln Gly Pro Arg Glu Met Leu Cys Leu Trp Gln
    530                 535                 540

Lys Ile Glu Glu Lys Lys Arg Ala Leu Cys Cys Gly Glu
545                 550                 555

<210>  44
<211>  1674
<212>  DNA
<213>  Nicotia tabacum

<400>  44
gtctctgatc tctttatcaa gaggaacctg tcagctcagg agcaacaatc tttgtctacg      60
tggtatcagt tgaaacgctt agttactcat gatcaagtct tacctaatgc tatagaggca     120
gttaaggaag ccagtgttgc atggaacaac ctcatgagtg cagtagagag ggaaaaactt     180
gatgcaaatg atagttcagt taagaccggg aaacagaaac aatgccctca ttttctcagc     240
aaaaccaatg ctacagaaat tgatgccagt ggctttaagt tgcggtttcc ttgtggtctg     300
actcagggtt cttccatcac aataattggc attccaaatg gtcttcttgg gaattttcgg     360
atagatttga ctggtgaacc acttccaggt gaaccagatc ctcctgttat tctgcattac     420
aatgttaggc tccatggtga taaaataact gaggatcccg taattgtaca aaacacctgg     480
acaattgcac atgactgggg tgaagaggag cgctgtccct taccatcgga tgaaaagagt     540
aagaaagtgg atgaattgga gcaatgcaat gagatggtgg gaaatgtcat aactcgacat     600
gtcattgcaa caaataaatc tagcctggct caggacgggt ctaaatcaag aaaatatttt     660
cctttcaagc aaggatatct ctctgttgcc actctgagag taggatctga aggaattcag     720
atggcagttg atggaaaaca cataacatct tttgctttcc gtgaaacttt ggaaccgtgg     780
cttgtaagtg aagtgaggat atccggagac ataaaattaa tttctgttgt cgcgagtggt     840
ttgccaacat ctgaggattc agagcatata agtgacttgg aagctctaaa agcagttcct     900
cttcctcctc ggaaaagact agatctcttt gttggtgtat tttctaccgc aaataatttt     960
aagcgcagaa tggctgtccg tagaacttgg atgcaatatg atgcagtgcg atctggacaa    1020
gttgcggtgc ggttttttgt tggcttgcat aaaaaccaaa tggtgaatga agagctctgg    1080
aatgaggcta ggacatatat ggacatccag ctgatgcctt ttgttgatta ctatagtctt    1140
atcgcttgga agaccattgc catatgtgtt tttgggacgg aggtcatttc ggcaaagttt    1200
gtcatgaaga cagatgatga cgcatttgtt cgagtggatg aaatcttgtc ttctatgcag    1260
aggattaatg tgactcgcgg attgctctat ggtcttatca actcagattc ccatcctcat    1320
aggagcccag acagcaagtg gtttatcagt ccagaggaat ggcctgaaga aacttaccct    1380
ccttgggcac atggaccggg ttatgttgtg tccagtgata tagcaaaaac aatcagctca    1440
aaacagagaa aaggccgcct aaagatgttt aagctggaag atgttgctat gggcatctgg    1500
atttcagaaa taaagaagaa agggttggaa gtgaagtatg aaaaagaaga gaggattttt    1560
aatgaaggtt gccgagatgg ttatgttatt gcacattacc aaggccctag agagatgctc    1620
tgtctttggc aaaagattga agagaaaaaa cgggctttat gttgtggcga gtaa          1674

<210>  45
<211>  548

<212>  PRT
<213>  Nicotiana benthamiana

<400>  45

Ser Ala Gln Glu Gln Gln Ser Leu Ser Thr Trp Tyr Gln Leu Lys Arg
1               5                   10                  15

Leu Val Thr His Asp Arg Val Leu Pro Asn Ala Ile Glu Ala Val Lys
            20                  25                  30

Glu Ala Ser Ile Ala Trp Asn Asn Leu Met Ser Ala Val Glu Arg Glu
        35                  40                  45

Lys Leu Asp Ala Asn Asp Thr Ser Val Lys Thr Gly Lys Gln Lys Gln
    50                  55                  60

Cys Pro His Phe Leu Ser Lys Thr Asn Ala Thr Glu Ile Asp Ala Ser
65                  70                  75                  80

Gly Phe Lys Leu Arg Phe Pro Cys Gly Leu Thr Gln Gly Ser Ser Ile
                85                  90                  95

Thr Ile Ile Gly Ile Pro Asn Gly Leu Leu Gly Asn Phe Arg Ile Asp
            100                 105                 110

Leu Thr Gly Glu Pro Leu Pro Gly Glu Pro Asp Pro Pro Val Ile Leu
        115                 120                 125

His Tyr Asn Val Arg Leu His Gly Asp Lys Ile Thr Glu Asp Pro Val
    130                 135                 140

Ile Val Gln Asn Thr Trp Thr Ile Ala His Asp Trp Gly Glu Glu Glu
145                 150                 155                 160

Arg Cys Pro Leu Pro Ser Asp Glu Lys Ser Lys Lys Val Asp Glu Leu
                165                 170                 175

Glu Gln Cys Asn Glu Met Val Gly Asn Ile Met Ala Thr Arg His Val
            180                 185                 190

Ile Ala Thr Asn Lys Ser Ser Leu Ala Gln Asp Arg Ser Lys Ser Arg
        195                 200                 205

Lys Tyr Phe Pro Phe Lys Gln Gly Tyr Leu Ser Val Ala Thr Leu Arg
    210                 215                 220

Val Gly Ser Glu Gly Ile Gln Met Thr Val Asp Gly Lys His Ile Thr
225                 230                 235                 240

Ser Phe Ser Phe Arg Glu Thr Leu Glu Pro Trp Leu Val Ser Glu Val
                245                 250                 255

Arg Ile Ser Gly Asp Ile Lys Leu Ile Ser Val Val Ala Ser Gly Leu

260 . 265 270

Pro Thr Ser Glu Asp Ser Glu His Ile Ser Asp Leu Glu Ala Leu Lys
275 280 285

Ala Ala Pro Leu Pro Pro Arg Lys Arg Leu Asp Leu Phe Val Gly Val
290 295 300

Phe Ser Thr Ala Asn Asn Phe Lys Arg Arg Met Ala Val Arg Arg Thr
305 310 315 320

Trp Met Gln Tyr Asp Ala Val Arg Ser Gly Gln Val Ala Val Arg Phe
325 330 335

Phe Val Gly Leu His Lys Asn Gln Met Val Asn Glu Glu Leu Trp Asn
340 345 350

Glu Ala Arg Thr Tyr Met Asp Ile Gln Leu Met Pro Phe Val Asp Tyr
355 360 365

Tyr Ser Leu Ile Ala Trp Lys Thr Ile Ala Ile Cys Val Phe Gly Thr
370 375 380

Glu Val Val Ser Ala Lys Phe Val Met Lys Thr Asp Asp Asp Ala Phe
385 390 395 400

Val Arg Val Asp Glu Ile Leu Ser Ser Met Gln Arg Ile Asn Val Thr
405 410 415

Arg Gly Leu Leu Tyr Gly Leu Ile Asn Ser Asp Ser His Pro His Arg
420 425 430

Ser Pro Asp Ser Lys Trp Phe Ile Ser Pro Glu Glu Trp Pro Glu Glu
435 440 445

Thr Tyr Pro Pro Trp Ala His Gly Pro Gly Tyr Val Val Ser Ser Asp
450 455 460

Ile Ala Lys Gln Ser Ala Gln Asn Arg Lys Lys Gly Arg Leu Lys Met
465 470 475 480

Phe Lys Leu Glu Asp Val Ala Met Gly Ile Trp Ile Ser Glu Met Asn
485 490 495

Lys Lys Gly Leu Glu Val Lys Tyr Glu Lys Glu Glu Arg Ile Phe Asn
500 505 510

Glu Gly Cys Arg Asp Gly Tyr Val Ile Ala His Tyr Gln Gly Pro Arg
515 520 525

Glu Met Leu Cys Leu Trp Gln Lys Ile Glu Glu Lys Lys Arg Ala Leu
530 535 540

Cys Trp Gly Glu
545

<210> 46
<211> 1647
<212> DNA
<213> Nicotiana benthamiana

<400> 46
tcagctcagg agcaacaatc tttgtctacg tggtatcagt tgaaacgctt agttactcat 60

gatcgagtct tacctaatgc tatagaggca gttaaggaag ccagtatcgc atggaacaac 120

ctcatgagtg cagtagagag ggaaaaactt gatgcaaatg atacttcagt taagacaggg 180

aaacagaaac aatgccctca ttttctcagc aaaaccaatg ctacagaaat tgatgccagt 240

ggctttaagt tgcgatttcc ttgtggtctg actcagggtt cttccatcac aataattggc 300

attccaaatg gtcttcttgg gaattttcgg atagatttga ctggtgaacc acttccaggt 360

gaaccagatc ctcctgttat tctgcactac aatgttagac tccatggtga taaaataact 420

gaggatcccg taattgtaca aaacacctgg acaattgcac atgactgggg tgaagaggag 480

cgctgtccct taccatcgga tgaaaagagt aagaaagtgg atgaattgga acaatgcaat 540

gagatggtgg gaaatatcat ggctactcga catgtcattg caacaaataa atctagcctg 600

gctcaggaca ggtctaaatc aagaaagtat tttcctttca agcaaggata tctctctgtc 660

gccactctga gagtaggatc tgaaggaatt cagatgacag ttgatggaaa acacataaca 720

tctttttctt tccgtgaaac tttggaaccg tggcttgtaa gtgaagtgag gatatccgga 780

gacataaaat taatttctgt tgtcgcaagt ggtttgccaa catctgagga ttcagagcat 840

ataagtgatt tggaagctct aaaagcagct cctcttcctc ctcggaaaag actagatctc 900

tttgttggtg tattttctac cgcaaataat tttaagcgca gaatggctgt ccgtagaact 960

tggatgcaat atgatgcagt gcgatctgga caagttgcgg tgcggttttt tgtcggcttg 1020

cataaaaacc aaatggtgaa tgaagagctc tggaatgagg ctaggacata tatggacatc 1080

cagttgatgc cttttgttga ttactatagt cttatcgctt ggaagaccat tgccatatgt 1140

gtttttggga ccgaggtcgt ttcggcaaag tttgtcatga agacagatga tgacgcattt 1200

gttcgagtgg atgaaatctt gtcttctatg cagaggatta acgtgactcg cggattgctc 1260

tatggtctta tcaactcaga ttcccatcct cataggagtc cagacagcaa gtggtttatc 1320

agtccagagg aatggcctga agaaacttac cctccttggg cacatggacc gggttatgtt 1380

gtgtccagtg atatagcaaa acaatcagct caaaacagaa aaaaaggccg cctaaagatg 1440

tttaagctgg aagatgttgc tatgggcatc tggatttcag aaatgaataa gaaagggttg 1500

gaagtgaagt atgaaaagga agagaggatt tttaatgaag gttgccgaga tggttatgtt 1560

attgcacatt accaaggccc tagagagatg ctgtgtcttt ggcaaaagat tgaagagaaa 1620

aaacgggctt tatgttgggg cgagtaa 1647

<210> 47
<211> 284

<212>  PRT
<213>  Malus domestica

<400>  47

Gly Gly Leu Lys Leu Ile Ser Val Leu Ala Ser Gly Leu Pro Thr Ser
1               5                   10                  15

Glu Asp Ser Glu His Ile Ile Asn Leu Gln Glu Leu Lys Ser Ala Pro
            20                  25                  30

Leu Ser Pro His Lys Gln Met Asp Leu Phe Ile Gly Val Phe Ser Thr
        35                  40                  45

Ala Asn Asn Phe Lys Arg Arg Met Ala Val Arg Arg Thr Trp Met Gln
    50                  55                  60

Tyr Ala Ala Val Arg Ser Gly Ala Val Ser Val Arg Phe Phe Val Gly
65                  70                  75                  80

Leu His Lys Asn Gln Ile Val Asn Glu Glu Leu Trp Lys Glu Ala Gln
                85                  90                  95

Thr Tyr Gly Asp Ile Gln Leu Met Pro Phe Val Asp Tyr Tyr Gly Leu
            100                 105                 110

Ile Thr Trp Lys Thr Leu Ala Ile Cys Ile Phe Gly Thr Glu Val Val
        115                 120                 125

Ser Ala Lys Phe Val Met Lys Thr Asp Asp Asp Ala Phe Val Arg Val
    130                 135                 140

Asp Glu Val Leu Ala Ser Leu His Arg Ile Lys Val Asn Arg Gly Leu
145                 150                 155                 160

Leu Tyr Gly Leu Ile Asn Ser Asp Ser Arg Pro His Arg Ser Pro Asp
                165                 170                 175

Ser Lys Trp Tyr Ile Ser Asn Glu Glu Trp Pro Glu Ala Thr Tyr Pro
            180                 185                 190

Pro Trp Ala His Gly Pro Gly Tyr Val Val Ser Asn Asp Ile Ala Lys
        195                 200                 205

Ala Val Ser Gln Arg Tyr Lys Lys Gly Arg Leu Lys Met Phe Lys Leu
    210                 215                 220

Glu Asp Val Ala Met Gly Ile Trp Ile Ala Asp Met Lys Lys Glu Gly
225                 230                 235                 240

Leu Asn Val Arg Tyr Glu Lys Glu Glu Lys Val Tyr Asn Glu Gly Cys
                245                 250                 255

Lys Asp Gly Tyr Val Val Ala His Tyr Gln Gly Pro Arg Glu Met Leu

260                 265                 270

Cys Leu Arg Gln Lys Ile Pro Lys Gly Gln Val Ala
        275                 280

<210>  48
<211>  855
<212>  DNA
<213>  Malus domestica

<400>  48
ggaggcctga aattaatttc tgtcctagcc agcggtttac ccacgtcaga ggattcagag      60

cacataatta atttacaaga actcaaatct gctcctctct cgcctcacaa acaaatggat     120

ctctttattg gtgttttctc taccgcaaac aattttaaac gacggatggc tgttcgaaga     180

acatggatgc agtatgctgc tgtacgatca ggggcagttt cagtgcgctt ttttgttggt     240

ttgcataaga accaaatagt gaatgaggaa ctttggaaag aggcacaaac ctatggagac     300

atacagctga tgccttttgt tgattactat ggcctcatta cctggaaaac tctggccatc     360

tgcatctttg ggacggaggt tgtttcagcg aagttcgtta tgaagacgga tgacgatgca     420

tttgttcgtg tggatgaagt gctagcttcc ttacacagga tcaaagtgaa tcgtggcttg     480

ctctatggcc tcattaactc agattctcga cctcatcgaa gtccagatag caagtggtat     540

atcagcaatg aggaatggcc tgaagcaact tacccacctt gggcgcacgg tcctggttat     600

gtggtatcga atgacatagc aaaggcagtt tcccagcgat ataaaaaggg cagattaaag     660

atgtttaagc tggaagacgt ggcgatgggt atctggatcg cggatatgaa aaaggagggt     720

ctaaacgtac ggtatgagaa ggaagagaag gtctacaacg aaggatgcaa ggacgggtat     780

gtcgttgcac attaccaagg tcccagggag atgctgtgtc tccggcagaa aattcctaaa     840

gggcaagttg cttga                                                      855

<210>  49
<211>  265
<212>  PRT
<213>  Gossypium hirsutum

<400>  49

Asp His Thr Val Asp Ile Glu Ala Leu Leu Ser Val Pro Leu Ser Leu
1               5                   10                  15

Gln Arg Pro Val Asp Leu Phe Ile Gly Val Phe Ser Thr Ala Asn Asn
            20                  25                  30

Phe Lys Arg Arg Met Ala Val Arg Arg Thr Trp Met Gln Tyr Pro Glu
        35                  40                  45

Val Arg Ser Gly Thr Val Val Val Arg Phe Phe Val Gly Leu His Lys
    50                  55                  60

Ser His Ile Val Asn Asp Glu Leu Trp Asn Glu Ala Arg Thr Tyr Gly
65                  70                  75                  80

Asp Ile Gln Leu Met Pro Phe Val Asp Tyr Tyr Ser Leu Ile Thr Trp
                85                  90                  95

Lys Thr Leu Ala Ile Cys Thr Phe Gly Thr Glu Val Val Ser Ala Lys
            100                 105                 110

Phe Val Met Lys Thr Asp Asp Asp Ala Phe Val Arg Leu Asp Glu Val
        115                 120                 125

Met Ala Ser Leu Ser Arg Ile Asn Val Thr His Gly Leu Leu Tyr Gly
    130                 135                 140

Leu Ile Asn Ser Asp Ser Gln Pro His Arg Ser Thr Asp Ser Lys Trp
145                 150                 155                 160

Phe Ile Ser Pro Glu Glu Trp Ser Glu Glu Lys Tyr Pro Pro Trp Ala
                165                 170                 175

His Gly Pro Gly Tyr Val Val Ser His Asp Ile Ala Lys Ala Val Tyr
            180                 185                 190

Lys Arg Phe Asn Glu Val Arg Leu Lys Met Phe Lys Leu Glu Asp Val
        195                 200                 205

Ala Met Gly Ile Trp Ile Ala Asp Met Gln Lys Asp Gly Leu Glu Val
    210                 215                 220

His Tyr Glu Lys Glu Glu Arg Ile Phe Asn Glu Gly Cys Lys Asp Gly
225                 230                 235                 240

Tyr Val Ile Ala His Tyr Gln Gly Pro Arg Glu Met Met Cys Leu Trp
                245                 250                 255

Gln Lys Leu Pro Gly Asp Gln Lys Ser
            260                 265

<210>  50
<211>  798
<212>  DNA
<213>  Gossypium hirsutum

<400>  50
   gaccatacgg ttgatataga agcactcctt tcagttcctc tttctcttca aagaccagtg      60
   gatctcttta ttggtgtttt ctctactgcc aataatttta aaagaagaat ggctgtgaga     120
   agaacatgga tgcagtatcc tgaagtccga tctgggacag ttgtagtgcg cttttttgtt     180
   ggtttgcata aaagccatat agtgaatgat gaactctgga atgaagcacg gacatatgga     240
   gacatacagc tgatgccttt tgttgattac tacagcctga tcacctggaa aaccttggct     300
   atttgcacct ttgggacaga agttgtttct gcaaagtttg tcatgaagac agatgatgat     360
   gcgtttgttc gtttggatga agtcatggct tcattaagca ggataaatgt aactcatgga     420
   ttgctttatg gactcattaa ctcagattcc caacctcatc gcagtacaga tagcaagtgg     480
   tttattagcc ctgaggaatg gtctgaagag aagtacccac cttgggcgca tggtcctggt 540
   tacgtggtgt cccatgacat agcaaaggct gtttacaaga ggttcaatga agtgcgctta 600
   aagatgttta aactcgaaga tgtggcaatg ggcatttgga tcgcggatat gcaaaaagat 660
   ggtttagaag tccattatga gaaggaagag aggatcttta acgagggttg taaggatggt 720
   tatgttattg ctcactatca aggtccaaga gagatgatgt gtttgtggca gaaactccca 780
   ggagaccaaa agagctag 798

<210> 51
<211> 186
<212> PRT
<213> Phaseolus vulgaris

<400> 51

Pro Phe Val Asp Tyr Tyr Ser Leu Ile Thr Trp Lys Thr Leu Ala Ile
1               5                   10                  15

Cys Leu Phe Gly Thr Gln Val Ser Ala Lys Phe Val Met Lys Thr Asp
            20                  25                  30

Asp Asp Ala Phe Val Arg Val Asp Glu Val Leu Gly Ser Leu His Arg
        35                  40                  45

Ile Asn Val Asp His Gly Leu Leu Tyr Gly Leu Ile Asn Ser Asp Ser
    50                  55                  60

Arg Pro His Arg Asn Thr Asp Ser Lys Trp Tyr Ile Ser Pro Glu Glu
65                  70                  75                  80

Trp Ser Glu Gly Thr Tyr Pro Pro Trp Ala His Gly Pro Gly Tyr Val
                85                  90                  95

Val Ser His Asp Ile Ala Arg Thr Val Ala Lys Lys Phe Arg Glu Asn
            100                 105                 110

His Leu Lys Met Phe Lys Leu Glu Asp Val Ala Met Gly Ile Trp Ile
        115                 120                 125

Ala Asp Met Lys Lys Glu Gly Val Asp Val Arg Cys Glu Asn Glu Asp
    130                 135                 140

Arg Val Tyr Pro Glu Gly Cys Lys Asp Gly Tyr Val Val Ala His Tyr
145                 150                 155                 160

Gln Gly Pro Arg Glu Met Leu Cys Leu Trp Gln Lys Leu Glu Glu Gly
                165                 170                 175

Lys Gly Ala Lys Cys Cys Gly Asp Arg Lys
            180                 185

<210> 52

<211>  560
<212>  DNA
<213>  Phaseolus vulgaris

<400>  52
ccatttgttg actactacag cctcatcacg tggaaaactt tggcaatctg cctttttggg      60

acacaagttt cggcaaagtt tgtcatgaag acggatgatg atgcgtttgt cagggtagat      120

gaagttctag gctctttgca caggatcaat gttgatcatg ggttactgta tggactcatc      180

aattcggatt ccagacctca tcgaaataca gatagcaaat ggtacatcag cccagaggaa      240

tggagcgaag gaacttatcc tccttgggca catggtcccg gctatgtggt ctcacacgat      300

atagcaagaa cggttgccaa gaaattcaga gagaaccatt tgaagatgtt taaattagaa      360

gacgttgcaa tgggaatttg gattgcagac atgaagaagg aaggtgtaga tgttcggtgt      420

gagaatgagg acagagtgta tcctgagggt tgtaaggatg gttatgtggt tgcccattac      480

cagggcccta gggagatgct ctgtttgtgg cagaaacttg aagaaggaaa aggtgcaaaa      540

tgctgtggtg ataggaaatg                                                  560

<210>  53
<211>  235
<212>  PRT
<213>  Euphorbia esula

<400>  53

Thr Ile Asp Gly Lys His Ile Thr Ser Phe Ala Tyr Arg Glu Thr Leu
1               5                   10                  15

Glu Pro Trp Leu Val Ser Glu Val Arg Ile Ser Gly Asp Met Asn Leu
            20                  25                  30

Met Ser Ile Val Ala Ser Gly Leu Pro Thr Ser Glu Asp Ser Glu His
        35                  40                  45

Ala Ile Asp Leu Glu Leu Leu Lys Ser Val Pro Leu Pro Ser Lys Lys
    50                  55                  60

Thr Pro Pro Pro His Leu Phe Ile Gly Val Phe Ser Thr Ala Asn Asn
65                  70                  75                  80

Phe Lys Arg Arg Met Ala Val Arg Arg Thr Trp Met Gln Tyr Pro His
                85                  90                  95

Val Arg Ser Gly Thr Val Ala Val Arg Phe Phe Val Gly Leu His Lys
            100                 105                 110

Asn Gln Leu Val Asn Lys Glu Leu Trp Asn Glu Ala Ser Thr Tyr Gly
        115                 120                 125

Asp Ile Gln Leu Met Pro Phe Val Asp Tyr Tyr Asn Leu Ile Thr Trp
    130                 135                 140

Lys Thr Leu Ala Ile Cys Ile Phe Gly Thr Glu Val Ala Ser Ala Lys

145                 150                 155                 160

Tyr Val Met Lys Thr Asp Asp Asp Ala Phe Val Arg Val Asp Gln Val
                165                 170                 175

Leu Ala Ser Leu Lys Arg Ile Lys Val Asn Arg Gly Leu Leu Tyr Gly
            180                 185                 190

Leu Ile Asn Ser Asp Ser Gln Pro His Arg Ser Thr Asp Ser Lys Trp
        195                 200                 205

Tyr Ile Ser Leu Glu Glu Trp Pro Glu Glu Lys Tyr Pro Pro Trp Ala
    210                 215                 220

His Gly Pro Gly Tyr Val Val Ser Gln Asp Ile
225                 230                 235

<210>  54
<211>  707
<212>  DNA
<213>  Euphorbia esula

<400>  54
accattgatg gaaaacacat cacatctttt gcgtatcgcg agacattgga gccatggctg      60

gttagcgaag ttagaatatc tggagacatg aatttaatgt ctatagtggc aagtggtcta     120

cctacatcag aggattcaga acatgcaatt gatctagagc tactcaaatc ggtccctctc     180

ccctcgaaga aaacaccacc acctcatctc tttattggag tattctccac agccaacaat     240

tttaaacgta ggatggctgt tcgaagaact tggatgcagt atcctcatgt tcgctctggg     300

actgttgctg tgcgcttttt tgttgggttg cacaaaaatc aattagttaa caaggaactt     360

tggaatgaag catcaacata tggagacata cagcttatgc cttttgtgga ctattataac     420

ctcatcacct ggaagacttt agctatctgc atctttggga ctgaggttgc atcagcaaaa     480

tatgtcatga agacagatga tgatgcattt gttcgagtgg atcaagtttt agcttcttta     540

aaacggatca aagtgaatcg cggcctgctt tatggactca taaattcaga ttcccagcct     600

cataggagta ctgatagcaa atggtatatt agccttgagg aatggcctga agagaaatat     660

ccgccatggg cacatggtcc tggttatgtg gtgtctcaag acatagc                   707

<210>  55
<211>  160
<212>  PRT
<213>  Lactuca sativa

<400>  55

Met Lys Lys Trp Tyr Ile Tyr Val Leu Ser Ala Ser Leu Leu Thr Leu
1               5                   10                  15

Thr Ile Leu Gly Tyr Leu Ile Ile Gln Asn Pro Ile Arg Glu Ala Tyr
            20                  25                  30

Ile Ser Ser Pro Pro Tyr Phe Asn Thr Thr Asn Pro Leu Glu Trp Ile

35                  40                  45

Asn Ser Ser Ser Pro Pro Val Gln Asn Gln Thr Pro Asn Pro Gln Ile
    50                  55                  60

Val Ser Thr Asp Glu Ile Val Ser Thr Leu Phe Ile His Arg Asn Ile
65                  70                  75                  80

Ser Phe Asn Glu Gln Gln Pro Leu His Thr Trp Asn His Leu Arg His
                85                  90                  95

Leu Ile Ser His Asp Arg Val Leu Pro Asn Thr Val Glu Ala Ile Lys
            100                 105                 110

Asp Gly Gly Ile Ala Trp Asn Asn Leu Met Thr Thr Val Glu Asp Asp
        115                 120                 125

Lys Arg Asp Met Asn Arg Ser Ser Gly Gly Lys Lys Lys Glu Lys Gln
    130                 135                 140

Cys Pro His Phe Leu Asn Lys Met Asn Ala Thr Glu Leu Asn Asp Thr
145                 150                 155                 160

<210>  56
<211>  480
<212>  DNA
<213>  Lactuca sativa

<400>  56
atgaagaaat ggtatatcta tgtcctatct gcatctctat taacattaac aatactcggg      60

tacttaatca ttcaaaatcc aataagagaa gcttacattt catcacctcc atacttcaac     120

acaacaaacc ctcttgaatg gataaattcc agttcccccc ctgttcaaaa ccaaaccccc     180

aaccctcaaa ttgtctccac tgatgaaata gtctccaccc tctttatcca cagaaacatc     240

tcattcaatg aacaacaacc attacacaca tggaaccatt taagacactt aataagccat     300

gatcgtgttc ttcctaacac agttgaagca atcaaagatg gtggaatcgc atggaataac     360

ctcatgacta cagttgaaga tgacaaacgt gacatgaata gaagctcagg ggggaaaaag     420

aaagagaaac aatgtcccca ttttcttaac aagatgaatg ccacagagct taatgatact     480

<210>  57
<211>  211
<212>  PRT
<213>  Solanum lycopersicum

<400>  57

Thr Met Leu Cys Val Phe His Glu Thr Leu Glu Pro Trp Leu Ile Ser
1               5                   10                  15

Glu Val Arg Ile Ser Gly Asp Ile Lys Leu Ile Ser Val Val Ala Ser
            20                  25                  30

Gly Leu Pro Thr Ser Glu Asp Ser Glu His Ile Ser Asp Leu Glu Ala

35                    40                    45

Leu Lys Ala Ala Pro Leu Pro Pro Arg Lys Arg Leu Asp Leu Phe Ile
    50                  55                  60

Gly Val Phe Ser Thr Ala Asn Asn Phe Lys Arg Arg Met Ala Val Arg
65                  70                  75                  80

Arg Thr Trp Met Gln Tyr Asp Ala Val Arg Ser Gly Gln Val Ala Val
                85                  90                  95

Arg Phe Phe Val Gly Leu His Lys Asn Gln Met Val Asn Glu Glu Leu
            100                 105                 110

Trp Asn Glu Ala Arg Thr Tyr Arg Asp Ile Gln Leu Met Pro Phe Val
        115                 120                 125

Asp Tyr Tyr Ser Leu Ile Thr Trp Lys Thr Ile Ala Ile Cys Val Phe
    130                 135                 140

Gly Thr Glu Val Val Ser Ala Lys Phe Val Met Lys Thr Asp Asp Asp
145                 150                 155                 160

Ala Phe Val Arg Val Asp Glu Ile Leu Ser Ser Met Glu Arg Val Asn
                165                 170                 175

Thr Thr Arg Gly Leu Leu Tyr Gly Leu Ile Asn Ala Asp Ser Gln Pro
            180                 185                 190

Tyr Arg Ser Pro Asp Ser Gln Trp Phe Ile Ser Pro Glu Glu Trp Ser
        195                 200                 205

Glu Glu Thr
    210

<210>  58
<211>  635
<212>  DNA
<213>  Solanum lycopersicum

<400>  58
acgatgttat gtgtcttcca tgaaactttg gaaccatggc tcataagtga agtgaggata      60

tcgggagaca taaaattaat ttctgttgtc gcaagtggtt taccaacatc agaggattca     120

gagcatataa gcgacttgga agccttaaaa gcagctcctc ttcctccacg aaaaagacta     180

gatctcttta ttggtgtatt ttctacagca aataatttta agcgtagaat ggctgtccgt     240

agaacttgga tgcaatatga tgcagtgcgg tctggacaag ttgcggtgcg gttttttgtt     300

ggcttgcata aaaaccaaat ggtgaatgaa gagctctgga atgaggctcg gacatacaga     360

gacatccagc tgatgccttt tgttgattac tacagtctta tcacttggaa aaccattgcc     420

atctgtgttt ttgggacgga ggttgtttct gcaaagtttg tcatgaagac agatgatgat     480

gcatttgttc gagtggatga aatcttatct tctatggaga gggttaacac gactcgtgga     540

ttgttatatg gacttattaa cgcagattcc caaccttaca ggagtccaga cagccagtgg 600

tttatcagtc cagaggaatg gtctgaagaa actta 635

<210> 59
<211> 192
<212> PRT
<213> Vitis vinifera

<400> 59

Gly Ala Val Ala Val Arg Phe Phe Val Gly Leu His Lys Asn Gln Met
1               5                   10                  15

Val Asn Glu Glu Leu Trp Lys Glu Val Gln Thr Tyr Gly Asp Ile Gln
            20                  25                  30

Leu Met Pro Phe Val Asp Tyr Tyr Ser Leu Ile Thr Trp Lys Thr Ile
        35                  40                  45

Ala Ile Cys Ile Phe Gly Thr Glu Ala Val Ser Ala Lys Tyr Val Met
    50                  55                  60

Lys Thr Asp Asp Asp Ser Phe Val Arg Val Asp Glu Val Leu Ala Ser
65                  70                  75                  80

Leu Lys Lys Thr Lys Val Thr His Gly Leu Leu Tyr Gly Leu Ile Asn
                85                  90                  95

Ser Asp Ala Arg Pro His Arg Ser Ser Asp Ser Lys Trp Tyr Ile Ser
            100                 105                 110

Pro Glu Glu Trp Ala Gly Glu Thr Tyr Pro Pro Trp Ala His Gly Pro
        115                 120                 125

Gly Tyr Val Val Ser Asn Asp Ile Ala Lys Thr Val Tyr Lys Arg His
    130                 135                 140

Lys Glu Gly His Leu Lys Met Phe Lys Leu Glu Asp Val Ala Met Gly
145                 150                 155                 160

Ile Trp Ile Ala Glu Met Lys Lys Gly Gly Met Glu Val Ser Tyr Ile
                165                 170                 175

Lys Glu Glu Arg Val Tyr Asn Glu Gly Cys Asn Asp Gly Tyr Val Val
            180                 185                 190

<210> 60
<211> 577
<212> DNA
<213> Vitis vinifera

<400> 60
ggggcagttg ccgtgcgctt ttttgttggt ctgcataaaa atcagatggt gaatgaggaa 60

ctctggaagg aggtgcagac atatggagac attcagctga tgccttttgt tgactattac 120

agtcttataa cctggaagac catagcaatt tgcatctttg ggactgaggc tgtttcggcc 180

aagtatgtga tgaaaacaga tgatgattca tttgttcgtg tggatgaagt gcttgcttct 240

ttaaagaaga ccaaagtgac tcatgggttg ctgtatggtc ttattaactc tgatgctcga 300

cctcatcgta gttctgacag taaatggtac atcagccctg aggaatgggc aggagaaact 360

tatccacctt gggcacatgg tcctggttac gtggtgtcaa atgacatagc aaaaacagtg 420

tacaagagac acaaggaagg tcatttaaag atgttcaagc tggaagatgt agcaatgggg 480

atctggatag cagagatgaa gaagggggga atggaggtta gttacataaa agaagagagg 540

gtctataatg aggggtgcaa cgatgggtac gttgttg 577

<210> 61
<211> 231
<212> PRT
<213> Triticum aestivum

<220>
<221> UNSURE
<222> (212)..(212)

<400> 61

Ala Glu Pro Lys Lys Tyr Tyr Pro Phe Lys Gln Gly Asn Leu Ala Ile
1               5                   10                  15

Ala Ile Leu Arg Val Gly Ala Glu Gly Ile His Met Thr Val Asp Gly
            20                  25                  30

Lys His Val Thr Ser Phe Ala Phe Arg Glu Asp Leu Glu Pro Gly Phe
        35                  40                  45

Val Gly Glu Val Arg Ile Glu Gly Asp Ile Lys Leu Leu Ser Val Leu
    50                  55                  60

Ala Ser Gly Leu Pro Thr Thr Glu Asp Phe Glu His Val Thr Asp Leu
65                  70                  75                  80

Glu Ile Leu Lys Ala Pro Pro Val Pro Lys Asp Lys Ser Ile Asp Leu
                85                  90                  95

Phe Ile Gly Ile Phe Ser Thr Ala Asn Asn Phe Lys Arg Arg Met Ala
            100                 105                 110

Val Arg Arg Thr Trp Met Gln Tyr Asp Ala Val Arg Ser Gly Lys Val
        115                 120                 125

Ala Val Arg Phe Phe Val Gly Leu His Lys Asn Asp Val Val Asn Lys
    130                 135                 140

Glu Leu Trp Asn Glu Ala Arg Thr Tyr Gly Asp Ile Gln Leu Met Pro
145                 150                 155                 160

Phe Val Asp Tyr Tyr Ser Leu Ile Leu Trp Lys Thr Ile Ala Ile Cys
                165                 170                 175

Ile Tyr Gly Thr Asn Val Leu Ser Ala Lys Tyr Val Met Lys Thr Asp
            180                 185                 190

Asp Asp Ala Phe Val Arg Val Asp Glu Ile Leu Ser Ser Leu His Gln
        195                 200                 205

Val Asn Ile Xaa His Gly Leu Leu Tyr Gly Arg Val Asn Ser Asp Ser
    210                 215                 220

Gln Pro His Arg Asp Pro Tyr
225                 230

<210>  62
<211>  693
<212>  DNA
<213>  Triticum aestivum

<220>
<221>  misc_feature
<222>  (635)..(635)
<223>  n is a, c, g, or t

<400>  62
gcagaaccta aaaaatatta tcccttcaaa caaggaaatc ttgctatagc aattcttcgt      60

gttggagcag aggggatcca tatgactgta gatgggaaac atgtcacttc ctttgcattt     120

cgagaggatt tggagcctgg gtttgttggt gaagtaagga ttgaaggaga tattaaattg     180

ctgtctgtgc tagcaagtgg cttgcctaca acagaggact ttgagcatgt cactgacttg     240

gaaatattga aggctccacc tgtccctaag gataaatcca ttgatctttt tattgggata     300

ttctctacag caaataattt taaacgcaga atggcggttc gaagaacctg gatgcagtac     360

gatgctgtcc gctcaggaaa agtcgctgtt cggttctttg ttggcctgca taaaaatgat     420

gtggtgaaca aggagctctg gaacgaagca cggacatatg gagacatcca attgatgcca     480

tttgttgatt attacagctt gattctttgg aagaccattg ccatttgcat atatggaaca     540

aatgtacttt cagccaagta tgttatgaaa accgacgatg acgctttcgt tcgtgtagat     600

gagatacttt cgtccctaca tcaagtaaat atcanccatg gactgctgta tggtcgcgtc     660

aattctgatt ctcagcctca ccgagatccg tat                                  693

<210>  63
<211>  193
<212>  PRT
<213>  Solanum tuberosum

<400>  63

Asn Asn Gln Val Ile Pro Ala Glu Ile Val Val Ser Asp Leu Phe Ile
1               5                   10                  15

Lys Arg Asn Leu Ser Ala Gln Glu Gln Gln Ser Leu Ser Thr Trp Tyr
            20                  25                  30

Gln Leu Lys Arg Leu Ile Thr His Asp Leu Val Leu Pro Asn Ala Ile
        35                  40                  45

Glu Ala Val Lys Glu Ala Thr Val Ala Trp Asn Asn Leu Met Ser Ala
    50                  55                  60

Val Glu Met Asp Lys Leu Asp Thr Asn Asp Ser Ser Ile Lys Thr Gly
65                  70                  75                  80

Lys Gln Lys Gln Cys Pro His Phe Leu Ser Lys Thr Asn Ala Thr Glu
                85                  90                  95

Leu Asp Ala Ser Gly Phe Lys Leu Arg Leu Pro Cys Gly Leu Thr Gln
            100                 105                 110

Gly Ser Ser Ile Thr Ile Ile Gly Ile Pro Asn Gly Leu Leu Gly Asn
        115                 120                 125

Phe Arg Ile Asp Leu Thr Gly Glu Pro Leu Pro Gly Glu Pro Asp Pro
    130                 135                 140

Pro Val Ile Leu His Tyr Asn Val Arg Leu His Gly Asp Lys Ile Thr
145                 150                 155                 160

Glu Asp Pro Val Ile Val Gln Asn Thr Trp Thr Ile Ala His Asp Trp
                165                 170                 175

Gly Glu Lys Glu Arg Cys Pro Leu Pro Ser Asp Glu Lys Ser Lys Lys
            180                 185                 190

Val

<210>  64
<211>  580
<212>  DNA
<213>  Solanum tuberosum

<400>  64
aataatcaag taatacctgc tgaaattgtt gtctctgatc tctttatcaa gaggaacctg      60

tcagctcagg agcaacaatc tttgtctacg tggtatcaat tgaaaaggtt aatcactcat     120

gatctagttt tacctaatgc tatagaggcg gttaaggaag ccactgttgc atggaacaac     180

ctcatgagtg cagtagagat ggacaaactc gatacaaatg atagttcaat taagacaggg     240

aaacagaaac aatgtcctca ttttcttagc aaaaccaatg ctacagaact tgatgctagc     300

ggctttaagt tgcggcttcc ttgtggtctg actcagggtt cttccatcac aataattggc     360

attccaaatg gtcttcttgg gaattttcgg atagatttga ctggtgaacc actaccaggt     420

gaaccagacc ctcctgttat tctgcactac aatgttaggc tccatggtga taaaataacc     480

gaggatcctg taatcgtaca aaacacctgg actattgcac atgactgggg tgaaaaggag     540

cgctgtccat taccatccga tgaaaagagt aagaaagtgg 580

<210> 65
<211> 137
<212> PRT
<213> Medicago sativa

<400> 65

Thr Leu Glu Pro Trp Leu Val Ser Glu Val Arg Ile Ala Gly Asp Leu
1               5                   10                  15

Lys Leu Leu Ser Ile Leu Ala Ser Gly Leu Pro Thr Ser Glu Glu Ser
            20                  25                  30

Glu His Val Val Asp Leu Glu Ala Leu Lys Ala Pro Pro Leu Ser Pro
        35                  40                  45

Leu Arg Pro Leu Asp Leu Val Ile Gly Val Phe Ser Thr Ala Asn Asn
    50                  55                  60

Phe Lys Arg Arg Met Ala Val Arg Arg Thr Trp Met Gln Tyr Asp Asp
65                  70                  75                  80

Val Arg Ser Gly Arg Val Ala Val Arg Phe Phe Val Gly Leu His Lys
                85                  90                  95

Ser Pro Ile Val Asn Leu Glu Leu Trp Asn Glu Ala Arg Thr Tyr Gly
            100                 105                 110

Asp Val Gln Leu Met Pro Phe Val Asp Tyr Tyr Ser Leu Ile Ser Trp
        115                 120                 125

Lys Thr Leu Ala Ile Cys Ile Phe Gly
    130                 135

<210> 66
<211> 411
<212> DNA
<213> Medicago sativa

<400> 66
acactggagc catggcttgt tagtgaagta cggattgcag gtgatttgaa gttactatcc 60
attctcgcta gcggtttacc cacatcagaa gaatcagagc atgttgttga tctagaggca 120
ctgaaggcac ctcccctttc accattaagg ccattagatc ttgtcattgg cgttttctcc 180
acagcaaaca attttaaacg acggatggct gtgagaagaa cttggatgca gtatgatgat 240
gtaagatctg gaagggttgc agtacgcttt tttgttggcc ttcacaagag tcctattgtt 300
aacttggaac tatggaacga ggctcggact tacggtgacg ttcagctaat gccctttgtt 360
gattactaca gtctcatcag ttggaaaaca ctagcaatct gcatcttcgg g 411

## Description

The present invention relates to the field of protein glycosylation engineering in plants, specifically to the production of glycoproteins in plants or plant cells.

Recombinant glycoproteins, like monoclonal antibodies show great promise as therapeutics and the demand for their production is steadily increasing. Hence, apart from the traditional ways of manufacturing recombinant proteins in living mammalian cells, mainly in Chinese hamster ovary cells, currently alternative expression systems, such as yeast, fungi, plants, insects and transgenic animals are being intensively investigated.

Production of recombinant glycoproteins in plants has the advantage of low cost, short generation times and easy scale-up. However, the N-glycan structures of plant-produced glycoproteins differ substantially from those found in animals. These differences in N-glycosylation can have important consequences on product safety and quality. It is well documented that β1,2-xylose and core α1,3-fucose containing N-linked glycans are immunogenic and potentially allergenic. The presence of these potentially immunogenic and/or allergenic plant-specific N-linked glycans therefore hinders the use of plants as production system for therapeutic glycoproteins. Furthermore, differences in N-glycosylation affect also the function and quality of the glycoprotein, for example due to reduced half-life of the drug, which results in the need to use higher doses. Achieving a proper glycosylation pattern as present on the natural human forms is therefore a major challenge for the production of therapeutic glycoproteins in plants.

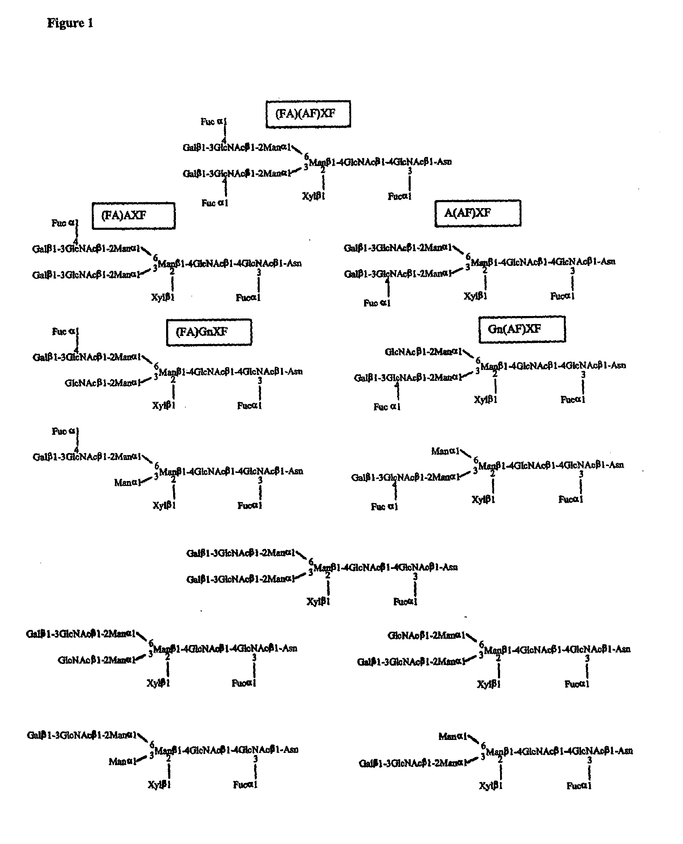

N-glycans from plants are typically much smaller and fewer glycoforms are detected compared to their mammalian counterparts. Plant glycoproteins contain two major types of oligosaccharides: paucimannosidic and complex N-glycans. Complex plant N-glycans carry β1,2-xylose and core α1,3-fucose, which are absent in mammalian glycoproteins, as well as one or two terminal N-acetylglucosamine (GlcNAc) residues attached to the pentasaccharide (Man₃GlcNAc₂) core structure. Plant glycoproteins differ from mammalian ones as they are devoid of β1,4-linked galactose and sialic acid. The only known plant N-glycan chain elongation is the attachment of a β1,3-galactose (Gal) and a α1,4-fucose (Fuc) residue, which form a trisaccharide (Galβ1-3(Fucα1-4)GlcNAc-R) known as the Lewis-A epitope (Figure 1).

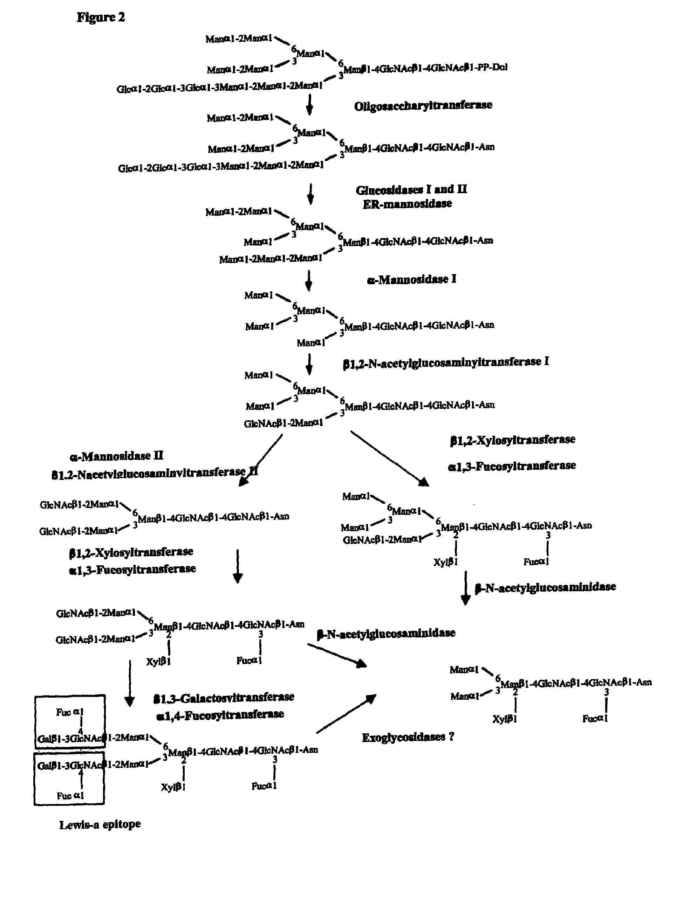

The N-glycosylation pathway in plants is well characterized (Figure 2). The genes which are responsible for the addition of GlcNAc residues (β1,2-N-acetylglucosaminyltransferase I, β1,2-~N-acetylglucosaminyltransferase II), processing (Golgi α-mannosidase II), attachment of β1,2-linked xylose (β1,2-xylosyltransferase) and core α1,3-linked fucose (core α1,3-fucosyltransferase) on complex N-glycans in plants have been cloned and characterized.

Lewis antigens, including type 1 (Le^{a}, Le^{b}) and type 2 chain (Le^{x}, Le^{y}) carbohydrates, are fucosylated oligosaccharide epitopes identified on the surface of certain eukaryotic cells. The Lewis-A antigen is structurally related to determinants of the human ABH blood group system. It is rare as such in healthy adults but as sialyl-Lewis-A it is notoriously found in malignant tissues but usually not attached to mammalian protein N-linked glycans.

In plants the Lewis-A epitope is present in many species and it is detected on plant produced recombinant glycoproteins.

Hence, the presence of the Lewis-A carbohydrate structure represents a significant barrier for the production of recombinant glycoproteins in plants, since this epitope is usually not present on natural human or animal glycoproteins intended for therapeutic use (e.g. on erythropoietin). As a consequence there is a need for technologies to eliminate Lewis-A epitopes from plants and also from plant made recombinant glycoproteins. However, up to now it has not been possible to modulate this in many cases unwanted glycan epitope, simply due to the fact, that the gene/enzyme that initiates the biosynthesis of the Lewis-A epitope in plants was not known.

Two enzymes are required for the synthesis of the plant Lewis-A epitope linked to complex N-glycans. First, the plant Lewis-type β1,3-galactosyltransferase transfers a galactose to a GlcNAc residue with a β1,3-linkage, resulting in the synthesis of a type 1 chain (Galβ1-3GlcNAc), and then the plant Lewis-type α 1,4-fucosyltransferase transfers a fucose to the GlcNAc of the type 1 chain with an α1,4-linkage to complete the synthesis of the Lewis-A structure (Galβ1-3(Fucα1-4)GlcNAc). The Lewis-type α 1,4-fucosyltransferase has been cloned from several plant species and the corresponding enzyme activity has been characterized. The plant Lewis-type α1,4-fucosyltransferase has a strict acceptor substrate specificity for type 1 chain-based glycan structures whereas type 2 chain-based oligosaccharides (Galβ1-4GlcNAc), which are typical for mammalian glycoproteins, are not utilized. A Lewis-type β1,3-galactosyltransferase has neither been cloned from plants nor has the corresponding activity been described in plants.

What is needed, therefore, is a method to eliminate the Lewis type β1,3-galactosyltransferase from plants and a method to eliminate the Lewis-A epitope from plant-produced recombinant glycoproteins. This will enable the production of recombinant glycoproteins in plants with authentic N-linked glycan structures, devoid of Lewis-A epitopes.

Another object of the present invention is to provide a plant protein having Lewis-type β1,3-galactosyltransferase activity, and a DNA encoding the protein.

In a first embodiment the invention provides a method of expressing a plant Lewis-type β1,3-galactosyltransferase in an organism comprising the step of providing the organism with a nucleic acid molecule comprising a sequence A which is defined as being selected from
a) a sequence according to SEQ ID NO: 1 with an open reading frame from base pair 1-1932,
b) a sequence which is at least 50% identical with SEQ ID NO: 1,
c) a sequence which hybridizes with SEQ ID NO: 1 under stringent conditions, or
d) a sequence which has degenerated to SEQ ID NO: 1 due to the genetic code,
   wherein the sequences a) to d) encode a plant protein having Lewis-type β1,3-galactosyltransferase activity, or
e) a sequence which is complementary to one of the sequences a) to d),
and expressing a protein encoded by sequence A or if the organism comprises a sequence A in its wildtype form overexpressing a protein encoded by sequence A. Preferably the nucleic acid molecule is a DNA molecule or a RNA or protein nucleic acid.

The present invention provides for the first time a sequence associated with the formation of the Lewis-A epitope in plants. This sequence can now for the first time be used for (over)expressing in plants for the production of glycoproteins comprising the Lewis-A epitope e.g. as a vaccine for immunotherapies. This is of particular interest since several types of cancer express the Lewis-A epitope in humans and mammals.

Although the sequence of SEQ ID NO: 1 was previously described (NCBI database AY133724) no explicit function especially the Lewis-type β1,3-galactosyltransferase activity was known. This knowledge however is of critical importance for any use therefore, including the design of knock-out constructs for the suppression of the Lewis-type β1,3-galactosyltransferase activity.

The activity of the plant Lewis-type β1,3-galactosyltransferase is detected by a method and measured, the galactosyltransferase being added to a reaction mixture containing UDP-galactose and a labeled acceptor (e.g. a glycopeptide or labeled oligosaccharide). After the reaction time, the content of bound galactose is measured. The activity of the galactosyltransferase in this case is seen as positive if the activity measurement is higher by at least 10 to 20%, in particular at least 30 to 50%, than the activity measurement of the negative control. The structure of the oligosaccharide may additionally be verified by means of HPLC. Such protocols are prior art (Staudacher et al., 1998, Anal. Biochem. 246: 96-101; Staudacher et al., 1991, Eur. J. Biochem. 199: 745-751). Whether the galactose is bound or not to the acceptor substrate can furthermore be determined by measuring the mass of the product by means of mass spectrometry (e.g. Strasser et al., 2000, FEBS Lett. 472: 105-108).

Lewis-A epitope can be detected on glycoproteins from plants by immunoblots using JIM84 antibody (Horsley et al., 1993, Journal of Exp. Bot. 44, Supplement: 223-229), which is specific for the plant Lewis-A epitope (Fitchette et al., 1999, Plant Physiol. 121: 333-343) and subsequently by total N-glycan analysis using mass spectrometry.

Preferably the protein encoded by the nucleic acid molecule having Lewis-type β1,3-galactosyltransferase activity has a sequence of SEQ ID NO: 2 or is at least 50% identical with SEQ ID NO: 2. In particular preferred the protein comprises the galactosyltransferase domain according to SEQ ID NO: 4, or a galactosyltransferase domain which is at least 50% identical with SEQ ID NO: 4. Thus the present invention provides for a group of homologuous enzymes with e.g. up to 5, 10, 20, etc. mutations which retain the Lewis-type β1,3-galactosyltransferase activity. In particular embodiments the sequence of the peptide is at least 60%, at least 70%, at least 75%, at least 80%, at least 90%, at least 95%, or at least 99% identical with SEQ ID NO: 2 or with the domain of SEQ ID NO: 4. Also provided in the method wherein the expression or overexpression is facilitated by transfecting the organism with a nucleic acid comprising a sequence A or by upregulation of expression of an endogenous sequence A.

Upregulation can be achieved by any means known in the art, especially the use of promoters or promoter-enhancer constructs. The constructs regulate the expression of sequence A on the nucleic acid molecule. Preferred examples for Lewis-type β1,3-galactosyltransferase expression enhancing compounds are substances which up-regulate Lewis-type β1,3-galactosyltransferase, e.g. toxins if expression of the transferase is controlled by toxin-inducible promoters on the nucleic acid. In principle, any cell which can be genetically manipulated so that it expresses a recombinant (i.e. a gene which has been artificially introduced into this cell or its precursor and which naturally is not present at this site of the genome of the cell) plant Lewis-type β1,3-galactosyltransferase gene can be used for this purpose. Preferably the promoter regulates increased expression when compared to the wildtype organism or is externally inducible to an increased expression when compared to the wildtype organism, preferably plant or plant cell. Upregulation can be achieved by transfecting the organism with a construct with sequence A or by stimulating endogenous expression of the Lewis-type β1,3-galactosyltransferase.

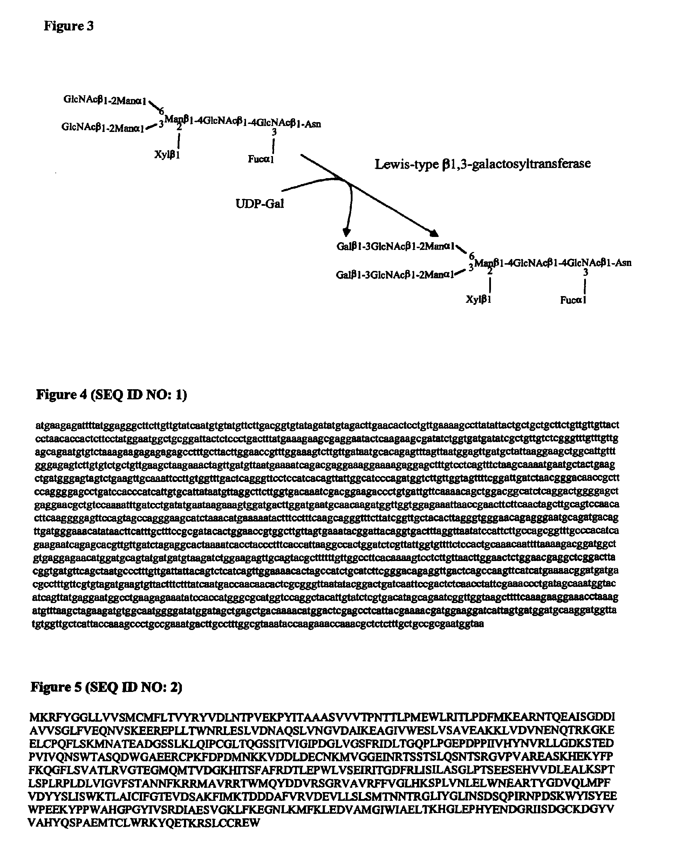

To facilitate understanding of the invention, a number of terms as used in this specification are defined below:
The term "glycosyltransferase" refers to any enzyme that transfers monosaccharide-residues from nucleotide sugar donor substrates to a carbohydrate structure.
The term "Lewis-type β1,3-galactosyltransferase" ("Lewis-type β1,3-GalT") refers to any glycosyltransferase that catalyses the first step in the formation of the Lewis-A epitope. The first step in the formation of the Lewis-A epitope is the transfer of a galactose residue in β1,3-linkage to a GlcNAc residue at the non-reducing end of a carbohydrate side-chain (see Figure 3). This reaction creates the type 1 chain structures (Galβ1-3GlcNAc), which can be further utilized by α1,4-fucosyltransferases. Typical plant N-glycans containing Lewis-A epitopes are illustrated in Figure 1. The Lewis-type β1,3-galactosyltransferase transfers galactose from UDP-α-D-galactose to the β-linked acceptor sugar GlcNAc at the non-reducing terminus of a carbohydrate side-chain of a glycoprotein in plants.
The term "humanization" or "humanized N-glycan structures" refers to steps - inactivation of endogenous plant enzymes and introduction of mammalian enzymes, which lead to the formation of protein linked N-glycans in plants which are more similar or identical to the N-glycans of humans (or mammals) than N-glycans on glycoproteins from wildtype ["non-humanized"] plants.
The term "organism" as used herein refers to all organisms and in particular organisms containing glycoproteins with N-linked glycans.
The term "plant" as used herein refers to a plurality of plant cells which are largely differentiated into a structure that is present at any stage of a plant's development. Such structures include, but are not limited to, a fruit, shoot, stem, root, leaf, silique, seed, flower petal, or similar structure.
The term "plant tissue" includes differentiated and undifferentiated tissues of plants including, but not limited to, roots, shoots, leaves, pollen, seeds, tumor tissue and various types of cells in culture (e.g., single cells, protoplasts, embryos, callus and other types of cells). Plant tissue may be in planta, in organ culture, tissue culture, or cell culture. Similarly, "plant cells" may be cells in culture or may be part of a plant.
The term "transfection" into a cell or "transfected" in the context of nucleic acid (e.g. vectors) is intended to include what the art calls "transformation" or "transduction". Transformation of a cell may be stable or transient. The present invention comprises introduction of vectors under conditions where, on the one hand, there is stable expression, and on the other hand, where there is only transient expression.
The term "transient transformation" or "transiently transformed" refers to the introduction of one or more transgenes into a cell in the absence of integration of the transgene into the host cell's genome. Transient transformation may be detected by, for example, enzyme-linked immunosorbent assay (ELISA) which detects the presence of a polypeptide encoded by one or more of the transgenes. Alternatively, transient transformation may be shown by detecting the activity of the protein (e. g. antigen binding of an antibody) encoded by the transgene (e.g. the antibody gene). The term "transient transformant" refers to a cell which has transiently incorporated one or more transgenes. In contrast, the term "stable transformation" or "stably transformed" refers to the introduction and integration of one or more transgenes into the genome of a cell. Stable transformation of a cell may be detected by Southern blot hybridization of genomic DNA of the cell with nucleic acid sequences which are capable of binding to one or more of the transgenes. Alternatively, stable transformation of a cell may also be detected by the polymerase chain reaction (PCR) of genomic DNA of the cell to amplify transgene sequences. The term "stable transformant" refers to a cell which has stably integrated one or more transgenes into the genomic DNA. Thus, a stable transformant is distinguished from a transient transformant in that, whereas genomic DNA from the stable transformant contains one or more transgenes, genomic DNA from the transient transformant does not contain a transgene.
The term "sequence identity" refers to identity between two sequences, usually amino acid sequences, which can be determined by sequence alignment programs like BLAST, PSI-BLAST (Altschul et al., 1990, J. Mol. Biol. 215: 403-410; Altschul et al., 1997, Nucleic Acids Res. 25: 3389-3402; http://www.ncbi.nlm.nih.gov/blast/) or ClustalW (Chenna et al., 2003, Nucleic Acids Res. 31: 3497-500; e.g. http://www.e-bi.ac.uk/clustalw/). These algorithms calculate the best match for the selected sequences, and line them up so that the identities, similarities and differences can be seen.
The term "stringent condition" relates to a condition that allows the specific pairing of two DNA molecules that is influenced by selection of the temperature and ionic strength of the sample. By stringent conditions, according to the invention conditions are understood which allow for a stringent binding with no significant background. For instance, the DNA molecules are hybridized in 7% sodium dodecyl sulfate (SDS), 0.5M sodium phosphate, pH 7.2, 1mM EDTA, 1% bovine serum albumine at 65°C, and washed with 40mM sodium phosphate buffer, pH 7.2, 1mM EDTA, 1% SDS at 65°C. Higher stringency is e.g. a hybridization in 5 x SSC at 50°C or 5 x SSC at 65°C (very high stringency). To identify genes that share >65% sequence identity a hybridization at low stringency can be performed according to Sambrook et al., 2001.

Hybridizations may be performed in a buffer containing 30% deionized formamide, 0.6M NaCl, 0.04 M sodium phosphate, pH 7.4, 2.5mM EDTA, 1% SDS at 42°C. At the end of the hybridization washing is performed with 2xSSC/0.1%SDS at room temperature and in 2xSSC/0.1%SDS at 55°C.

The invention also discloses a nucleic acid molecule comprising a sequence according to SEQ ID NO: 1 with an open reading frame from base pair 1 to base pair 1932 or being at least 50% identical to the above sequence or hybridizing with the above indicated sequence under stringent conditions, or comprising a sequence which has degenerated to the above nucleic acid sequence due to the genetic code, the sequence coding for a plant protein, which has plant Lewis-type β1,3-galactosyltransferase activity or is complementary thereto. With the knowledge of its activity such a nucleic acid molecule derived from SEQ ID NO: 1 can be used especially for the inactivation or suppression of the plant Lewis-type β1,3-galactosyltransferase activity as well as for overexpression and production of the recombinant enzyme. Complementary sequences can be used either to express the protein or to inhibit expression via RNA binding. To express the enzyme or the RNA a promoter has to be placed at the right position and direction for expression from the (in vivo formed) double strand.

In further embodiments the present invention provides a plant protein having a plant Lewis-type β1,3-galactosyltransferase activity, a nucleic acid encoding the protein, a recombinant DNA containing the DNA, a transformant containing the recombinant DNA, a process for producing a protein having a plant Lewis-type β1,3-galactosyltransferase activity using the transformant and a method for producing a galactose-containing carbohydrate using the transformant. The present invention further relates to a method for inactivation or inhibition of the plant Lewis-type β1,3-galactosyltransferase activity and a method for elimination of the Lewis-A carbohydrate epitope from plant glycoptroteins and plant-produced glycoproteins that are particularly useful as therapeutic agents.

Alternative strategies, which aim at the inactivation of glycosylation steps, that take place earlier and are a prerequisite for β1,3-galactosyltransferase have the disadvantage that they affect not only the formation of the Lewis-A epitope but also change the overall glycosylation machinery of the host plant. For example the inactivation of N-acetylglucosaminyltransferase I leads to the production of only oligo-mannosidic N-linked glycan structures (von Schaewen et al., 1993, Plant Physiol. 102: 1109-1118; Strasser et al., 2005, Biochemical J. 387: 385-391). As a consequence recombinant glycoproteins produced in such plants contain oligo-mannosidic N-glycans and no complex-type N-glycans (Downing et al., 2006, Plant Biotechn. J., 4: 169-181). Such glycans cannot be further modified *in vivo* and represent no improvement towards the humanisation of the plant N-glycosylation pathway, since these oligomannosidic structures are not common on mammalian glycoproteins and thus represent "non-authentic" N-glycosylation. Other alternative strategies to circumvent the attachment of unwanted potentially immunogenic or allergenic sugar-residues to glycoproteins include the retention in the ER (e.g. by adding an ER retention signal; Gomord et al., 2005, Trends in Biotechnology, 23: 559-565) or Golgi bypassing by a direct transport of a recombinant protein from the ER to a protein storage vacuole (Wang et al., 2006, Trends in Biotechn., 24: 147-149). However, all these strategies prevent the formation of N-glycan processing and lead to the formation of oligo-mannosidic N-glycan structures (e.g. Man₇GlcNac₂), which are far from being a mammalian-like N-glycan pattern found on glycoproteins intended for therapeutic use. Besides being non-authentic these oligo-mannosidic structures affect also the quality of the product, e.g. reduced half-life due to rapid uptake by high-mannose receptors present on macrophages and endothelial cells.

For the specific suppression or inactivation of proteins it is best to carry this out at the level of transcription and translation, respectively. For this it is necessary to isolate and characterize the nucleotide sequence which codes for the active protein. It is an object of the present invention to clone and to sequence the whole gene, which codes for a plant Lewis-type β1,3-galactosyltransferase and to prepare vectors comprising this gene or an altered DNA or a DNA derived therefrom, to transfect plants as well as cells thereof with one of these vectors, to produce glycoproteins that do not contain the normally occurring β1,3-linked galactose attached to N-glycans, as well as to provide corresponding methods thereof.

Therefore, the present invention provides a method for elimination of the Lewis-A epitope from glycoproteins produced in plants. To block specifically the formation of the Lewis-A epitope linked to N-glycans without also interfering in other glycosylation steps, solely that enzyme would have to be inactivated which is directly responsible for this specific glycosylation, i.e. the Lewis-type β1,3-galactosyltransferase. This glycosyltransferase would have to be inactivated on purpose or suppressed so that human or other mammalian proteins which are produced in plants or in plant cells, respectively, would no longer contain this potentially immune-reaction-triggering epitope, as has been the case so far. This inactivation (or at least reduction of activity) of Lewis-type β1,3-GalT can be done in many ways on many levels by a skilled man in the art on the basis of the information contained herein. For example, expression (or transcription, translation, etc.) of the Lewis-type β1,3-GalT may be hindered or made impossible by recombinant DNA/RNA technology. On the other hand, the activity of the enzyme may be reduced or abolished by introduction of mutations in the coding sequence of the gene of this protein or by inactivating the enzyme by inhibitors (by specific antibodies of the Lewis-type β1,3-GalT, etc.) The inactivation of the Lewis-type β1,3-galactosyltransferase will also block the transfer of α1,4-linked fucose, since the type 1 chain structure is required as a substrate for the plant Lewis-type α1,4-fucosyltransferase.

Also provided is the use of a nucleic acid molecule comprising a sequence A as defined above for expressing a plant Lewis-type β1,3-galactosyltransferase.

In another embodiment the present invention provides for a nucleic acid molecule comprising a sequence derived from sequence A further comprising a disruption, deletion, insertion and/or substitution mutation, preferably in the galactosyltransferase domain as defined in claim 3, encoding an inactive Lewis-type β1,3-galactosyltransferase, preferably encoded by a sequence as defined in claims 1 to 3. The number of mutant nucleotides is variable and varies from a single one to several deleted, inserted or substituted nucleotides. It is also possible that the reading frame is shifted by the mutation. In such a "knockout gene" it is merely important that the expression of a β1,3-galactosyltransferase is disturbed, and the formation of an active, functional enzyme is prevented. In doing so, the site of the mutation is variable, as long as expression of an enzymatically active protein is prevented. Preferably, the mutation is in the catalytic region of the enzyme which is located in the C-terminal region. The method of inserting mutations in DNA sequences are well known to the skilled artisan, and therefore the various possibilities of mutageneses need not be discussed here in detail. Coincidental mutageneses as well as, in particular, directed mutageneses, e.g. the site-directed mutagenesis, oligonucleotide-controlled mutagenesis or mutagenesis by aid of restriction enzymes may be employed in this instance. Of course, all nucleic acid and protein molecules according to the present invention are referred to herein in isolated form (if not explicitly disclosed otherwise).

Preferably the nucleic acid molecule with the inactive enzyme hybridizes to SEQ ID NO: 1 under stringent conditions. A hybridization reaction in vivo can result in an disruption, deletion, insertion and/or substitution mutation of the endogenous Lewis-type β1,3-galactosyltransferase. Preferably the organism is a plant or plant cell, especially preferred Arabidopsis or tobacco.

Preferably the nucleic acid molecule comprising a disrupted Lewis-type β1,3-galactosyltransferase gene has a disruption by insertion of an exogenous sequence into said gene such that the disruption prevents expression of functional β1,3-galactosyltransferase, wherein the gene, prior to disruption, codes for a plant Lewis-type β1,3-galactosyltransferase with an amino acid sequence of SEQ ID NO: 2 or with at least 50% identity to SEQ ID NO: 2. The exogeneous sequence can be of any size, e.g. 1, 2, 3, up to 10, up to 50, up to 100, up to 200 nucleotides or also up to 5,000 nucleotides or up to 10,000 nucleotides in length, and can be inserted, for example at any position e.g. in the galactosyltransferase domain of SEQ ID NO: 1. Preferably the exogeneous sequence is an transformation marker and a successful transfection can thus be easily detected.

The invention further provides a nucleic acid molecule which codes for a ribozyme which comprises two sequence sections, each of which has a length of at least 10 to 15 base pairs each, which are complementary to sequence sections of an inventive DNA molecule as described above so that the ribozyme complexes and cleaves the mRNA which is transcribed from a natural plant Lewis-type β1,3-galactosyltransferase DNA molecule. The publication by John M. Burke "Clearing the way for ribozymes" (Nature Biotechnology 15: 414-415; 1997) relates to the general mode of function of ribozymes. The ribozyme will recognize the mRNA of the plant Lewis-type β1,3-galactosyltransferase by complementary base pairing with the mRNA. Subsequently, the ribozyme will cleave and destroy the RNA in a sequence-specific manner, before the enzyme is translated. After dissociation from the cleaved substrate, the ribozyme will repeatedly hybridize with RNA molecules and act as specific endonuclease. In general, ribozymes may specifically be produced for inactivation of a certain mRNA, even if not the entire DNA sequence which codes for the protein is known. Ribozymes are particularly efficient if the ribosomes move slowly along the mRNA. In that case it is easier for the ribozyme to find a ribosome-free site on the mR-NA. For this reason, slow ribosome mutants are also suitable as a system for ribozymes (Burke, 1997, Nature Biotechnology; 15: 414-415).

One possible way is also to use a varied form of a ribozmye, i.e. a minizyme. Minizymes are efficient particularly for cleaving larger mRNA molecules. A minizyme is a hammer head ribozyme which has a short oligonucleotide linker instead of the stem/loop II. Dimer-minizymes are particularly efficient (Kuwabara et al., 1998, Nature Biotechnology, 16: 961-965).

If the vector comprising the nucleic acid molecule coding for a ribozyme is transfected, the active ribozyme will be expressed in the host cell. The ribozyme complexes the complementary mRNA sequence of the Lewis-type β1,3-galactosyltransferase at least at a certain site, cleaves this site, and in this manner it can inhibit the translation of the enzyme. In this host cell as well as in cell lines, or optionally, plant, respectively, derived therefrom, Lewis-type β1,3-galactosyltransferase will not be expressed.

A further aspect of the invention relates to a biologically functional vector which comprises one of the above indicated nucleic acid molecules, preferably DNA, as well as the method of using the vector to transfect an organism, preferably suitable to transfect a plant or a plant cell. For transfection into host cells, an independent vector capable of amplification is necessary, wherein, depending on the host cell, transfection mechanism, task and size of the nucleic acid molecule, a suitable vector can be used. Since a large number of different vectors is known, an enumeration thereof would go beyond the limits of the present application and therefore is done without here, particularly since the vectors are very well known to the skilled artisan (as regards the vectors as well as all the techniques and terms used in this specification which are known to the skilled artisan, cf. also Maniatis). Ideally, the vector has a small molecule mass and should comprise selectable genes so as to lead to an easily recognizable phenotype in a cell so thus enable an easy selection of vector-containing and vector-free host cells. To obtain a high yield of DNA and corresponding gene products, the vector should comprise a strong promoter, as well as an enhancer, gene amplification signals and regulator sequences. For an autonomous replication of the vector, furthermore, a replication origin is important. Polyadenylation sites are responsible for correct processing of the mRNA and splice signals for the RNA transcripts. If phages, viruses or virus particles are used as the vectors, packaging signals will control the packaging of the vector DNA. For instance, for transcription in plants, Ti plasmids are suitable.

If the above described inventive vector is inserted into a plant or into a plant cell, a post-transcriptional suppression of the gene expression of the endogenous Lewis-type β1,3-galactosyltransferase gene is attained by transcription of a transgene homologous thereto or of parts thereof, in sense orientation. For this sense technique, furthermore, reference is made to the publications by Baulcombe 1996, Plant. Mol. Biol., 9: 373-382, and Brigneti et al., 1998, EMBO J., 17: 6739-6746. This strategy of "gene silencing" is an effective way of suppressing the expression of the plant Lewis-type β1,3-galactosyltransferase gene (see also Waterhouse et al., 1998, Proc. Natl. Acad. Sci. USA, 95: 13959-13964).

Furthermore, the invention relates to a biologically functional vector comprising a DNA molecule according to one of the above described embodiments, being inversely orientated with respect to the promoter. If this vector is transfected in a host cell, an "antisense mRNA" will be read, which is complementary to the mRNA of the β1,3-galactosyltransferase and complexes the latter. This bond will either lead to the cleavage of the mRNA or hinder correct processing, transportation, stability or, by preventing ribosome annealing, it will hinder translation and thus the normal gene expression of the Lewis-type β1,3-galactosyltransferase. Although the entire sequence of the DNA molecule could be inserted into the vector, partial sequences thereof because of their smaller size may be advantageous for certain purposes. Different sequence regions of the DNA molecule may be inserted into the vector. One possibility consists, e.g., in inserting into the vector only that part which is responsible for ribosome annealing. Blocking in this region of the mRNA will suffice to stop the entire translation (e.g. microRNAs can suppress translation). A particularly high efficiency of the antisense molecules also results for the 5'- and 3'-untranslated regions of the gene.

For a particularly effective inhibition of the expression of an active Lewis-type β1,3-galactosyltransferase, a combination of the sense technique and the antisense technique is suitable (Waterhouse et al., 1998, Proc. Natl. Acad. Sci., USA, 95: 13959-13964). Especially the use of "double-stranded RNA" (dsRNA), e.g generated from transgenes that produce dsRNA (inverted repeats constructs or hairpin constructs), is suitable for inhibition of the expression of an active Lewis-type β1,3-galactosyltransferase. The potent gene silencing process using dsRNA, known also as RNA interference (RNAi), mainly induces silencing through mRNA breakdown in plants. The design of such gene silencing constructs is well known to the artisan and described in several publications (e.g. Smith et al., 2000, Nature, 407: 319-320; Wesley et al., 2001, Plant J., 27: 581-590; Douchkov et al., 2005, Mol. Plant Microbe Interact., 18: 755-761).

According to a specific embodiment of the invention the inhibition of the Lewis-type β1,3-galactosyltransferase is performed by double-stranded "small interfering RNAs" (siRNAs), which are e.g. delivered biolistically into plants cells (Vanitharani et al., 2003, Proc. Natl. Acad. Sci. USA., 100: 9632-9636). SiRNA molecules or microRNAs are provided which have a base sequence of 10 to 100 base length, preferably 15 to 40 bases, more preferred 18 to 30 bases or double-stranded hairpin-RNA comprising a base sequence of 10 to 1000, preferably 50 to 500, especially 100 to 300 bases of SEQ ID NO: 1 or being complementary to SEQ ID NO: 1. For this purposes also sequences not being 100% identical to SEQ ID NO: 1 also with certain base exchanges silencing is still enabled. For this purpose, accordingly homologous sequences of other organisms can be used. The use of siRNAs mainly leads to cleavage of the target mRNA but can also affect the chromatin structure of the target gene, resulting in transcriptional inhibition (Lippman and Martienssen, 2004, Nature 431: 364-370).

Preferably the inhibition of the Lewis-type β1,3-galactosyltransferase according to the present invention is performed by "microRNAs", which are processed in the nucleus from a precursor, and either lead to microRNA-mediated cleavage of the target mRNA or to microRNA-mediated translation prevention (e.g. reviewed by Bartel, 2004, Cell 116: 281-297).

A further embodiment of the present invention relates to the inhibition of Lewis-type β1,3-GalT being performed by "artificial micro RNAs". The design principle and use for artificial micro RNAs is described in detail by Schwab et al., 2006, Plant Cell 118: 1121-1133.

Preferably, the inhibition of the Lewis-type β1,3-GaltT by these molecules (sense, antisense, dsRNA, siRNAs, microRNAs, artificial microRNAs, etc.) is performed by vectors comprising the respective molecules, whereby the vectors will be used to transform plants or plant cells.

According to another preferred embodiment of the invention the inhibition of the Lewis-type β1,3-galactosyltransferase by these molecules (sense, antisense, dsRNA, siRNAs) is performed by virus comprising the respective molecules (virus induced gene silencing: e.g. Lu et al., 2003, Methods, 30: 296-303).

According to another aspect of the invention a method of reducing or preventing the expression of an endogenous plant Lewis-type β1,3-galactosyltransferase in an organism, preferably a plant or plant cell, is provided comprising the step of (at least transiently) transfecting the organism with a nucleic acid molecule or a vector or treating the organism with a RNA as disclosed herein. The inhibition of the Lewis-type β1,3-galactosyltransferase is e.g. performed by insertional mutagenesis, whereby a T-DNA from Agrobacteria can be inserted into the target gene (Krysan et al., 1999, Plant Cell, 11: 2283-2290) or a transposon element (e.g. Kumar et al., 2005, Plant J., 44: 879-892) can be utilized for insertion into the target gene. The method includes, but is not limited to insertion into an exon of the target gene, into an intron of the target gene or into regulatory sequence, like the promoter region of the target gene. The insertion leads to disruption of the open reading frame or interferes with the regulation of gene expression and thus blocks or reduces the expression of the target gene. For example, RNA according to the present invention can preferably be obtained by using vectors containing an expression cassette. This vector DNA can be transferred into the plant or plant cell and expresses the RNA.

According to a preferred embodiment of the invention the inhibition of the Lewis-type β1,3-galactosyltransferase is performed by insertional mutagenesis either directed or by random insertion into the plant genome.

The inhibition of the Lewis-type β1,3-galactosyltransferase according to the present invention may also be performed by physical or chemical mutagenesis. Physical mutagenesis includes, but is not limited to gamma and fast neutron irradiation of seeds. Chemical mutagenesis includes, but is not limited to treatment of seeds with ethyl-methanesulfonate leading to point mutations, which inactivate glycosyltransferases (Strasser et al., 2005, Biochem. J., 387: 385-391).

According to another preferred embodiment of the invention the inhibition of the Lewis-type β1,3-galactosyltransferase is performed by "Targeting Induced Local Lesions IN Genomes" (TILLING), a method whereby chemical mutagenesis followed by screening for single-base changes to discover induced mutations that alter protein function is used (described by Henikoff et al., 2004, Plant Physiol. 135: 630-636).

According to another aspect of the present invention, there is provided a method of preparing a cDNA comprising the nucleic acid molecule, in particular the DNA of the invention, wherein RNA is isolated from a plant cell, in particular from stem or silique cells, by means of which a reverse transcription is carried out after the addition of a reverse transcriptase and primers. The individual steps of this method are carried out according to protocols known per se. For the reverse transcription, on the one hand, it is possible to produce the cDNA of the entire mRNA with the help of oligo (dT) primers, and only then to carry out a PCR by means of selected primers so as to prepare nucleic acid molecules comprising the Lewis-type β1,3-galactosyltransferase. On the other hand, the selected primers may directly be used for the reverse transcription so as to obtain short, specific cDNA. The suitable primers may be prepared e.g. synthetically according to the pattern of cDNA sequences of the transferase.

The invention furthermore relates to a method of cloning a plant Lewis-type β1,3-galactosyltransferase, characterized in that the nucleic acid molecule of the invention is cloned into a vector which subsequently is transfected into a host cell or host, respectively, wherein, by selection and amplification of transfected host cells, cell lines are obtained which express the active Lewis-type β1,3-galactosyltransferase. The DNA molecule is inserted into the vector by aid of restriction endonucleases. For the vector, there applies what has already been said above. What is important in this method is that an efficient host-vector system is chosen. To obtain an active enzyme, eukaryotic host cells are particularly suitable. One possible way is to transfect the vector in insect cells. In doing so, in particular an insect virus would have to be used as vector, such as, e.g., baculovirus.

Of course, plants or plant cells, human or other vertebrate cells, generally "organisms", can also be transfected, in which case the latter would express an enzyme foreign to them.

Preferably, a method of preparing recombinant host cells, in particular plant cells or plants, respectively, with a suppressed or completely stopped Lewis-type β1,3-galactosyltransferase production is provided, which is characterized in that at least one of the vectors according to the invention, i.e. that one comprising the inventive nucleic acid molecule, the mutant nucleic acid molecule or the nucleic acid molecule coding for ribozymes or the one comprising the nucleic acid molecule in inverse orientation to the promoter, or the one comprising the nucleic acid molecule in sense and antisense orientation is inserted into the host cell or plant, respectively. What has been said above for the transfection also is applicable in this case.

Also provided is the method of producing a plant or plant cells having blocked or reduced expression of endogenous Lewis-type β1,3-galactosyltransferase present in the wildtype plant or plant cell at the transcription or translation level, wherein the plant or plant cell is stably or transiently transfected by a method as mentioned herein. The plant or plant cell is then cultivated. Preferably, the reduction is in amounts of at least 10%, at least 20%, at least 50%, as compared to the wildtype plant or plant cell. Specifically preferred are plant or plant cells which do not exhibit the endogenous Lewis-type β1,3-GalT activity of the wild type plant or plant cell (i.e. a reduction of the endogenous Lewis-type β1,3-GalT activity to under 10%, preferably to under 1%, of the wild type). Even more preferred are plants or plant cells wherein the reduction in Lewis-type β 1,3-GalT activity results in no activity at all (i.e. an activity which is below the detection limit). This may be achieved e.g. with "knock-out" mutants where this gene is completely silenced by methods using recombinant nucleic acid technology.

This method can also be used, to prevent the expression of the Lewis-A epitope in the plant or plant cell which is capable of producing complex N-glycans after the transfection.

As host cells, plant cells may, e.g., be used, wherein, e.g., the Ti plasmid with the agrobacterium system is eligible. With the agrobacterium system it is possible to transfect a plant directly: agrobacteria cause root stem galls in plants. If agrobacteria infect an injured plant, the bacteria themselves do not get into the plant, but they insert the recombinant DNA portion, the so-called T-DNA, from the annular, extrachromosomal, tumor-inducing Ti-plasmid into the plant cells. The T-DNA, and thus also the DNA molecule inserted therein, are installed in the chromosomal DNA of the cell in a stable manner so that the genes of the T-DNA will be expressed in the plant. There exist numerous known, efficient transfection mechanisms for different host systems. Some examples are electroporation, the calcium phosphate method, microinjection and the liposome method. Subsequently, the transfected cells are selected, e. g. on the basis of antibiotic resistances for which the vector comprises genes, or other marker genes. Then the transfected cell lines are amplified, either in small amounts, e.g. in Petri dishes, or in large amounts, e.g. in fermentors. Furthermore, plants have a particular characteristic, i.e. they are capable to redevelop from one (transfected) cell or from a protoplast, respectively, to a complete plant which can be grown. Depending on the vector used, processes will occur in the host so that the enzyme expression will be suppressed or completely blocked: If the vector comprising the DNA molecule with the deletion, insertion or substitution mutation is transfected, a homologous recombination will occur: the mutant DNA molecule will recognize the identical sequence in the genome of the host cell despite its mutation and will be inserted exactly on that place so that a "knockout gene" is formed. In this manner, a mutation is introduced into the gene for the Lewis-type β1,3-galactosyltransferase which is capable of inhibiting the faultless expression of the Lewis-type β1,3-galactosyltransferase enzyme. As has been explained above, with this technique it is important that the mutation suffices to block the expression of the active protein. After selection and amplification, the gene may be sequenced as an additional check so as to determine the success of the homologous recombination or the degree of mutation, respectively.

In case the vector comprises the inventive DNA molecule in sense or inverse direction to the promoter, a sense or antisense-mRNA will be expressed in the transfected cell (or plant, respectively). The antisense mRNA is complementary at least to a part of the mRNA sequence of the Lewis-type β1,3-galactosyltransferase and may likewise inhibit translation of the enzyme or lead to degradation of the target mRNA. As an example of a method of suppressing the expression of a gene by antisense technique, reference is made to the publication by Smith et al., 1990, Mol. Gen. Genet., 224: 477-481, wherein in this publication the expression of a gene involved in the maturing process of tomatoes is inhibited. Double-stranded RNA (dsRNA) has recently been shown to trigger sequence-specific gene silencing in a wide variety of organisms, including plants (for review see Watson et al., 2005, FEBS Letters, 579: 5982-5987).

In all the systems, expression of the Lewis-type β1,3-galactosyltransferase is at least suppressed, preferably even completely blocked. The degree of the disturbance of the gene expression will depend on the degree of complexing, homologous recombination, on possible subsequent coincidental mutations and on other processes in the region of the genome. The transfected cells are checked for Lewis-type β1,3-galactosyltransferase activity and selected.

Moreover, it is possible to still further increase the above described suppression of the expression of the Lewis-type β1,3-galactosyltransferase by introducing into the host a vector comprising a gene coding for a β1,4-galactosyltransferase (e.g. from human or any other source) in addition to the insertion of an above described vector. The attachment of β1,3-galactose may be reduced by the action of other enzymes from any other source, the combination of the inhibition of the expression of an active Lewis-type β1,3-galactosyltransferase by means of the inventive vector and by means of a mammalian enzyme vector being particularly efficient.

Preferably, the transformation of plants, which lack an active Lewis-type β1,3-galactosyltransferase with mammalian glycosylation enzymes includes in particular but not exclusively human β1,4-galactosyltransferase, α2,6-sialyltransferase, α2,3-sialyltransferase, β1,4-N-acetylglucosaminyltransferase III, β 1,4-N-acetylglucosaminyltransferase IV, β1,6-N-acetylglucosaminyltransferase V and β1,4-N-acetylglucosaminyltransferase VI or any combination of these enzymes. Any plant species may be used for transfection, including but not limited to alfaalfa, apple, *Arabidopsis thaliana*, cotton, common bean, maize, mung bean, poplar, potato, rice, soybean, tobacco, grapewine, tomato plant, wheat, barley, Nicotiana benthamiana, lemna, lettuce, spinach, sugarcane, banana, cowpea, papaya, carrot or Brassica.

In a preferred embodiment normal expression of the Lewis-A epitope is prevented in at least one organ or tissue of the plant, and the plant or plant cell comprises a dysfunctional β 1,2-xylosyltransferase and/or a dysfunctional α1,3-fucosyltransferase. The Lewis-type β1,3-galactosyltransferase is at least suppressed, preferably even completely blocked in plants that have reduced amounts of complex N-glycans carrying β1,2-linked xylose and/or core α1,3-linked fucose, preferably those plants even lack completely complex N-glycans with β1,2-linked xylose and/or core α1,3-linked fucose. The generation of such plants has been described (Strasser et al., 2004, FEBS Lett., 561: 132-136).

A specifically preferred embodiment according to the present invention is a plant or plant cell wherein the β1,3-GalT activity is reduced or erased in addition to a reduction or complete inactivation of β1,2-xylosyltransferase and core α1,3-fucosyl-transferase and wherein at least one, preferably at least two, especially at least three mammalian glycosyltransferase activities have been introduced.

In yet another embodiment of the invention the Lewis-A epitope is at least reduced, preferably even completely absent from plants that have in addition reduced amounts of complex N-glycans carrying β1,2-linked xylose and core α1,3-linked fucose, preferably those plants even lack completely complex N-glycans with β1,2-linked xylose and core α1,3-linked fucose. The generation of such plants, which can still produce complex N-glycans with terminal GlcNAc residues, has been described (Strasser et al., 2004, FEBS Lett., 561: 132-136) and the generation of such plants by knockout or gene silencing is known to the skilled artisan.

Provided in another aspect is a transformed plant or plant cell with an increased expression of a Lewis-type β1,3-galactosyltransferase, comprising a DNA molecule with a sequence of an active Lewis-type β1,3-galactosyltransferase as described above.

Alternatively a transformed plant or plant cell with a reduced or no expression of a Lewis-type β1,3-galactosyltransferase is provided, comprising a DNA molecule, a vector or a RNA which interfere with the expression of a Lewis-type β1,3-galactosyltransferase or are used to knock-out the Lewis-type β1,3-galactosyltransferase.

Another advantageous method of producing recombinant host cells, in particular plant cells, or plants, respectively, consists in that the DNA molecule comprising the mutation is inserted into the genome of the host cell, or plant, respectively, in the place of the non-mutated homologous sequence (Schaefer et al., 1997, Plant J.; 11: 1195-1206). This method thus does not function with a vector, but with a pure DNA molecule. The DNA molecule is inserted into the host e.g. by gene bombardment, microinjection or electroporation, to mention just three examples. As has already been explained, the DNA molecule binds to the homologous sequence in the genome of the host so that a homologous recombination and thus reception of the deletion, insertion or substitution mutation, respectively, will result in the genome: Expression of the Lewis-type β1,3-galactosyltransferase can be suppressed or completely blocked, respectively.

Preferably, recombinant plants or plant cells, respectively, are provided which have been prepared by one of the methods described above, their Lewis-type β1,3-galactosyltransferase production being suppressed or completely blocked, respectively. Preferably, their Lewis-type β1,3-galactosyltransferase activity is less than 50%, in particular less than 20% or less than 10%, particularly preferred 0%, of the Lewis-type β1,3-galactosyltransferase activity occurring in natural plants or plant cells, respectively. The advantage of these plants or plant cells, respectively, is that the glycoproteins produced by them do not comprise any or hardly comprise any β1,3-galactose linked to glycoprotein N-glycans and do not comprise any or hardly comprise any Lewis-A type containing N-glycans. If products of these plants are taken up by or delivered to human or vertebrate bodies, there will be no immune reaction due to the plant Lewis-A epitope or due to the β1,3-linked galactose.

Preferably, recombinant plants or plant cells, respectively, are provided which have been prepared by one of the methods described above, their Lewis-type β1,3-galactosyltransferase production being suppressed or completely blocked, respectively. Preferably, their proportion of Lewis-A epitope containing N-glycans is less than 50%, in particular less than 20%, particularly preferred 0%, of the Lewis-A epitope containing N-glycans occurring in natural plants or plant cells, respectively. The advantage of these plants or plant cells, respectively, is that the glycoproteins produced by them do not comprise any or hardly comprise any β1,3-galactose linked to glycoprotein N-glycans and do not comprise any or hardly comprise any Lewis-A type containing N-glycans. Further advantage of these plants compared to other glycosylation mutants (e.g. von Schaewen et al., 1993, Plant Physiol., 102: 1109-1118) is that the glycosylation is not blocked at an early stage in the pathway and complex N-glycans can be formed and elongated by the introduction of mammalian glycosyltransferases as described above (e.g. β1,4-galactosyltransferase, sialyltransferase). Glycoproteins from these plants comprise a N-glycosylation pattern, which is more comparable to mammalian-type glycosylation than glycoproteins from wildtype plants or plant cells.

A further aspect relates to a method of preparing plants or plant cells, respectively, which comprise changes in β1,3-galactose linked to glycoprotein N-glycans due to interfering with the protein targeting and localization of the Lewis-type β1,3-galactosyltransferase within the plant cell, e.g. by retention of the Lewis-type β1,3-galactosyltransferase in the endoplasmic reticulum due to overexpression or elimination of proteins, which are required for proper transport to the site of action (e.g. Golgi apparatus) (e.g. Nilson et al., 1994, 13: 562-574). For example prevention of proper targeting can be achieved by overexpression of the cytoplasmic tail of either the β1,3-galactosyltransferase or of another glycosyltransferase, which interacts with the Lewis-type β1,3-galactosyltransferase (e.g. Milland et al., 2002, J. Biol. Chem., 277: 10374-10378).

The transformed plant or plant cell with increased or reduced expression of the Lewis-type β1,3-galactosyltransferase preferably comprises a nucleotide sequence linked to an exogenous promoter that drives expression in said plant or plant cell, respectively, wherein said nucleotide sequence encodes a functional mammalian galactosyltransferase that is expressed in the plant or plant cell, respectively. The mammalian galactosyltransferase is preferably a β1,4-galactosyltransferase, especially preferred a human β1,4-galactosyltransferase.

Furthermore in another embodiment the transformed plant or plant cell comprises a nucleotide sequence linked to an exogenous promoter that drives expression in said plant or plant cell, respectively, wherein said nucleotide sequence encodes a functional mammalian N-acetylglucosaminyltransferase that is expressed in the plant or plant cell, respectively. Preferably, the mammalian N-acetylglucosaminyltransferase is a β1,2-N-acetylglucosaminyltransferase I or II, preferably a human β1,2-N-acetylglucosaminyltransferase I or II, or a β1,4-N-acetylglucosaminyltransferase III or IV, preferably a human β1,4-N-acetylglucosaminyltransferase III or IV, or a β1,6-N-acetylglucosaminyltransferase V, preferably a human β1,6-N-acetylglucosaminyltransferase V.

Additionally the transformed plant or plant cell may comprise a nucleotide sequence linked to an exogenous promoter that drives expression in said plant or plant cell, respectively, wherein said nucleotide sequence encodes a functional mammalian sialyltransferase that is expressed in the plant or plant cell, respectively. Preferably, the mammalian sialyltransferase is a human or rat α2,6-sialyltransferase or a human or rat α2,3-sialyltransferase.

In another aspect the invention provides a method of producing recombinant mammalian glycoproteins or glycosylated polypeptides, wherein a transformed plant or plant cell expresses the glycoprotein or polypeptide and a plant is transfected with a nucleic acid that encodes the glycoprotein or polypeptide. The plant can be transfected with the nucleic acid encoding the glycoprotein or polypeptide before or after the plant or plant cell is transformed for increased or reduced expression of the Lewis-type β1,3-galactosyltransferase. Preferably the glycoprotein is a human antibody or a fragment thereof, preferably a human immunoglobulin G (IgG) and wherein the fragment is preferably a Fab, Fab', Fab₂ or scFv (single chain variable fragment) fragment.

Provided is the method of preparing recombinant glycoproteins which is characterized in that the inventive, recombinant plants or plant cells, respectively, whose Lewis-type β1,3-galactosyltransferase production is suppressed or completely blocked, respectively, or plants or cells, respectively, in which the Lewis-A epitope formation is partially or completely blocked according to the method of the invention, are transfected with the gene coding for any glycoprotein of interest so that the recombinant glycoproteins are expressed. In doing so, as has already been described above, vectors comprising genes for the desired proteins are transfected into the host plant or host plant cells, respectively, as has also already been described above. The transfected plant cells will express the desired proteins, and they have no or hardly any β1,3-galactose linked to glycoprotein N-glycans. Thus, they do not trigger potentially immune reactions directed against β1,3-galactose linked to glycoprotein N-glycans or against the Lewis-A epitope linked to N-glycans and provide a mammalian-like N-glycosylation, which is preferably for therapeutic proteins. Any proteins may be produced in these systems.

Advantageously, a method of preparing recombinant human glycoproteins is provided which is characterized in that the recombinant plants or plant cells, respectively, whose Lewis-type β 1,3-galactosyltransferase production is suppressed or completely blocked, respectively, or plants or cells, respectively, in which the Lewis-A epitope formation is partially or completely blocked according to the method of the invention, are transfected with the gene that expresses the glycoprotein so that the recombinant glycoproteins are expressed. By this method it becomes possible to produce human or other mammalian proteins in plants (plant cells) which, if taken up by the human or other mammalian body, do not trigger any immune reaction directed against β1,3-galactose linked to glycoprotein N-glycans or against the Lewis-A epitope linked to N-glycans. There, it is possible to utilize plant types for producing the recombinant glycoproteins which serve as food stuffs, e.g. banana, potato and/or tomato.

The tissues of this plant comprise the recombinant glycoprotein so that, e.g. by extraction of the recombinant glycoprotein from the tissue and subsequent administration, or directly by eating the plant tissue, respectively, the recombinant glycoprotein is taken up in the human or other mammalian body.

Preferably, a method of preparing recombinant human glycoproteins for medical or veterinary medical use is provided, wherein the inventive, recombinant plants or plant cells, respectively, whose Lewis-type β1,3-galactosyltransferase production is suppressed or completely blocked, respectively, or plants or cells, respectively, in which the Lewis-A epitope formation is partially or completely blocked according to the method of the invention, are transfected with the gene that expresses the glycoprotein so that the recombinant glycoproteins are expressed. In doing so, any protein can be used which is of medical or veterinary medical interest including, but not limited to antibodies, hormones, growth factors. It is also possible to use non-glycoproteins in which a N-glycosylation site has been introduced (e.g. by recombinant DNA technology) into the wildtype sequence and which therefore have been transformed from non-glycosylated proteins to a glycoprotein ("artificial glycoproteins").

Preferably the glycoprotein or glycosylated polypeptide is selected from the group consisting of human insulin, preproinsulin, proinsulin, glucagon, interferons such as alpha-interferon, beta-interferon, gamma-interferon, blood-clotting factors selected from Factor VII, VIII, IX, X, XI, and XII, fertility hormones including luteinising hormone, follicle stimulating hormone growth factors including epidermal growth factor, platelet-derived growth factor, granulocyte colony stimulating, prolactin, oxytocin, thyroid stimulating hormone, adrenocorticotropic hormone, calcitonin, parathyroid hormone, somatostatin, erythropoietin (EPO), haemoglobin, serum albumin, collagen and enzymes such as beta-glucocerebrosidase and human and non-human proteins selected from amidases, amylases, carbohydrases, cellulase, dextranase, esterases, glucanases, glucoamylase, lactase, lipases, pepsin, peptidases, phytases, proteases, pectinases, casein, whey proteins, soya proteins, gluten and egg albumin. Of course, the present invention is preferably applied on glycoproteins which are native glycoproteins (i.e. which are glycosylated in their physiological or physiologically active form).

Moreover, the present invention relates to recombinant glycoproteins according to a method described above, wherein they have been prepared in plant systems and wherein their peptide sequence comprises less than 50%, in particular less than 20%, particularly preferred 0%, of the β1,3-bound galactose residues occurring in proteins expressed in non-Lewis-type β1,3-galactosyltransferase-reduced plant systems. Naturally, glycoproteins which do not comprise β1,3-linked galactose residues are to be preferred. The amount of the β1,3-linked galactose and the amount of the Lewis-A epitope will depend on the degree of the above described suppression of the Lewis-type β1,3-galactosyltransferase.

Preferably, the invention relates to recombinant human glycoproteins which have been produced in plant systems according to a method described above and whose peptide sequence comprises less than 50%, in particular less than 20%, particularly preferred 0%, of the β1,3-bound galactose residues occurring in the proteins expressed in natural, non-Lewis-type β1,3-galactosyltransferase-reduced, plant systems.
Preferably, the invention relates to recombinant human glycoproteins which have been produced in plant systems according to a method described above and whose peptide sequence comprises less than 50%, in particular less than 20%, particularly preferred 0%, of the Lewis-A epitope occurring in the proteins expressed in natural, non-Lewis-type β1,3-galactosyltransferase-reduced, plant systems.

A particularly preferred embodiment relates to recombinant human glycoproteins for medical or veterinary medical use which have been prepared in plant systems according to a method described above and whose peptide sequence comprises less than 50%, in particular less than 20%, particularly preferred 0%, of the β1,3-bound galactose residues occurring in the proteins expressed in non- Lewis-type β1,3-galactosyltransferase-reduced plant systems.

A particularly preferred embodiment relates to recombinant human glycoproteins for medical or veterinary medical use which have been prepared in plant systems according to a method described above and whose peptide sequence comprises less than 50%, in particular less than 20%, particularly preferred 0%, of the Lewis-A epitope occurring in the proteins expressed in natural, non- Lewis-type β1,3-galactosyltransferase-reduced, plant systems.

The glycoproteins according to the invention may include other bound oligosaccharide units specific for plants, whereby in the case of human glycoproteins they differ from these natural glycoproteins. Nevertheless, by the glycoproteins according to the invention, a slighter immune reaction or no immune reaction at all, respectively, is triggered in the human or other mammalian body, since, as has already been explained in the introductory portion of the specification, the β1,3-linked galactose residue, together with α1,4-linked fucose residues, could lead to immune reactions or cross immune reaction, respectively.

The glycoproteins according to the invention may include other bound oligosaccharide units specific for plants, whereby - in the case of human glycoproteins - they differ from these natural glycoproteins. Nevertheless, by the glycoproteins according to the invention, a better efficacy is triggered in the human body, since, as has already been explained in the introductory portion of the specification, the β1,3-linked galactose residue, together with α1,4-linked fucose residues, are usually not present on mammalian N-linked glycans.

A further aspect comprises a pharmaceutical composition comprising the glycoproteins according to the invention. In addition to the glycoproteins of the invention, the pharmaceutical composition comprises further additions common for such compositions.

These are, e.g., suitable diluting agents of various buffer contents (e.g. Tris-HCl, acetate, phosphate, pH and ionic strength), additives, such as tensides and solubilizers (e.g. Tween 80, Polysorbate 80), preservatives (e.g. Thimerosal, benzyl alcohol), adjuvants, antioxidants (e.g. ascorbic acid, sodium metabisulfite), emulsifiers, fillers (e.g. lactose, mannitol), covalent bonds of polymers, such as polyethylene glycol, to the protein, incorporation of the material in particulate compositions of polymeric compounds, such as polylactic acid, polyglycolic acid, etc. or in liposomes, auxiliary agents and/or carrier substances which are suitable in the respective treatment. Such compositions will influence the physical condition, stability, rate of in vivo liberation and rate of in vivo excretion of the glycoproteins of the invention.

The invention also relates to a method of preparing "plantified" carbohydrate units of human and other vertebrate glycoproteins or other glycoconjugates, wherein UDP-galactose as well as plant Lewis-type β1,3-galactosyltransferase encoded by an above-described DNA molecule are added to a sample that comprises a carbohydrate unit or a glycoprotein, respectively, so that galactose in β1,3-position is transferred by the plant Lewis-type β1,3-galactosyltransferase to the carbohydrate unit or to the glycoprotein, respectively. By the method according to the invention for cloning of plant Lewis-type β1,3-galactosyltransferase it is possible to produce large amounts of purified enzyme. To obtain a fully active transferase, suitable reaction conditions are provided.

The invention also relates to a method of overexpression of the plant Lewis-type β1,3-galactosyltransferase in plants. The overexpression of the plant Lewis-type β1,3-galactosyltransferase leads to increased amounts of Lewis-A epitopes in plants. Overexpression includes but is not limited to expression in tissues, like leaves, which in natural plants have limited amounts of Lewis-A epitopes. Overexpression in plants can be achieved by using a constitutive promoter, which includes but is not limited to the cauliflower mosaic virus 35S (CaMV35S) promoter and the nopaline synthase (nos) promoter, or the use of tissue-specific promoter like for example the seed-specific promoter arcelin5-I from common bean (DeJaeger et al., 2002, Nature Biotechn., 20: 1265-1268). These techniques are well known to the skilled artisan. Overexpression of the Lewis-A epitope can be detected on glycoproteins from plants by immunoblots using JIM84 antibodies (Horsley et al., 1993, Journal of Exp. Botany 44, Supplement: 223-229), which are specific for the plant Lewis-A epitope (Fitchette et al., 1999, Plant Physiol. 121: 333-343) and subsequently by total N-glycan analysis using mass spectrometry (Wilson et al., 2001, Glycobiology 11: 261-274).

The invention also relates to a method of overexpression of Lewis-type β1,3-galactosyltransferase in plants, whereby the Lewis-type β1,3-galactosyltransferase is derived from bryophytes such as Physcomitrella patens. The overexpression of the bryophyte Lewis-type β1,3-galactosyltransferase leads to increased amounts of Lewis-A epitopes in plants. For overexpression the same promoters as described above are suitable.

The invention also relates to a method of overexpression of animal β1,3-galactosyltransferase in plants. The overexpression of the animal (vertebrate or non-vertebrate) β1,3-galactosyltransferase leads to increased amounts of Lewis-A epitopes in plants. Preferably, a mammalian β1,3-galactosyltransferase is expressed in plants, which includes but is not limited to β1,3-galactosyltransferase I, β1,3-galactosyltransferase II, β1,3-galactosyltransferase III, β1,3-galactosyltransferase IV, β1,3-galactosyltransferase V, β1,3-galactosyltransferase VI and β1,3-galactosyltransferase VII (Amado et al., 1999, Biochim Biophys Acta., 1473: 35-53.; Hennet, 2002, Cell. Mol. Life Sci. 59: 1081-1095). In another embodiment of the invention preferably, an invertebrate β1,3-galactosyltransferase is expressed in plants, which includes but is not limited to core 1 galactosyl-transferases (Muller et al., 2005, FEBS J.; 272: 4295-4305). For overexpression the same promoters as described above are suitable. In another embodiment of the invention preferably a bacterial β1,3-galactosyltransferase (Appelmelk et al., 2000, Infect. Immun. 68: 5928-5932; Gilbert et al., 2000, J. Biol. Chem. 275, 3896-3906) is expressed in plants.

The invention also provides a method of producing at least a plant or a plant cell wherein the plant Lewis-type β1,3-galactosyltransferase activity is increased that comprises introducing into the plant or plant cell a nucleic acid sequence encoding a plant Lewis-type β1,3-galactosyltransferase nucleotide sequence, mammalian β1,3-galactosyltransferase nucleotide sequence, or bryophyte Lewis-type β1,3-galactosyltransferase nucleotide sequence.

The invention further relates to a transformed plant or plant cell with increased expression of the Lewis-A epitope, characterized in that the plant or plant cell comprises a mammalian β1,3-galactosyltransferase nucleotide sequence, preferably selected from β1,3-galactosyltransferase 1, β1,3-galactosyltransferase 2 or β1,3-galactosyltransferase 5, a bryophyte Lewis-type β1,3-galactosyltransferase nucleotide sequence or a plant Lewis-type β1,3-galactosyltransferase nucleotide sequence.

The invention will be explained in more detail by way of the following examples and drawing figures to which, of course, it shall not be restricted. In detail, in the drawings,
Fig. 1: shows typical plant Lewis-A type N-glycans and type 1 chain containing N-glycan structures;
Fig. 2: shows a schematic presentation of the plant N-glycosylation pathway;
Fig. 3: shows a schematic presentation of the enzymatic reaction catalysed by a plant Lewis-type β1,3-galactosyltransferase;
Fig. 4: shows the cDNA (SEQ ID NO: 1) encoding a plant Lewis-type β1,3-galactosyltransferase from *A. thaliana.*
Fig. 5: shows the amino acid sequence (SEQ ID NO: 2) of the plant Lewis-type β1,3-galactosyltransferase derived therefrom;

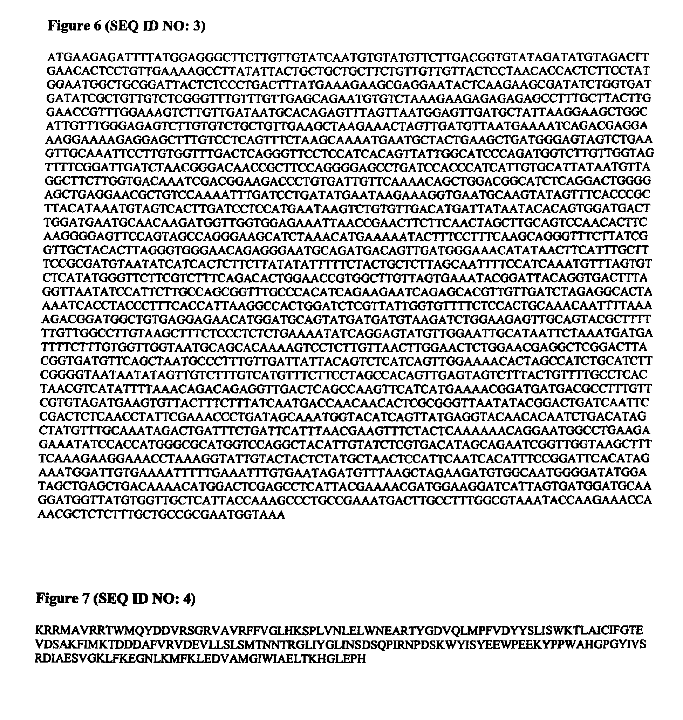

Fig. 6: shows the genomic sequence (SEQ ID NO: 3) of the plant Lewis-type β1,3-galactosyltransferase;
Fig. 7: shows the amino acid sequence (aa 406-597 of SEQ ID No: 2) of the putative catalytic domain (SEQ ID NO: 4) of plant Lewis-type β1,3-galactosyltransferase from *A. thaliana;*
Fig. 8: shows the amino acid alignment of SEQ ID NO: 4 with one homologous sequence from *A. thaliana* (At3g06440) and two rice (Os02g36770, Os06g12390) and one soybean (Gm) sequence;
Fig. 9: shows a table, which displays the amino acid sequence identity between SEQ ID NO: 4 and the predicted catalytic domain of putative plant Lewis-type β1,3-galactosyltransferases;
Fig. 10: shows the RT-PCR amplification product of SEQ ID NO: 1 from *A. thaliana* siliques;
Fig. 11: shows the PCR amplification from genomic DNA isolated from plants transformed with the plant Lewis-type β1,3-galactosyltransferase and RT-PCR from RNA extracted from those plants (Col-0: wildtype);
Fig. 12: shows a comparison by immunoblot analysis of the plant Lewis-type β1,3-galactosyltransferase activity of plants expressing the plant Lewis-type β1,3-galactosyltransferase from *A. thaliana* with that of a control (untransformed wildtype plant: Col-0);
Fig. 13: shows a comparison by mass (mass spectra) of plants expressing the plant Lewis-type β1,3-galactosyltransferase from *A.* thaliana (transgenic plant) with that of a control (wildtype);
Fig. 14: shows the structure of the acceptor substrate (GnGn) and products (A3Gn / GnA3 / A3A3) of the plant Lewis-type β1,3-galactosyltransferase from A. *thaliana* suitable for an assay;
Fig. 15: shows the analysis of the plant Lewis-type β1,3-galactosyltransferase product by mass spectrometry: (A) lower panel: only substrate (GnGn see Fig. 14 for detailed structures), upper panel product after incubation with Lewis-type β1,3-galactosyltransferase; (B) treatment with different β-galactosidases, lower panel: untreated, middle panel treatment with β-galactosidase from Aspergillus oryzae, upper panel: digestion with β-galactosidase from bovine testis;
Fig. 16: shows the analysis of the plant Lewis-type β1,3-galactosyltransferase product by reversed-phase HPLC (A) and normal phase HPLC (B), the additional peak from the normal phase HPLC was collected and analyzed by MALDI-TOF MS (C);
Fig. 17: shows the forward primer for ORF amplification (SEQ ID NO: 5);
Fig. 18: shows the reverse primer for ORF amplification (SEQ ID NO: 6);
Fig. 19: shows the 5' untranslated region (SEQ ID NO: 7) of the Arabidopsis Lewis-type β1,3-galactosyltransferase;
Fig. 20: shows the 3' untranslated region (SEQ ID NO: 8) of the Arabidopsis Lewis-type β1,3-galactosyltransferase;
Fig. 21: shows the 2481 bp promoter region (SEQ ID NO: 9) of the Arabidopsis Lewis-type β1,3-galactosyltransferase;

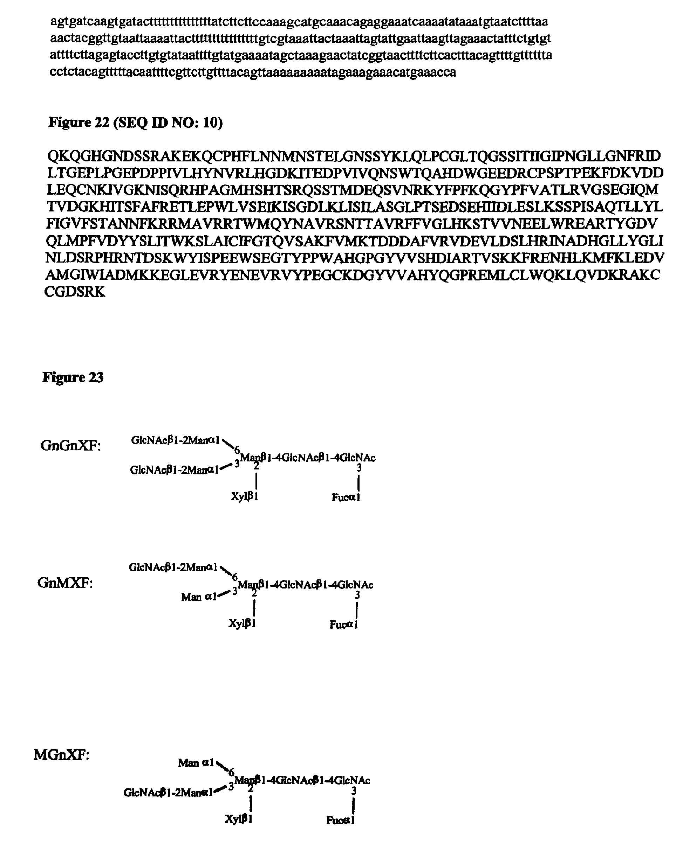

Fig. 22: shows the C-terminal amino acid sequence of a putative soybean (Glycine max) Lewis-type β1,3-galactosyltransferase (SEQ ID NO: 10);
Fig. 23: shows typical complex-type N-glycans structures;
Fig. 24: shows a comparison by immunoblot analysis of the β1,3-galactosyltransferase activity of plants expressing a murine Lewis-type β1,3-galactosyltransferase with that of a control;
Fig. 25: shows the sense cDNA (SEQ ID NO: 26), which is present in the RNAi construct as shown in Fig 27;
Fig. 26: shows the antisense cDNA (SEQ ID NO: 27), which is present in the RNAi construct as shown in Fig 27;
Fig. 27: shows a diagram of the hairpin construct used for gene silencing of Lewis-type β1,3-galactosyltransferase in *A. thaliana* plants;
Fig. 28: shows a semi-quantitative RT-PCR of RNA extracted from transgenic plants expressing the gene silencing construct as shown in Fig. 27;
Fig. 29: shows an immunoblot of transgenic plants expressing the gene silencing construct (A): stems, (B): siliques;
Fig. 30: shows the partial amino acid sequence of a putative tobacco (Nicotiana tabacum) Lewis-type β1,3-galactosyltransferase (SEQ ID NO: 28);
Fig. 31: shows the partial amino acid sequence of a putative Nicotiana benthamiana Lewis-type β1,3-galactosyltransferase (SEQ ID NO: 29);
Fig. 32: shows the amino acid sequences of a putative maize *(Zea mays)* Lewis-type β1,3-galactosyltransferase (SEQ ID NO: 30);
Fig. 33: shows an immunoblot of human transferrin incubated with the plant Lewis-type β1,3-galactosyltransferase and controls.

### Examples:

The identification of the Lewis-type β1,3-galactosyltransferase from plants was hindered by the fact that the epitope was reported absent from the model plant *A. thaliana* (Fitchette-Laine et al., 1997, The Plant J., 12: 1411-1417).

In fact, no publication ever described the measurement of the corresponding Lewis-type β1,3-GalT activity in plant cells or extracts from plants.

In addition to that the enzyme, which adds the fucose in α1-4 linkage to the GlcNAc, α1,4-fucosyltransferase from A. *thaliana*, was expressed in *Pichia pastoris,* but no activity was detected (see Wilson et al., 2001, Biochimica et Biophysica Acta, 1527: 88-96).

Although recently a publication by Leonard et al., 2002 (Glycobiology 12: 299-306) claimed to have shown the presence of the epitope by Western blots in young *A. thaliana* seedlings and that the *A. thaliana* α1,4-fucosyltransferase is an active enzyme, no N-glycan structures carrying Lewis-A epitopes were identified on glycoproteins extracted from A. *thaliana* so far (no MS-data).

In mammals β1,3-galactosyltransferases occur as a gene family (see Hennet, 2002, Cell. Mol. Life Sci. 59: 1081-1095; Table I). The detailed function (e.g. in vivo glycosylation of glycoprotein N-glycans) of these enzymes is not completely known (Holgersson and Löfling, 2006, Glycobiology 16: 584-593).

A gene family exists also in *A. thaliana*. Family GT31 from the CAZY database (http://afmb.cnrs-mrs.fr/CAZY/fam/GT31.html) contains 33 putative galactosyltransferase sequences from A. *thaliana*. 21 of these sequences contain the PF01762 Galactosyl-T domain and could potentially be candidates for the Lewis-type β1,3-GalT (Table II).

The domain structure of the *A. thaliana* Lewis type β1,3-GalT is different from the domain structure of mammalian B3GalTs. Mammalian B3GalTs do not contain any Gal-lectin binding domain (PF00337), which is present in the *A. thaliana* Lewis-type β1,3-GalT (see Tables I and II, appendix 1). Lectin binding domains are very untypical for glycosyltransferases and only found in rare cases (see for example Kubota et al., 2006, J. Mol. Biol. 359: 708-727).

All known mammalian B3GalTs are type II membrane proteins composed of cytoplasmic, transmembrane, stem and catalytic domains as typical for Golgi localised glycosyltransferases. In contrast to that the majority of transmembrane prediction programs predict no transmembrane domain for the *A. thaliana* Lewis-type β1,3-GalT (AGI code: At1g26810).
e.g. Aramemnon database: At1g26810 not predicted as membrane protein
http://aramemnon.botanik.uni-koeln.de/seg view.ep?orgm=0&search=At1g26810&cat=0&term=1
Furthermore At1g26810 is not annotated as a transmembrane domain containing protein:
http://mips.gsf.de/cgi-bin/proj/thal/search gene?code=At1g26810
http://www.tigr.org/tigr-scripts/euk_manatee/shared/ORF_infopage.cgi?db=ath1&orf=At1g26810
http://www.plantenergy.uwa.edu.au/applications/suba/flatfile.php?id=AT1G26810.1
http://www.arabidopsis.org/servlets/TairObject?type=locus&name=A t1g26810

The *A. thaliana* Lewis type β1,3-GalT is not included on the available A. *thaliana* microarrays (e.g. NASCarray.
http://affymetrix.arabidopsis.info/; Genevestigator:
https://www.genevestigator.ethz.ch/at/), which makes analysis of the function more difficult (e.g. development- or organ-specific expression). Co-expression analysis with the α1,4-fucosyltransferase is not possible (e.g. co-response database: http://csbd-b.mpimp-golm.mpg.de/csbdb/dbcor/ath.html#transcor).

**Table I: Mammalian B3GalTs used to search for A. thaliana orthologues. The mouse B3GalT1 (MmB3GalT1) was used instead of the human B3GalT1 (Q9Y5Z6) in all subsequent in silico comparisons, because it was used by us to generate the Lewis-A epitope in vivo in A. thaliana leaves (see example 7). Mouse and human B3-GalT1 have 100% amino acid sequence identity.**

| | | | | | | | |
|---|---|---|---|---|---|---|---|
| | Swiss-prot | Length | MW | PI¹ | TMD² | | Pfam 01762³ |
| | | (aa) | kDa | | No. | aa | (aa) |
| MmB3GalT1 | 054904 | 326 | 37,9 | 9,3 | 1 | 7-26 | 92-283 |
| HsB3GalT2 | 043825 | 422 | 49,2 | 9,5 | 1 | 25-43 | 165-359 |
| HsB3GalT5 | Q9Y2C3 | 310 | 36,2 | 8,8 | 1 | 8-28 | 71-261 |

| | | | | | | | |
|---|---|---|---|---|---|---|---|
| ¹Calculated PI DNAstar software (Lasergene) ²Transmembrane domain (TMD) prediction: TMHMM (http://www.cbs.dtu.dk/services/TMHMM/) ³Pfam domain prediction: PF01762: Galactosyltransferase (http://www.sanger.ac.uk/Software/Pfam/) | | | | | | | |

**Table II: 21 A. thaliana sequences present in CAZY family GT31, which contain the GalT Pfam domain. The members of subfamily 1 (see Fig. 1) are shown underlined, and the Lewis-type β1,3-GalT is shown in bold.**

| AGI code⁵ | GenBank Accession | Swissprot Accession | Length (aa) | MW kDa | PI¹ | TMD² | | Pfam 01762³ (aa) | Pfam 00337⁴ (aa) |
|---|---|---|---|---|---|---|---|---|---|
| | | | | | | No. | aa | | |
| At1g05170 | NM_100395 | | 404 | 45,6 | 9,0 | 1 | 13-35 | 149-347 | - |
| At1g11730 | NM_101045 | | 384 | 43,6 | 9,1 | 1 | 21-43 | 129-327 | - |
| At1g22015 | NM_102051 | | 398 | 45,2 | 8,3 | 1 | 13-32 | 140-338 | - |
| **At1g26810** | **NM_102445** | **Q8L7F9** | **643** | **72,3** | **5,0** | **0** | **-** | **406-597** | **170-363** |
| At1g27120 | NM_102474 | Q8GXG6 | 673 | 77,0 | 7,3 | 1 | 13-32 | 439-633 | 184-393 |
| At1g32930 | NM_103026 | | 399 | 44,6 | 7,2 | 1 | 13-35 | 144-342 | - |
| At1g33430 | NM_103068 | | 395 | 44,7 | 8,8 | 1 | 5-27 | 138-336 | - |
| At1g53290 | NM_104207 | | 346 | 38,7 | 9,0 | 1 | 21-43 | 100-295 | - |
| At1g74800 | NM_106138 | Q8RX55 | 672 | 77,3 | 8,6 | 1 | 27-49 | 437-625 | 190-391 |
| At1g77810 | NM_106430 | | 393 | 44,6 | 7,1 | 1 | 9-31 | 132-336 | - |
| At2g25300 | NM_128087 | | 341 | 39,1 | 8,3 | 0 | - | 133-330 | - |
| At2g26100 | NM_128168 | | 371 | 41,2 | 8,4 | 1 | 46-68 | 76-319 | - |
| At2g32430 | NM_128802 | | 409 | 46,5 | 9,1 | 1 | 20-42 | 154-352 | - |
| At3g06440 | NM_111519 | Q9ASW1 | 619 | 70,6 | 7,1 | 0 | - | 385-580 | 165-343 |
| At3g14960 | NM_112358 | | 343 | 38,7 | 8,0 | 1 | 21-43 | 98-293 | - |
| At4g21060 | NM_118224 | Q9SUA8 | 741 | 84,3 | 8,5 | 1 | 70-92 | 507-696 | 247-461 |
| At4g26940 | NM_118828 | | 407 | 46,0 | 7,3 | 0 | - | 153-351 | - |
| At4g32120 | NM_119364 | | 345 | 39,3 | 8,5 | 0 | - | 132-329 | - |
| At5g53340 | NM_124713 | | 338 | 37,8 | 8,4 | 1 | 13-32 | 124-322 | - |
| At5g57500 | NM_125131 | | 318 | 37,0 | 7,5 | 1 | 12-29 | 83-239 | - |
| At5g62620 | NM_125657 | Q9LV16 | 681 | 77,7 | 8,6 | 1 | 26-48 | 446-637 | 186-400 |

| | | | | | | | | | |
|---|---|---|---|---|---|---|---|---|---|
| ¹Calculated PI (http://www.arabidopsis.org/) ²Transmembrane domain (TMD) prediction: TMHMM (http://www.cbs.dtu.dk/services/TMHMM/) ³Pfam domain prediction: PF01762: Galactosyltransferase (http://www.sanger.ac.uk/Software/Pfam/) ⁴Pfam domain prediction: PF00337: Gal-binding-lectin (http://www.sanger.ac.uk/Software/Pfam/) ⁵Unique gene id: http://mips.gsf.de/proj/thal/db/about/codes.html | | | | | | | | | |

A comparison of Table I and II reveals that the *A. thaliana* Lewis-type β1,3-GalT (At1g26810) is different from the mammalian B3GalTs with respect to length of the protein, PI, number of TMDs and domain structure. There are many other A. *thaliana* proteins which are based on the listed parameters better candidates (e.g. At1g05170, At1g11730, At1g22015, At1g33430, At1g53290,...).

**Table III: Amino acid sequence identities among B3GalT1-like proteins: The full-length coding sequences (Met-Stop) were used in pair-wise BLAST alignments (Matrix: Blossum62) to obtain the identity (similarity) values in % (http://www.ncbi.nlm.nih.gov/blast/bl2seq/).**

| | At1g27120 | At1g74800 | At3g06440 | At4g21060 | At5g62620 | Hs B3GalT5 | Hs B3GalT2 | Mm 83GalT1 |
|---|---|---|---|---|---|---|---|---|
| At1g26810 | 34 (52) | 33 (50) | 43 (59) | 32 (48) | 35 (50) | 28 (46) | 25 (43) | 28 (46) |
| At1g27120 | - | 62 (75) | 34 (51) | 51 (66) | 58 (70) | 25 (44) | 23 (41) | 28 (45) |
| At1g74800 | | - | 34 (52) | 55 (72) | 66 (76) | 22 (41) | 25 (44) | 31 (51) |
| At3g06440 | | | - | 33 (50) | 34 (51) | 29 (49) | 27 (46) | 33 (54) |
| At4g21060 | | | | - | 54 (69) | 27 (45) | 21 (44) | 29 (55) |
| At5g62620 | | | | | - | 27 (45) | 23 (43) | 33 (52) |
| HsB3GalT5 | | | | | | - | 41 (61) | 43 (60) |
| HsB3GalT2 | | | | | | | - | 52 (69) |
| MmB3GalT1 | | | | | | | | - |

**Table IV: Amino acid sequence identities among B3GalT1-like proteins: Only the GalactosylT-domain sequences (pfam01762) of the A. thaliana candidate proteins (see Table II, subfamily 1) and pfam domains of the mammalian B3GalTs (see Table I) were used in pair-wise BLAST alignments to obtain the identity (similarity) values in % (http://www.ncbi.nlm.nih.gov/blast/bl2seg/).**

| | At1g27120 | At1g74800 | At3g06440 | At4g21060 | At5g62620 | Hs B3GalT5 | Hs B3GalT2 | Mm B3GalT1 |
|---|---|---|---|---|---|---|---|---|
| At1g26810 | 40 (61) | 40 (59) | 57 (72) | 39 (59) | 44 (61) | 33 (52) | 30 (48) | 31 (54) |
| At1g27120 | - | 70 (80) | 43 (64) | 62 (79) | 71 (84) | 30 (53) | 27 (48) | 32 (52) |
| At1g74800 | | - | 40 (62) | 60 (75) | 73 (84) | 27 (49) | 27 (46) | 31 (51) |
| At3g06440 | | | - | 41 (64) | 45 (67) | 33 (55) | 29 (50) | 32 (54) |
| At4g21060 | | | | - | 63 (83) | 28 (50) | 23 (45) | 29 (55) |
| At5g62620 | | | | | - | 31 (52) | 25 (48) | 30 (52) |
| HsB3GalT5 | | | | | | - | 48 (67) | 51 (65) |
| HsB3GalT2 | | | | | | | - | 58 (74) |
| MmB3GalT1 | | | | | | | | - |

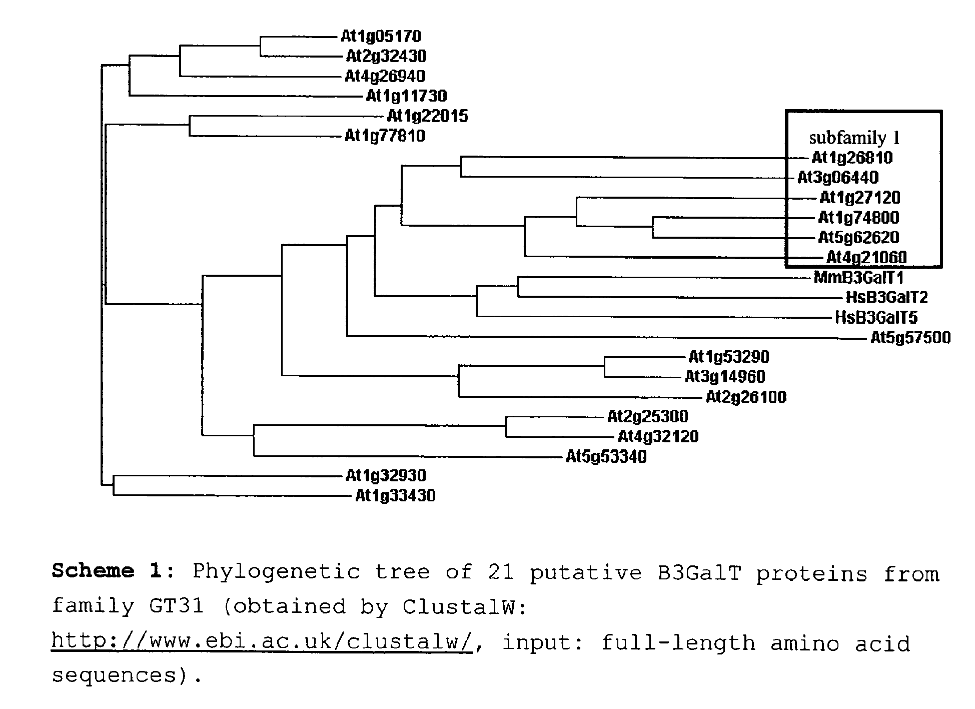

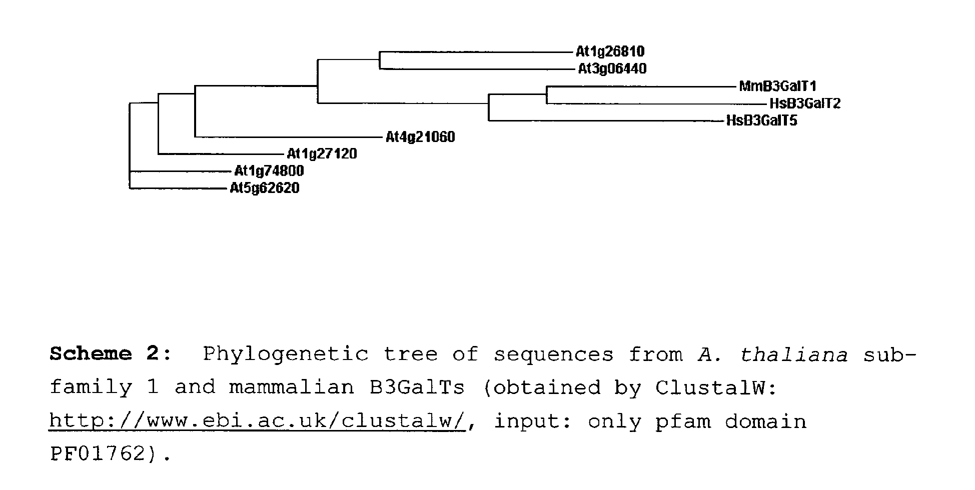

As shown in Scheme 1 *A. thaliana* contains a subfamily (subfamily 1) consisting of six sequences, which display homology to the mammalian B3GalTs. An alignment of the putative galactosyltransferase domain reveals that two sequences (At1g26810 and At3g06440) are closest to the mammalian B3GalTs based on the analysis of the primary amino acid sequence. Interestingly, for both A. *thaliana* proteins no transmembrane domain is predicted. All six members of subfamily 1 contain in addition to the GalT domain a Gal-lectin binding domain (PF00337).

A pairwise BLAST comparison (Tables III and IV) and ClustalW analysis (Scheme 2) shows that the members of A. *thaliana* subfamily 1 share a similar degree of identity to the mammalian B3-GalTs (e.g. 29 - 32 % identity to MmB3GalT1 (Table IV). From these data it cannot be deduced that the At1g26810 protein is the obvious A. *thaliana* Lewis-type β1,3-GalT. The comparison of conserved motifs reveals also no obvious *A. thaliana* Lewis-type β1,3-GalT protein (Table V).

A BLASTP search in the A. *thaliana* database (AGI proteins; http://www.arabidopsis.org/Blast/) with the MmB3GalT1 or HsB3-GalT2 sequences as query lists At1g26810 as the first hit. A search using HsB3GalT5 lists At3g06440 as the first hit and At1g26810 as the second hit. These searches reveal that the E-values of the first six hits (= all six members of *A. thaliana* subfamily 1) are in a similar low range, which makes all six of them good candidates for a Lewis-type β1,3-GalT acting on N-glycan substrates (highly similar results were obtained by FASTA and WU-BLAST2 searches, http://www.arabidopsis.org/).

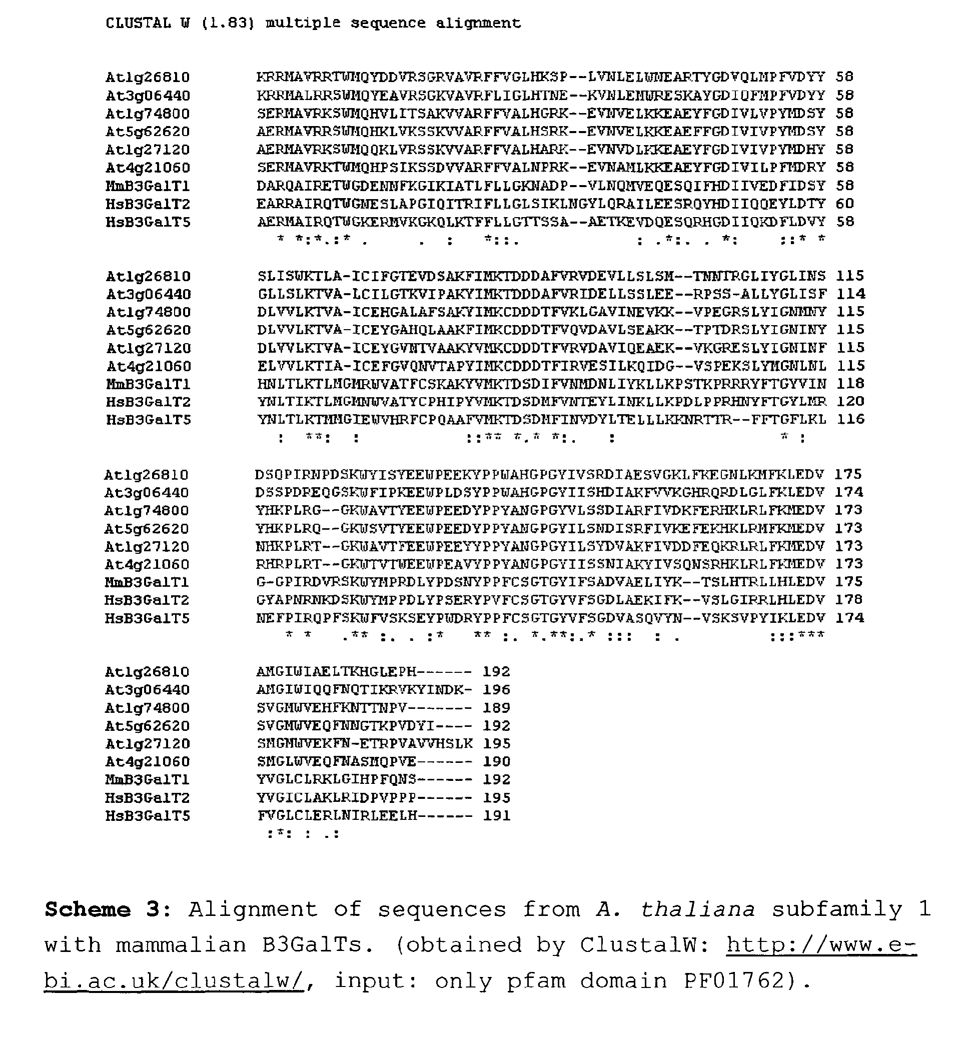

### Comparison A. thaliana and rice sequences

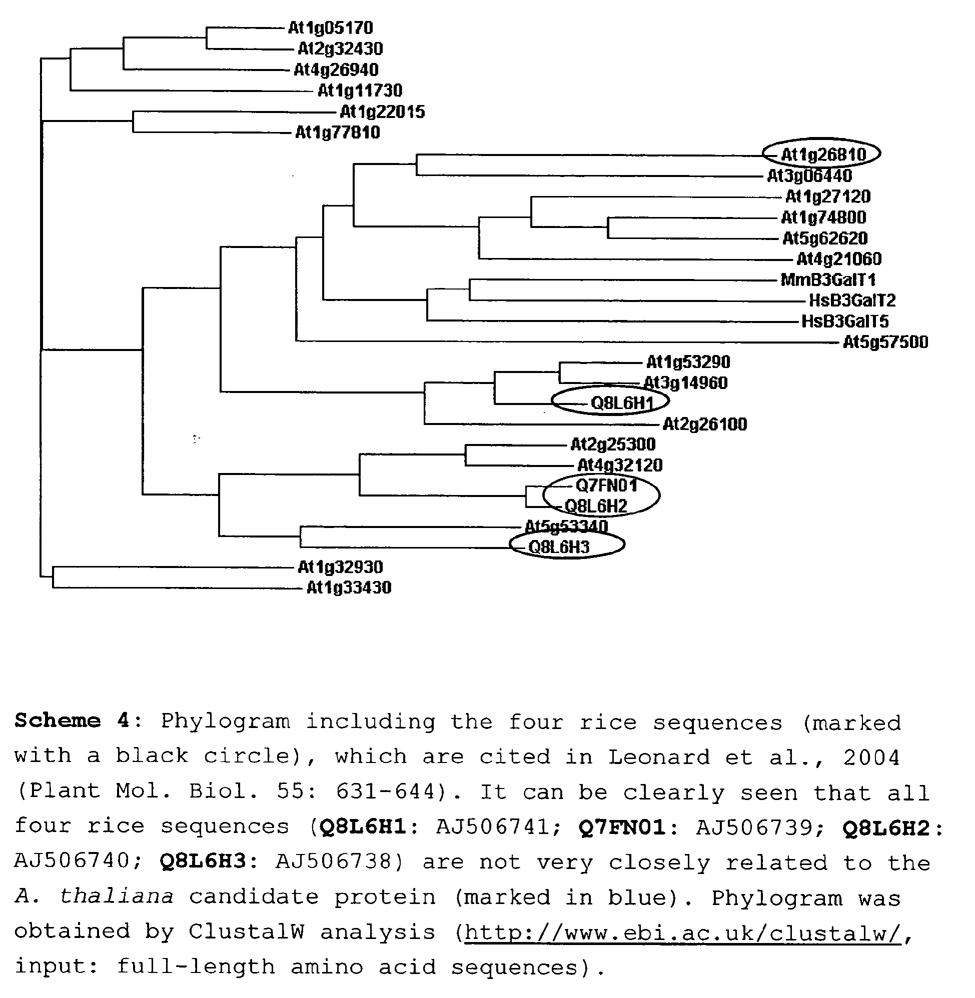

These 4 cited rice sequences could be excluded as Lewis-type β1,3-GalTs:

Other rice sequences (like Q67X5J and Q69JV6, see Scheme 5 and Table V) display a higher sequence identity to the A. *thaliana* Lewis-type β1,3-GalT protein.

The domain structure of the four proteins is different from the *A. thaliana* Lewis-type β1,3-GalT protein. They do not contain any lectin binding domain (PF00337).

Overexpression of *A. thaliana* protein At1g53290, which seems to be an orthologue of rice protein Q8L6H1 in A. *thaliana* leaves, showed no increase in Lewis-A epitope expression.

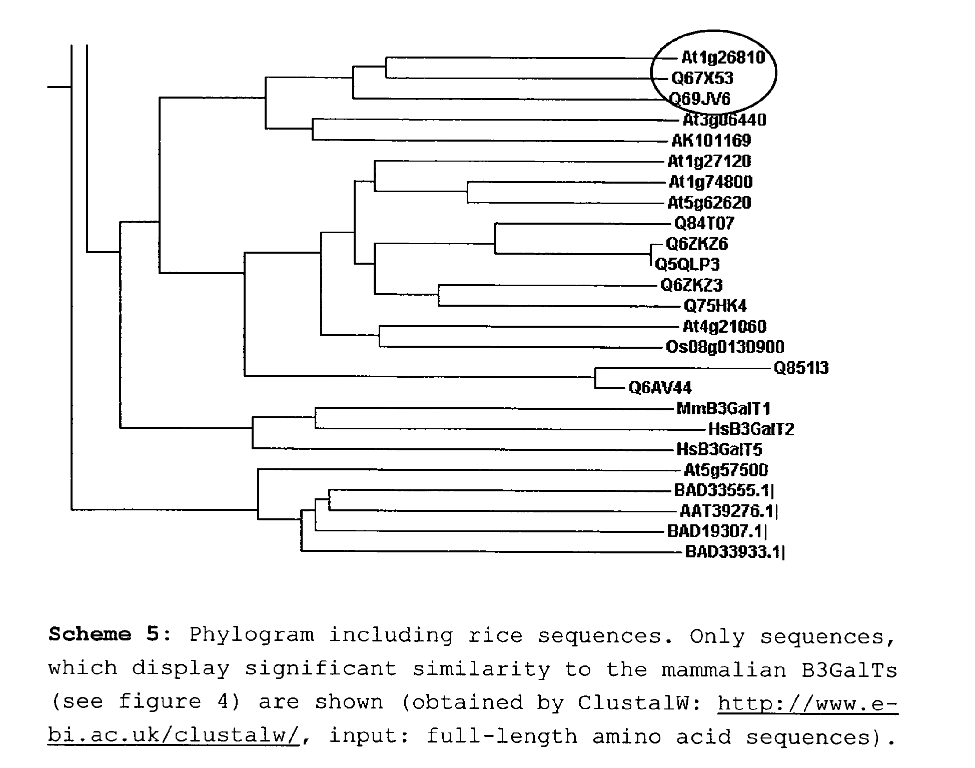

**Table V: Rice Lewis-type β1,3-GalT candidate proteins:**

| Unirprot ID | Genbank Acc. Nr. | Length aa | MW kDa | PI | PF01762 (aa-aa) | PF00337 (aa-aa) | % identity¹ | % similarity¹ |
|---|---|---|---|---|---|---|---|---|
| Q69JV6 | BAD34256 | 621 | 69,0 | 6,22 | 386-582 | 157-343 | 72 | 84 |
| Q67X53 | BAD37266 | 637 | 72,0 | 6,43 | 401-597 | 163-358 | 76 | 86 |

| | | | | | | | | |
|---|---|---|---|---|---|---|---|---|
| ¹The identity and similarity values are obtained by pairwise BLAST alignment of the PF01762 galactosyltransferase domains with the PF01762 domain of At1g26810.http://www.ncbi.nlm.nih.gov/blast/bl2seg/wblast2.cgi) | | | | | | | | |

Q69JV6 and Q67X53 PF01762 domains display 76% identity (87% similarity) to each other.

Based on the sequence identity and predicted domain structure the rice genome could contain two sequences, which represent functional Lewis-type β1,3-GalTs.

### Comparison of A. thaliana and sequences from other species

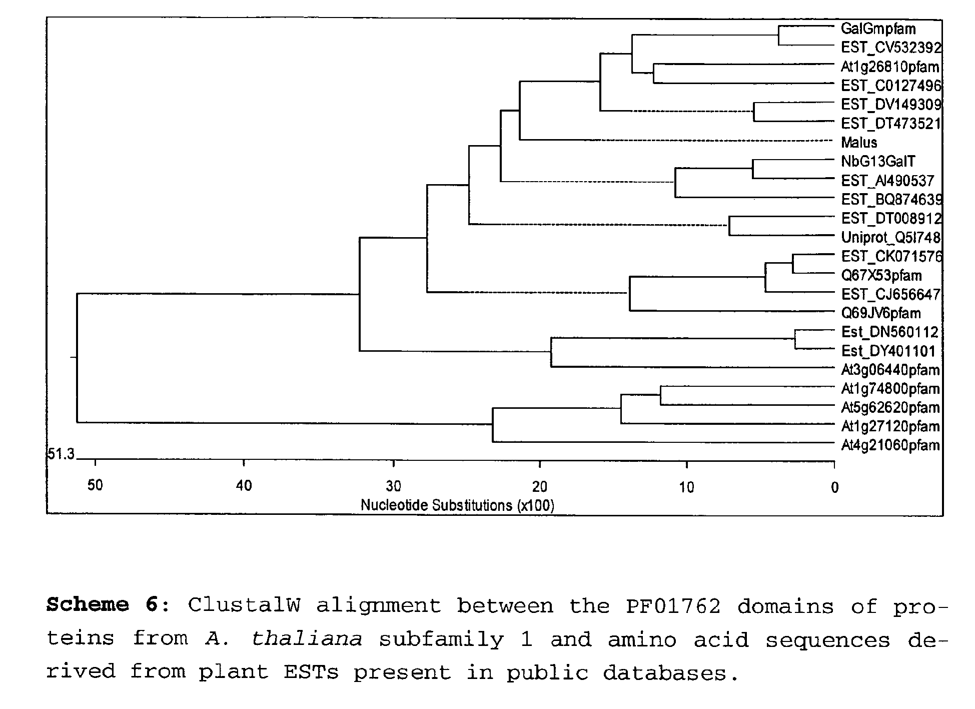

### Sequences with more than 75 % identity (to At1g26810) in the PF01762 domain:

| Name(sequences are listed in appendix 2) species | |
|---|---|
| GalGm | *Glycine max* (soybean) sequence assembled from several ESTs |
| Malus | *Malus domestica* (apple) assembled from several ESTs |
| Nb13GalT | *Nicotiana benthamiana* partial cDNA |

| Genbank accession species | |
|---|---|
| CO127496 | *Gossypium raimondii* (cotton) EST |

### EST sequences with more than 70 % identity to At1g26810 (full-length amino acid sequence):

| Genbank accession species | |
|---|---|
| CV532292 | *Phaseolus vulgaris* (common bean) EST |
| DV149309 | *Euphorbia esula* (leafy spurge) EST |
| DT473521 | *Populus trichocarpa* (poplar) EST |
| AI490537 | *Lycopersicon esculentum* (tomato) EST |
| BQ874639 | *Lactuca sativa* (lettuce) EST |
| DT008912 | *Vitis vinifera* (grapewine) EST |
| Q51748 | *Fortunella hindsii* swissprot (kumquat) partial cDNA |
| CK071576 | *Oryza sativa* indica cultivar-group (rice) partial cDNA |
| CJ656647 | *Triticum aestivum* (wheat) partial cDNA |

An important part of the invention was therefore the surprising results showing unambiguously the presence of N-linked glycans with Lewis-A structures in *A. thaliana* by immunoblots and mass spectroscopy. This result implied the presence of a Lewis-type β1,3-galactosyltransferase in *A. thaliana* and enabled to perform a search in the *A. thaliana* genome for candidate genes. Based on homology searches using mammalian β1,3-galactosyltransferases (Hennet, 2002, Cell. Mol. Life Sci. 59: 1081-1095) it was possible to identify 21 putative candidate genes. The biological function of all of these genes was completely unknown. To identify the plant Lewis-type β1,3-galactosyltransferase the mRNA expression level of the 21 candidate genes was analysed and correlated with the expression pattern of the Lewis-A epitope in different organs of the plants. Based on the data derived from homology searches and mRNA expression transgenic *A. thaliana* plants were generated, which overexpressed the candidate genes in tissues normally lacking the Lewis-A epitope. Screening of the transgenic overexpression lines resulted in the identification of the complete open reading frame (SEQ ID NO: 1) coding for the Lewis-type β1,3-galactosyltransferase (SEQ ID NO: 2) from *A. thaliana*. The β1,3-galactosyltransferase activity was also shown by *in vitro* assays using heterologously expressed recombinant plant Lewis-type β1,3-galactosyltransferase. In the databases SEQ ID NO: 1 was annotated as coding for an "unknown protein" (GenBank Acc. Nr. AAM91658) and as "galactosyltransferase family protein" (GenBank Acc. Nr.: NP_174003), but was neither annotated as β1,3-galactosyltransferase, nor as Lewis-type galactosyltransferase, nor as Lewis type β1,3-galactosyltransferase.

In the rice genome database (http://rgp.dna.affrc.go.jp/) two rice amino acid sequences (GenBank Acc. Nr. BAD37266 and BAD34256), which have more than 50% identity to SEQ ID NO: 2 (based on ClustalW analysis), were classified as "putative beta-1,3-galactosyltransferase". This classification is based on homology search only and is not verified by experimental data. None of the two sequences was classified as a Lewis-type β1,3-galactosyltransferase. Furthermore the *A. thaliana* as well as the rice databases contain other sequences, which have been annotated as either "putative beta-1,3-galactosyltransferase" (e.g. GenBank Acc. Nrs. AAQ77230, AAQ77230) or "beta1,3-galactosyltransferase-like protein" (e.g. GenBank Acc. Nrs.CAD44836, CAD44837, CAD44838, CAD44839). All of these sequences have in common that the sequence identity to SEQ ID NO: 2 is below 50%. None of these sequences have been annotated as a Lewis-type β1,3-galactosyltransferase and no experimental data are shown which describe a corresponding activity.

### Example 1: RT-PCR and cDNA cloning of Lewis-type β1,3-galactosyltransferase

The entire RNA was isolated from siliques of A. *thaliana* ecotype Columbia using the SV Total RNA isolation kit (Promega). RNA was treated with DNase to remove traces of genomic DNA. First-strand cDNA was synthesized from 500 ng of total RNA at 42°C using oligo(dT) primers and AMV reverse transcriptase (Promega).

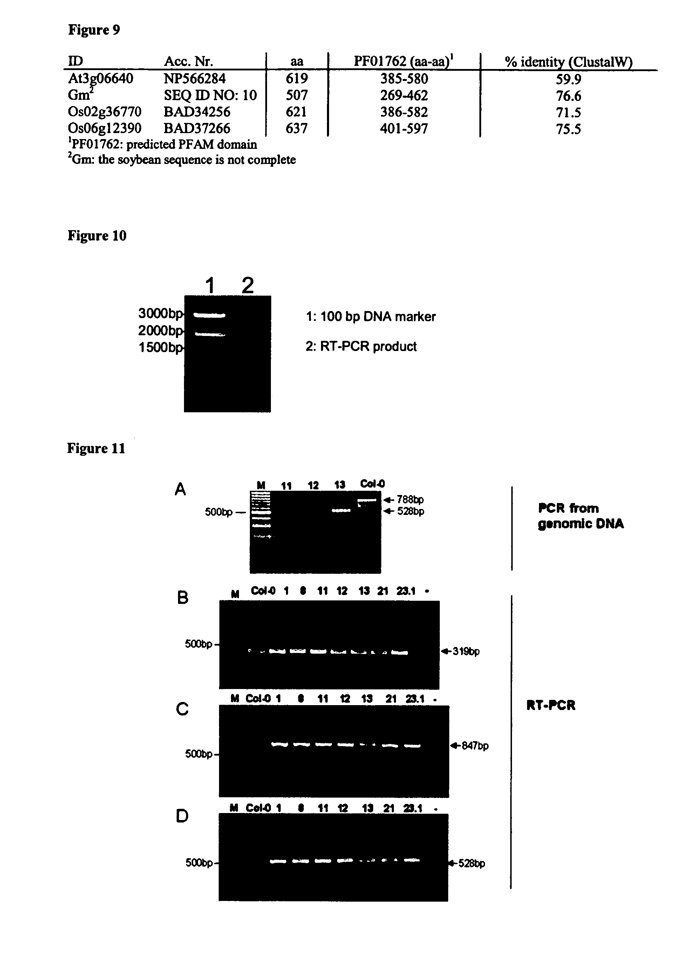

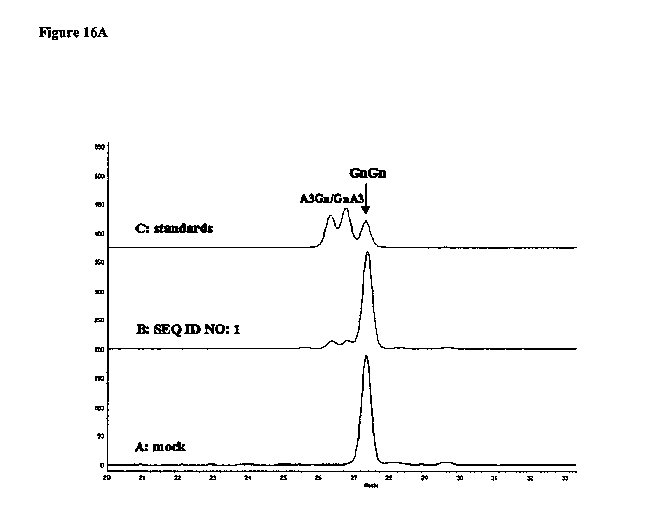

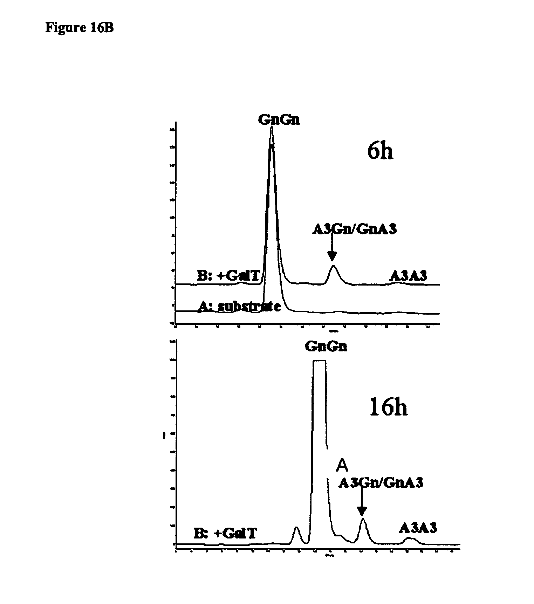

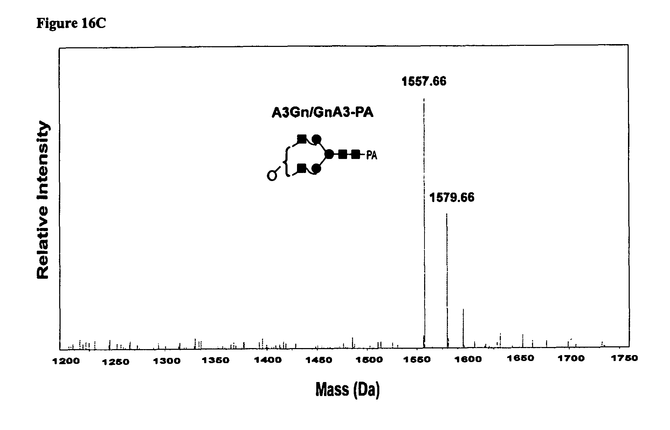

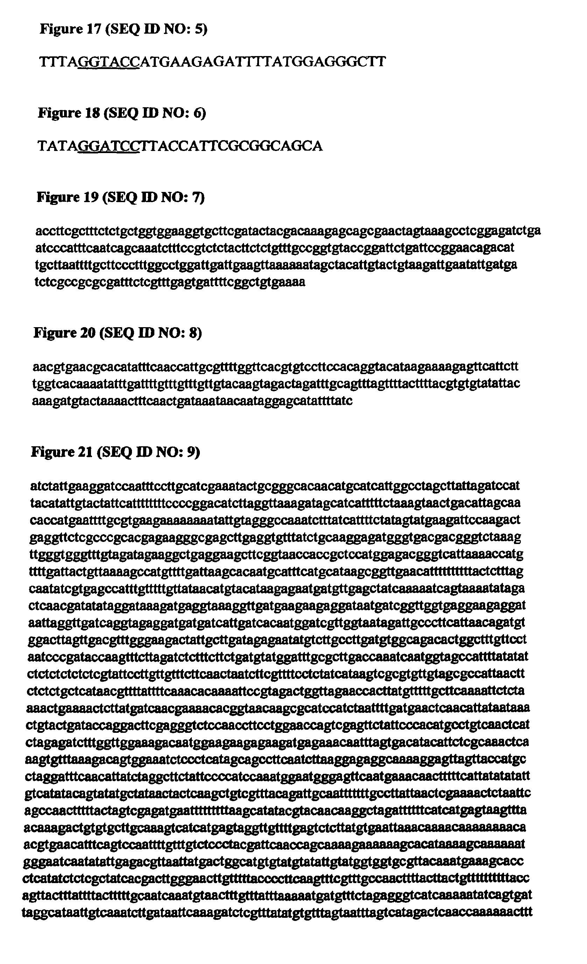

The first strand cDNA was subjected to a PCR, wherein a forward primer (SEQ ID NO: 5; Figure 16), a reverse primer (SEQ ID NO: 6; Figure 17) and a pfu-Polymerase (Stratagene) containing a proofreading activity were used. The primers for amplification contained KpnI and BamHI restriction enzyme cleavage sites to facilitate subsequent cloning steps. The amplified PCR product was a DNA fragment comprising the putative open reading frame and additional base pairs for the cleavage sites on each end (Figure 10, lane 2). Subsequently the PCR product was purified from an agarose-gel using a gel extraction kit (Promega), subcloned into a Zero Blunt Topo-cloning vector (Invitrogen) and sequenced by means of the didesoxynucleotide method (BIG Dye termination cycle sequencing kit, Applied Biosystems). The sequence of the amplified open reading frame has a size of 1932 bp (SEQ ID NO: 1) and codes for a protein of 643 amino acids (SEQ ID NO: 2) having a calculated molecular weight of 72.3 kDa and a theoretical pI value of 5.0. Apart from a base pair change (at position 1206: "G" instead of "A") SEQ ID NO: 1 is identical to the annotated genomic sequence (SEQ ID NO: 3) after removing of 6 introns. The nucleotide change from G to A does not lead to a change of the respective amino acid (GCA and GCG code for alanine).

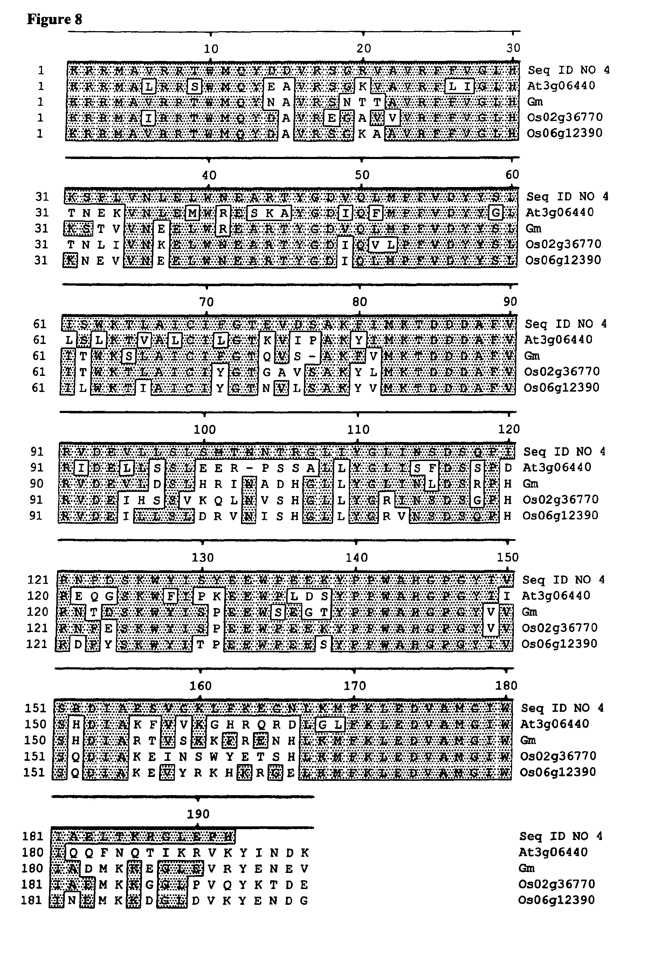

Comparison of SEQ ID NO: 2 with other amino acid sequences from A. *thaliana* reveals no protein with identity of more than 50%. Comparison of SEQ ID NO: 2 with sequences from rice reveals two proteins with more than 50% identity. A BLAST search in databases accessible via NCBI
(http://www.ncbi.nlm.nih.gov/BLAST/) or present in the Plant Genome DataBase (http://www.plantgdb.org/) showed sequences homologous to SEQ ID NO: 2 in other plant species (e.g. tomato: AI490537; onion: CF436881; soybean: BM085070; potato: BG599392; tobacco: EB682269; cotton: CO127496; bean: CV532292; lettuce: BQ874639; poplar: DT473521; wheat: CJ656647). An alignment of amino acid sequences homologous to SEQ ID NO: 4 is shown in Figure 8. The alignment was done using ClustalW (http://www.e-bi.ac.uk/clustalw/) and residues, which are identical to SEQ ID NO: 4 are boxed and shaded. For each protein the aligned sequence stretch corresponds to the putative "galactosyltransferase domain" (PF01762) as predicted by the Pfam protein family database (http://www.sanger.ac.uk/Software/Pfam/Sanger; Bateman et al., 2004, Nucleic Acids Res., Database Issue 32: D138-D141; see also Figure 9).

### Example 2: Expression of recombinant plant Lewis-type β1,3-galactosyltransferase in leaves of A. thaliana plants

The entire coding region for the assumed Lewis-type β1,3-galactosyltransferase (SEQ ID NO: 1) was removed from the Topo-cloning vector by KpnI and BamHI digestion and subcloned into KpnI/BamHI digested and dephosphorylated plant expression vector pPT2 (Strasser et al., 2005, Biochem. J. 387: 385-391). Correct cloning was confirmed by sequencing. In the vector pPT2, the recombinant Lewis-type β1,3-galactosyltransferase protein will be expressed from the strong constitutive cauliflower mosaic virus 35S promoter. The *Agrobacterium tumefaciens* strain UIA143 containing the pMP90 plasmid (Hamilton, 1997, Gene, 200: 107-116) was used to transform *A. thaliana* ecotype Columbia plants by the floral dip procedure (Clough and Bent, 1998, The Plant J. 16: 735-743). Seeds were selected on MS-medium (Sigma) (Murashige and Skoog, 1962, Physiol. Plant 15: 473-497) containing 100 mg/l kanamycin and subsequently transferred to soil. Genomic DNA was isolated by following the procedure of the GenElute Plant Genomic DNA Purification Kit (Sigma).

The integration of the heterologous SEQ ID NO: 1 sequence was confirmed by PCR from genomic DNA using forward primer 5 '-AAGGCCACTGGATCTCGTTATTG-3' (SEQ ID NO: 13) and reverse primer 5'-TTACCAACCGATTCTGCTATGTCAC-3' (SEQ ID NO: 14), which amplify a 528 bp fragment from SEQ ID NO: 1 (Figure 11, panel A). This PCR-fragment is derived from the integrated transgene since a PCR-product from the endogenous gene SEQ ID NO: 3 results in a larger 788 bp product due to the presence of an intron. To confirm the expression of the integrated transgene corresponding to SEQ ID NO: 1 RNA was isolated from leaves of transgenic plants using the SV Total RNA isolation kit (Promega). Total RNA was treated with DNAse (Promega) to remove any traces of genomic DNA. cDNA was made from 500 ng of total RNA using oligo(dT) primers and AMV-reverse transcriptase (Promega) in a total volume of 20µl. To analyse the quality of the cDNA a control reaction was performed using forward primer 5'-GGTGCTCGGAATTTTCGTC-TACA-3' (SEQ ID NO: 15) and reverse primer 5'-TCATTCTCTTTG-CAACTGGCTCTA-3' (SEQ ID NO: 16), which amplify a 319 bp fragment of the α1,3-fucosyltransferase B cDNA (Figure 11, panel B). This fragment is detectable in *A. thaliana* wildtype plants and in transgenic lines containing the stable integration of the SEQ ID NO: 1 T-DNA construct. To detect the expression of the transcript derived from the transgene in the transgenic plants the forward gene-specific primer SEQ ID NO: 13 and a reverse pPT2 vector-specific primer 5'-ATTGCCGTAGATGAAAGACTGA-3' (SEQ ID NO: 17) (Figure 11, panel C) were used for PCR. As a result a fragment of the expected size of 847 bp was detected in all transgenic lines. In the wildtype control plant, which is not transgenic, no corresponding transcript could be detected. In addition to the amplification of the cDNA with primers SEQ ID NO: 13 and SEQ ID NO: 14 resulted in the detection of increased amounts of product (SEQ ID NO: 1 expression) compared to wildtype plants (Figure 11, panel D).

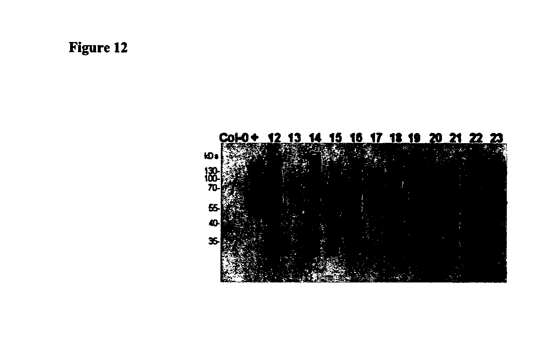

In order to detect changes in the proportion of glycoproteins carrying Lewis-A epitopes leaves from transgenic plants and non-transgenic plants (wildtype plants ecotype Columbia) were analysed by immunoblot and the intensity of the signal was compared. Leaves of *A. thaliana* wildtype plants are very much suitable for that screening approach since they only contain trace amounts of proteins having Lewis-A epitopes (Fitchette-Laine et al., 1997, The Plant J. 12: 1411-1417; Figure 12). Leaves were ground in liquid nitrogen and proteins were extracted in extraction buffer containing 10 mM Tris pH 8.0, 1 mM EDTA and 1 % (w/v) SDS.

Plant material was ground in liquid nitrogen, resuspended in phosphate-buffered saline buffer (137 mM NaCl, 2.7 mM KCl, 10mM Na2HP04, 2 mM KH2PO4, pH 7.4) supplemented with 1% Triton X100 (10 µl per mg of plant material) and cleared by centrifugation (two times 3 min at 16 000g). The protein content in the samples was determined using the BCA protein assay kit (Pierce) and bovine serum albumin as a standard.

10 µg of each sample were mixed with SDS-polyacrylamide gel electrophoresis (PAGE) loading buffer, denatured at 95°C for 5 min and subjected to 12.5% SDS-PAGE under reducing conditions. Separated proteins were blotted onto Hybond enhanced chemiluminescence (ECL) nitrocellulose membranes (Amersham Biosciences). The blot was blocked in 3% (w/v) bovine serum albumin in phosphate-buffered saline buffer for 1 h and incubated in a 1:40 dilution of the rat monoclonal JIM84 antibody (Horsley et al., 1993, Journal of Exp. Bot. 44, Supplement: 223-229) in phosphate-buffered saline buffer supplemented with 0.1% (v/v) Tween 20. In plants the JIM84 antibody recognizes specifically the Lewis-A epitope on glycoproteins (Fitchette et al., 1999, Plant Physiol. 121: 333-343). The detection was performed after incubation in a 1:20000 dilution of a horseradish peroxidase-conjugated goat anti-rat IgM antibody (Jackson) in phosphate-buffered saline buffer with 0.1% (v/v) Tween 20 with Supersignal West Pico chemiluminescent substrate (Pierce). As shown in Figure 12 a clear signal is detected in transgenic plants (#12-#23), which overexpress the plant Lewis-type β1,3-galactosyltransferase in *A. thaliana* leaves. In contrast to that the control (Col-0) does not show any Lewis-A epitope.

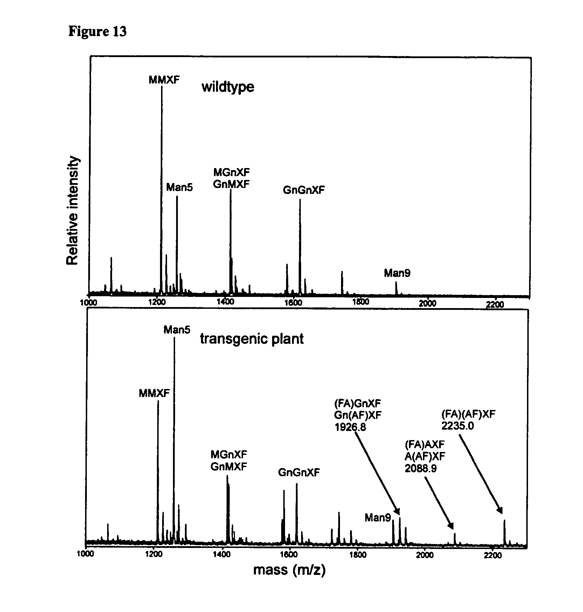

To confirm the increase of the Lewis-A epitope containing N-glycans in the transgenic plants plant material (leaves, stems, siliques) was subjected to total N-glycan analyses performed according to Kolarich and Altmann, 2000 (Anal. Biochem. 285: 64-75). Figure 13 shows mass spectra of N-glycans isolated from wildtype leaves compared to leaves from transgenic plants. The presence of galacotsylated peaks in the transgenic plant is marked by arrows.

### Example 3: Expression of recombinant plant Lewis-type β1,3-galactosyltransferase in insect cells

The coding region of the assumed plant Lewis-type β1,3-galactosyltransferase lacking the putative cytoplasmatic and transmembrane region was amplified by PCR from the Topo-cloning vector using the forward primer 5'- TATACTGCAGAAGCCTTATATTACT-GCTGCT -3' (SEQ ID NO: 11) and the reverse primer 5'- TAAAG-GTACCTTACCATTCGCGGCAGCAAAGAG-3' (SEQ ID NO: 12), digested with PstI and KpnI and cloned into PstI / KpnI digested and dephosphorylated baculovirus transfer vector pVTBacHis1 (Sarkar et al., 1998; Glycoconj. J. 15: 193-197). After the final cloning step, the expression construct was subjected to DNA sequencing to rule out any artifactual mutation. DNA sequencing was performed in a thermocycler using the BigDye Terminator 3.1 Cycle Sequencing kit and a Prism 3100 genetic analyzer (Applied Biosystems). Sequence data were obtained using gene-specific or vector derived sense and antisense oligonucleotides as the primers.

Spodoptera frugiperda Sf9 and Sf21 cells (both obtained from A.T.C.C., Manassas, VA, U.S.A.) were grown in IPL-41 (Sigma, St. Louis, MO, U.S.A.) medium containing 5% (v/v) heat inactivated fetal bovine serum (Invitrogen). Each recombinant baculovirus transfer vector (1 µg) was co-transfected with 200 ng of Baculo-Gold viral DNA (BD PharMingen, Erembodegem, Belgium) into Sf9 cells using Lipofectin (Invitrogen) according to the manufacturer's instructions. After maintaining for 5 days at 27°C, the supernatant containing recombinant baculovirus was used for the infection of Sf21 cells, which were cultivated in IPL-41 medium containing 5% (v/v) heat inactivated fetal bovine serum. After 4 days at 27°C cells and conditioned media were harvested. Cells were washed 2x with phosphate-buffered saline solution and resuspended in the following buffer (1 ml per 10⁷ cells): 100 mM MES buffer, pH 7.0, with 1 % Triton X-100, 1 mM DTT, 1 mM PMSF, 5 µg/ml Leupeptin (Sigma), 5 µg/ml E-64 (Serva) and incubated on ice for 30 min.

### Example 4: Assay for the plant Lewis-type β1,3-galactosyltransferase

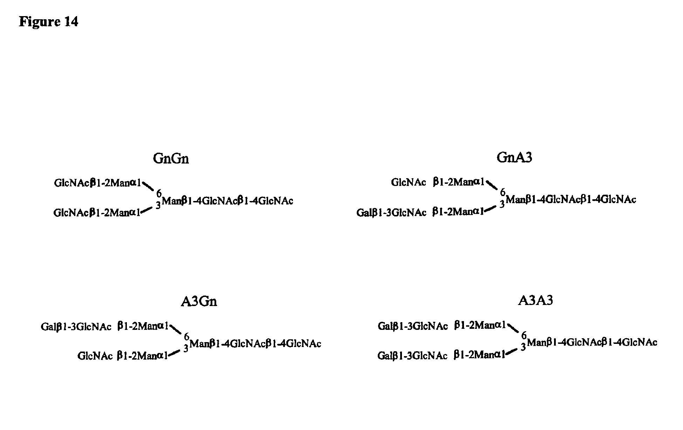

Recombinant expressed plant Lewis-type β1,3-galactosyltransferase was purified from the medium of Sf9 insect cells infected with recombinant virus harboring the plant enzyme using a column of chelating Sepharose (Amersham Biosciences) charged with Ni2+ ions (according to Bencur et al., 2005, Biochem. J. 388: 515-525). Purified plant Lewis-type β1,3-galactosyltransferase protein was assayed using a dabsylated glycopeptide substrate and analyzed by MALDI-TOF MS (as described in Strasser et al., 2000, FEBS LETT. 472: 105-108). Briefly, the assay mixture contained, in a total volume of 20 µl, 2 µg of purified protein, 2 nmol dabsylated GnGn-peptide as acceptor substrate (Figure 14), 1 mM UDP-galactose as donor substrate, 10 mM ATP, 20 mM MnCl2 and 1mM 2-acetamido-1,2-dideoxy-nojirimycin was included to prevent degradation of the product by N-acetylglucosaminidase. The samples were incubated for 3 and 16 hours at 30°C and analyzed by MALDI-TOF mass spectrometry. Protein purified from mock infected insect cells was analyzed as a negative control.

### Example 5: Mass spectrometry of the plant Lewis-type β1,3-galactosyltransferase product

Mass spectrometry was performed on a DYNAMO (BioAnalysis, Santa Fe, NM), a MALDI-TOF MS, which is capable of dynamic extraction (synonym for late extraction). After addition of 2% 2,5-dihydroxy benzoic acid, the samples containing dabsylated glycopeptides were immediately dried by applying vacuum.

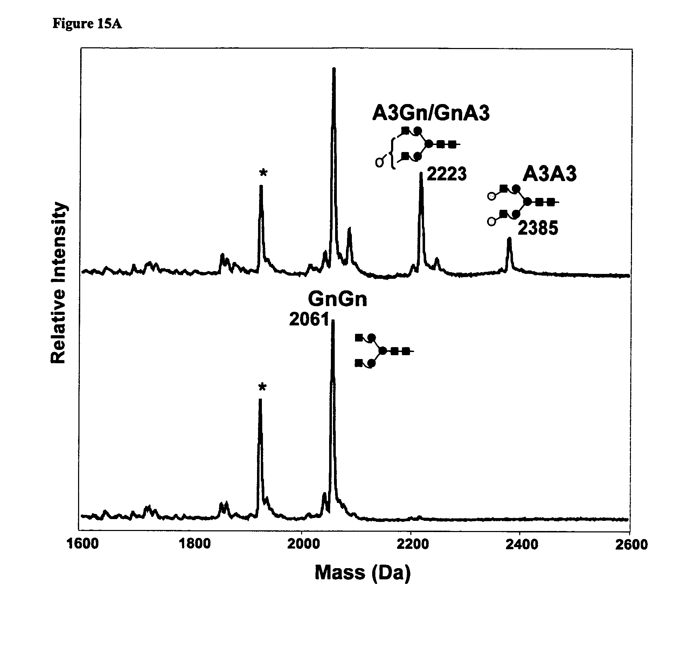

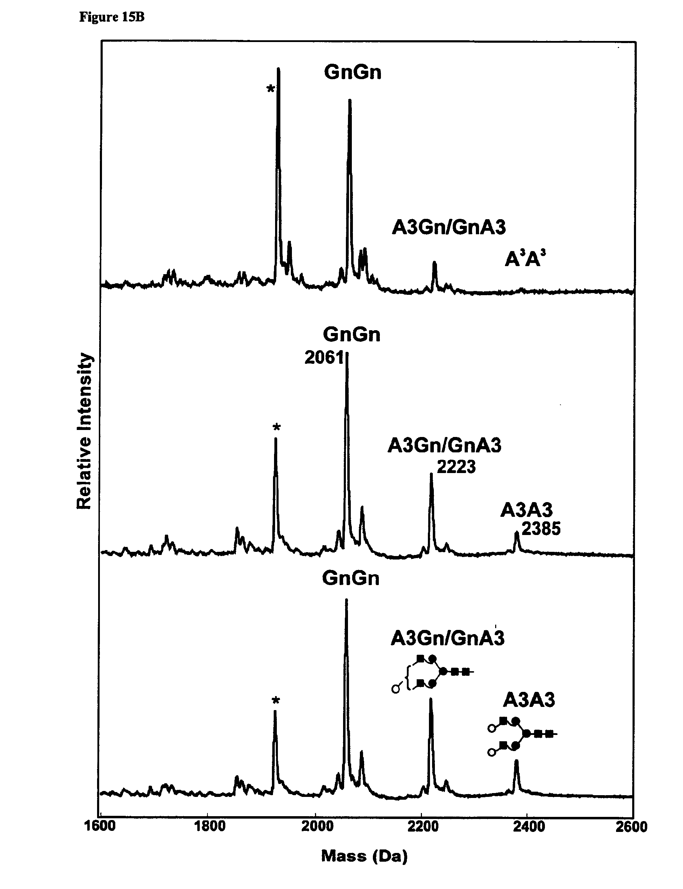

Figure 15A shows the measured enzyme activity of the recombinant Lewis-type β1,3-galactosyltransferase as well as of the negative control, when dabsylated GnGn-peptide was used as a substrate. The detected increase in mass (162 Da for a single galactose residue) corresponds well with the expected value, which confirms the cloning of an active galactosyltransferase. To rule out that the galactose was added in β1,4-linkage to the glycopeptide, the galactosylated GnGn-peptide was incubated overnight with *Aspergillus oryzae* β-galactosidase, which was shown to remove β1,4-linked and β1,6-linked galactose (Zeleny et al., 1997, Anal. Biochem. 246: 96-101). As shown in Figure 15B the galactosylated peak is resistant to *Aspergillus oryzae* β-galactosidase treatment. In contrast to that, treatment with bovine testes galactosidase, which removes both β1,3- and β1,4-linked, yielded again a GnGn-peptide and incubation with β1,3-galactosidase (New England Biolabs) resulted in the removal of the terminal galactose residues. These data show the presence of terminal β1,3-linked galactose.

### Example 6: HPLC-analysis of the plant Lewis-type β1,3-galactosyltransferase product

Galactosyltransferase assays were performed as described above under example 4 using the recombinant plant Lewis-type β 1,3-galactosyltransferase except that 10 nmol of GnGn-pyridylamine (GnGn-PA) were used as the acceptor substrate. After 20 h of incubation the sample was analyzed by normal-phase and reverse-phase HPLC. Figure 16 shows the measured enzyme activity of the recombinant Lewis-type β1,3-galactosyltransferase as well as of the negative control,
when GnGn-pyridylamine was used as a substrate. Normal-phase and reverse-phase HPLC data are shown. A3Gn-PA/GnA3-PA standards were derived from bovine fibrin.

The presumed product peak eluting slightly after the substrate GnGn-PA was collected (Figure 16B). By MALDI-TOF MS the product's mass was determined to be 1557.66 which is in good agreement with being A3Gn-PA/GnA3-PA (Figure 16C). Upon digestion with β1,3-galactosidase (New England Biolabs) in 50 mM sodium citrate buffer of pH 4.5 for 20 h at 37°C the glycan eluted with about the retention of GnGn-PA.

### Example 7: Expression of a recombinant mammalian Lewis-type β1,3-galactosyltransferase in leaves of A. thaliana

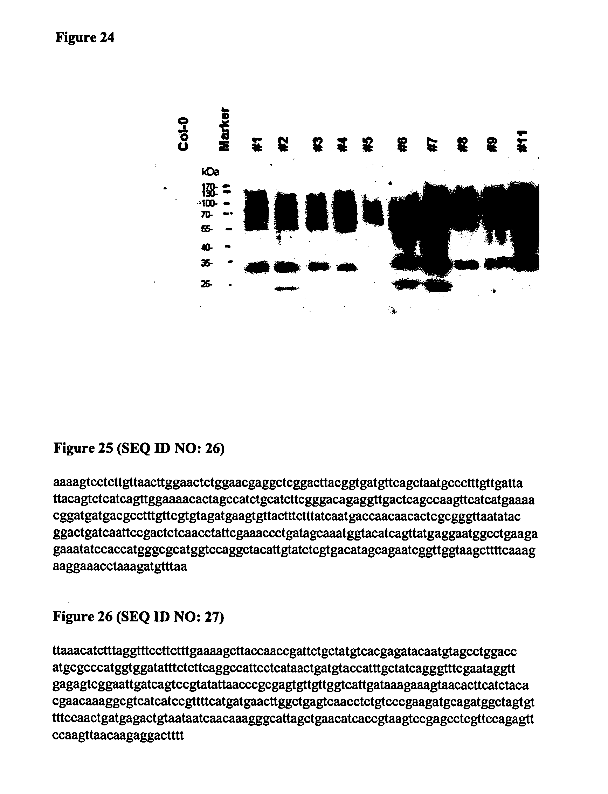

Expression of the murine β1,3-galactosyltransferase 1 (NP_064679) was performed as described for the plant enzyme in Example 2. Murine β1,3-galactosyltransferase 1 was amplified from mouse genomic DNA using the primers 5'- TTTATCTAGATGGCTTCAAAG-GTCTCCTGCC- 3' (SEQ ID NO: 18) and 5'- TTCTGGATCCTAACATCTCAGAT-GCTTCTTG (SEQ ID NO: 19), subcloned into Topo vector and sequenced. Subsequently the entire open reading frame was excised from the Topo vector by XbaI/BamHI digestion and ligated into XbaI/BamHI digested binary plant expression vector pPT2. Transgenic plants were prepared and analyzed as described for the plant Lewis-type β1,3-galactosyltransferase in Example 2. Figure 24 shows the immunoblot analysis of transgenic A. *thaliana* lines (#1-#11) expressing the murine β1,3-GalT1 in leaves. The Lewis-A specific antibody produced a strong signal in the transgenic plants, which was not present in untransformed wildtype plants (Col-0) and showed the presence of increased levels of Lewis-A epitope.

### Example 8: Analysis of A. thaliana mutants with reduced amounts of plant Lewis-type β1,3-galactosyltransferase

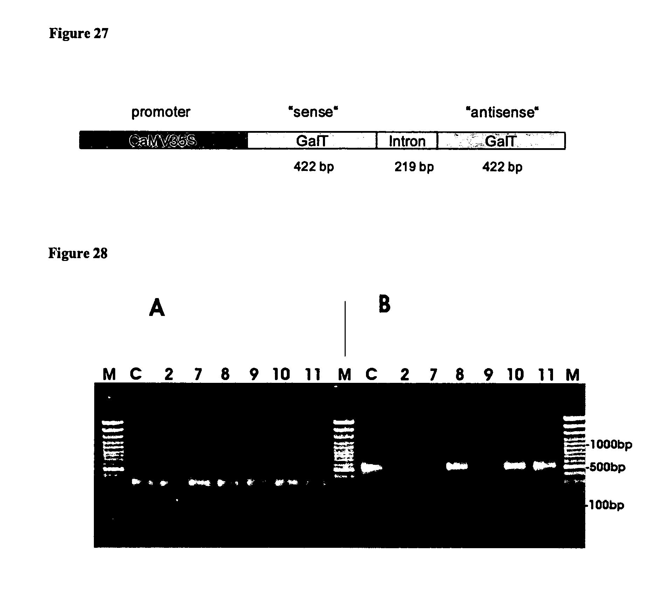

Gene silencing of plant Lewis-type β1,3-galactosyltransferase was performed using a hairpin construct to mediate efficient knockdown of SEQ ID NO 1 expression in A. *thaliana* plants. For this purpose a binary plant expression vector, which contains intron 2 of the A. *thaliana* β1,2-xylosyltransferase gene (Strasser et al., 2000, FEBS LETT. 472: 105-108) was generated. Genomic DNA was isolated from *A. thaliana* leaves using a GenElute Plant Genomic DNA Purification Kit (Sigma) and used as a template to amplify the intron 2 of the A. *thaliana* β1,2-xylosyltransferase gene with the primer combination 5'- ATCAGGGATCCACT-GCACGGTATGCTCCTC-3 (SEQ ID NO: 20) and 5'- ATCGTGGTACCTAGCT-GCGTCTGCAAAAAG-3' (SEQ ID NO: 21). The resulting product was BamHI/KpnI digested and cloned into cloning vector puc18 to create p18XTI2. A 422 bp fragment from SEQ ID NO. 1 was amplified with primers 5'-TATATCTAGAAAAGTCCTCTTGTTAACTTGGAAC-3' (SEQ ID NO: 22) and 5'- TATAAGATCTTAAACATCTTTAGGTTTCCTTC-3' (SEQ ID NO: 23) and cloned into XbaI/BamHI digested vector p18XTI2. To amplify the antisense construct the primers 5'-TATAG-GTACCTTAAACATCTTTAGGTTTCCTTC-3' (SEQ ID NO: 24) and 5'-TATAGAATTC*GGATCC*AAAAGTCCTCTTGTTAACTTGGAAC-3' (SEQ ID NO: 25) were used and the resulting PCR product was KpnI/EcoRI digested and cloned into the sense-intron fragment containing vector. The sense-intron-antisense cassette was removed from the cloning vector by XbaI/BamHI digestion and cloned into the binary plant expression vector pPT2. A schematic presentation of the resulting construct is shown in Figure 27. The resulting binary plant expression vector, which harbours the Lewis-type β1,3-galactosyltransferase sense-intron-antisense cassette was transferred into *Agrobacterium tumefaciens* and transgenic plants were generated by floral dipping of wildtype *A. thaliana* plants as described in Example 2. Transgenic plants were selected on kanamycin and subsequently transferred to soil. Total RNA and total protein extracts were isolated from siliques and stems as described in Example 2. Reverse transcriptase reaction was carried out using oligodT primers and AMV-RT (Promega). A control PCR reaction was performed from cDNA to monitor the expression of A. *thaliana* core α1,3-fucosyltransferase B (FucTB) mRNA. As shown in Figure 28A FucTB transcript can be detected in all tested lines (c: wild type control; transgenic lines 2, 7, 8, 9, 10, 11). To analyse changes in the expression of the Lewis-type β1,3-galactosyltransferase mRNA the same cDNA was amplified with primers SEQ ID NO: 13 and SEQ ID NO: 14. Clearly reduced amounts of transcripts can be detected in some transgenic lines (especially lines 2, 7 and 9)(Figure 28B), which indicates a successful gene silencing of the target gene, Lewis-type β1,3-galactosyltransferase.

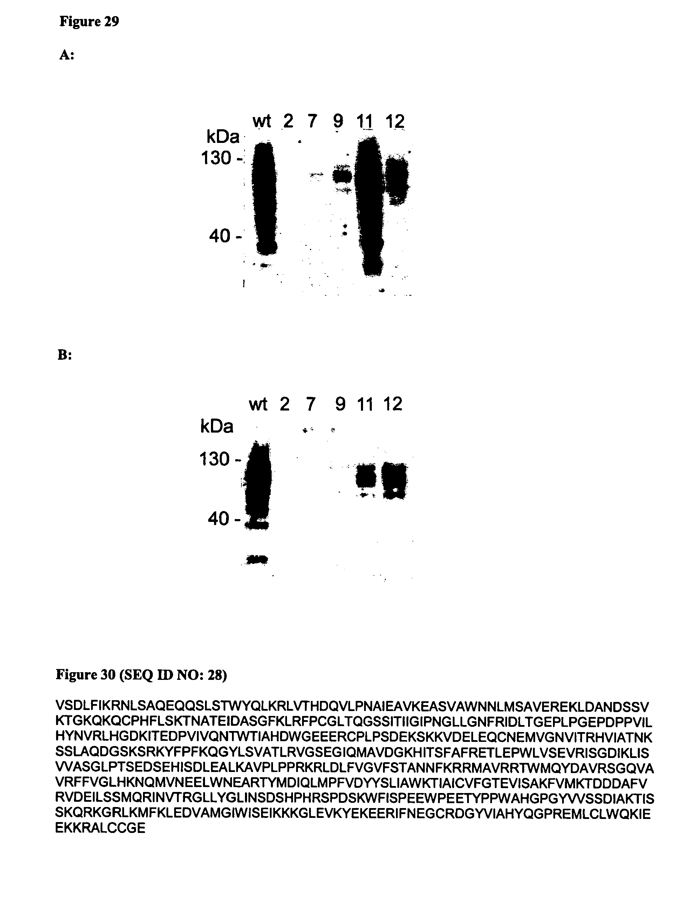

Analysis of the Lewis-A epitope expression was performed by immunoblots as described in Example 2. Instead of leaves, which do not produce detectable amounts of Lewis-A epitope a reduction was monitored in protein extracts from stems (shown in Figure 29A) and siliques (Figure 29B), which display significant amounts of Lewis-A carbohydrate structures in wildtype A. *thaliana* plants. Reduction of Lewis-A epitope formation was clearly visible in transgenic lines, which contain the Lewis-type β1,3-galactosyltransferase hairpin gene silencing construct.

### Example 9: Assay for the plant Lewis-type β1,3-galactosyltransferase using a human glycoprotein as acceptor substrate

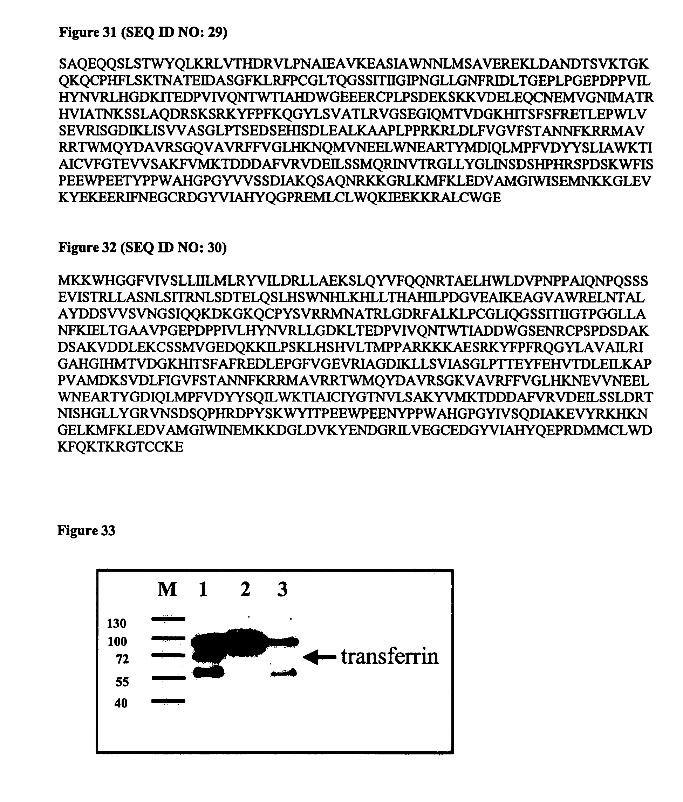

Human transferrin (Sigma-Aldrich) was converted to the GnGn-glycoform by neuraminidase and β-galactosidase treatment as described (Bencurova et al., 2004, Glycobiology, 14: 457-466). The GnGn-transferrin was incubated with recombinantly expressed and purified plant Lewis-type β1,3-GalT and α1,4-fucosyltransferase to generate the Lewis-A epitope on transferrin. 2 µg of human GnGn-transferrin was first incubated overnight at 30°C with 10 mM MnCl2, 0.1 M MES buffer pH 7.0, 0.5 mM UDP-galactose and purified A. thaliana Lewis-type β1,3-GalT. Control reactions were performed either without UDP-galactose or without acceptor substrate GnGn-transferrin. Subsequently, samples were incubated overnight with 2 µl of purified A. thaliana α1,4-fucosyltransferase in 10 mM MnCl2, 0.1 M MES buffer pH 7.0 and 0.5 mM GDP-fucose at 30°C. Samples were separated on 12% SDS-PAGE and blotted onto a ECL-membrane (Amersham Biosciences). The membrane was blocked with 3% (w/v) bovine serum albumin in PBST (phosphate buffer saline with 0.1%(v/v) Tween 20.) pH 7.4 and subsequently incubated for 16h at 4°C with JIM84 antibody as described in Example 2. As shown in Figure 33 a specific signal on the transferrin can only be detected in lane 1, which contains all substrates. Lane 2 (no transferrin in the assay) and lane 3 (no UDP-galactose in the assay) show only unspecific bands.

### Example 10: Identification and Sequences of Lewis-type β1,3-GalT of important plants:

1. rice (2 x complete sequences)
2. poplar (1 x complete)
3. maize (3 x complete sequences)
4. tobacco (N. tabacum, partial sequence)
5.N. benthamiana (partial sequence)
6. apple (Malus domestica, partial sequence)
7. cotton (partial sequence)
8. common bean (Phaseolus vulgaris, partial sequence)
9. leafy spurge (partial sequence)
10.lettuce (partial sequence)
11.tomato (partial sequence)
12.grapewine (partial sequence)
13. wheat (partial sequence)
14.potato (partial sequence)
15. Medicago (partial sequence)

>Rice1
MKKWHGGFVIVSLFIILMLRYVILDSPLAEKSLQYVFQQNNTTPLHWLDVPNPPAVQNPQNISQVISTEL
LASNLSITRNFSDRELQSLHSWNHLKDLVSHAHILPDGVEAIKEAGVAWRELNAALEYDESAVSVNGSTH
QKSKEKQCPYSIRRMNATRSGDRFVLKIPCGLIQGSSITIIGTPGGLLGSFKIELTGATVPGEPDPPIVL
HYNVRLLGDKLTEDPVIVQNTWTIADDWGSEDRCPSPGSDAKDIAKVDELEKCGSMVGNDQKQAWATKLK
SNVSSIQPAWKKNTEPKKYFPFRQGYLAIAILRVGAHGIHMTVDGKHVTSFAFREDLEPGFVGEVRIAGD
IKLLSVIASGLPTTEDFEHVTDLEILKAPPVPMDKPLDLFIGIFSTANNFKRRMAVRRTWMQYDAVRSGK
AAVRFFVGLHKNEVVNEELWNEARTYGDIQLMPFVDYYSLILWKTIAICIYGTNVLSAKYVMKTDDDAFV
RVDEILLSLDRVNISHGLLYGRVNSDSQPHRDPYSKWYITPEEWPEESYPPWAHGPGYIVSQDIAKEVYR
KHKRGELKMFKLEDVAMGIWINEMKKDGLDVKYENDGRILVEGCEEGYVVAHYQEPRDMMCLWDKFLKTK
RGTCCKE

ATGAAGAAATGGCACGGTGGTTTTGTCATAGTATCTTTGTTCATTATATTGATGCTAAGATATGTTATAT
TGGATAGTCCACTTGCTGAAAAATCTCTTCAGTATGTCTTCCAACAGAATAACACAACCCCACTACATTG
GCTGGATGTTCCAAACCCGCCTGCAGTTCAGAATCCACAGAACATCTCTCAAGTAATATCAACTGAATTG
TTAGCTTCAAACCTTTCCATAACAAGAAATTTCTCCGATAGAGAACTGCAGTCATTGCATTCTTGGAATC
ATTTAAAAGACCTAGTAAGTCATGCGCACATCTTACCAGATGGAGTAGAAGCAATCAAGGAAGCTGGAGT
AGCATGGAGGGAGCTAAATGCAGCCCTTGAATACGATGAATCTGCTGTTTCTGTCAATGGTAGCACTCAT
CAAAAAAGTAAAGAGAAGCAATGCCCATATTCCATTCGAAGAATGAATGCTACAAGATCAGGTGATAGAT
TTGTCTTAAAGATTCCTTGTGGACTGATTCAGGGCTCTTCAATAACTATTATCGGTACTCCTGGTGGTCT
TCTTGGTAGTTTTAAGATAGAATTAACTGGGGCTACTGTACCTGGTGAACCAGACCCTCCAATTGTGCTT
CATTACAATGTCCGTCTTCTTGGTGATAAACTTACAGAAGATCCTGTAATAGTCCAAAATACATGGACTA
TAGCTGACGACTGGGGTTCTGAAGATCGTTGCCCATCTCCTGGTTCAGATGCTAAGGACATTGCAAAAGT
GGATGAGTTAGAAAAATGCGGTAGCATGGTGGGCAATGATCAAAAACAAGCATGGGCCACTAAGTTAAAG
TCCAATGTTTCAAGCATACAACCTGCCTGGAAAAAGAACACAGAACCTAAGAAATATTTTCCCTTCAGAC
AAGGATATCTTGCTATAGCAATTCTTCGTGTTGGAGCACATGGGATCCATATGACTGTAGATGGGAAACA
TGTCACTTCATTTGCCTTTCGGGAGGATTTGGAGCCTGGATTTGTTGGTGAAGTAAGAATTGCAGGAGAT
ATTAAATTGTTGTCTGTGATAGCAAGTGGTTTGCCTACAACAGAGGACTTTGAACATGTGACTGACTTAG
AAATTTTGAAGGCTCCACCAGTTCCCATGGACAAACCCCTGGATCTTTTTATTGGGATCTTTTCTACAGC
TAACAATTTCAAGCGCAGAATGGCAGTTCGCAGAACTTGGATGCAGTATGATGCTGTCCGCTCAGGAAAG
GCTGCAGTTCGATTCTTTGTTGGCCTGCATAAAAATGAGGTGGTGAATGAGGAACTTTGGAATGAAGCAC
GAACATATGGAGACATCCAATTGATGCCCTTTGTTGATTATTATAGCCTGATTCTATGGAAGACTATAGC
CATCTGCATCTATGGGACAAACGTCTTGTCAGCCAAGTATGTGATGAAAACAGATGATGATGCTTTTGTC
CGTGTGGATGAGATACTTTTATCTTTAGATCGCGTGAACATCAGTCATGGGTTACTGTACGGTCGTGTCA

ATTCTGATTCTCAGCCTCATCGAGACCCATATAGCAAGTGGTACATAACCCCAGAGGAATGGCCTGAGGA
GAGTTATCCTCCATGGGCACATGGTCCGGGATACATTGTATCACAAGACATAGCAAAAGAAGTTTACAGG
AAACACAAGAGAGGAGAACTTAAGATGTTCAAACTAGAAGATGTCGCAATGGGAATATGGATCAATGAGA
TGAAGAAGGATGGGTTGGATGTAAAGTATGAAAATGATGGGAGGATTTTGGTCGAAGGCTGTGAGGAAGG
ATATGTGGTTGCACATTACCAGGAGCCAAGGGACATGATGTGCCTATGGGACAAATTTCTGAAAACAAAA
CGAGGAACATGTTGCAAGGAGTAA

>Rice2
MWVTKRLGITVLIVLFPLLIVHHLIVNSPVSGPSRYQVIHSNLLGWLSDSLGNSVAQNPDNTPVEVIPADASASNSSDSGNSSLEG
FQWLNTWNHMKQLTNISDGLPHANEAIDNARTAWENLTISVHNSTSKQIKKERQCPYSIHRMNASKPDTGDFTIDIPCGLIVGSSV
TIIGTPGSLSGNFRIDLVGTELPGGSGKPIVLHYDVRLTSDELTGGPVIVQNAFTASNGWGYEDRCPCSNCNNATQVDDLERCNSM
VGREEKRAINSKQHLNAKKDEHPSTYFPFKQGHLAISTLRIGLEGIHMTVDGKHVTSFPYKAGLEAWFVTEVGVSGDFKLVSAIAS
GLPTSEDLENSFDLAMLKSSPIPEGKDVDLLIGIFSTANNFKRRMAIRRTWMQYDAVREGAVVVRFFVGLHTNLIVNKELWNEART
YGDIQVLPFVDYYSLITWKTLAICIYGTGAVSAKYLMKTDDDAFVRVDEIHSSVKQLNVSHGLLYGRINSDSGPHRNPESKWYISP
EEWPEEKYPPWAHGPGYVVSQDIAKEINSWYETSHLKMFKLEDVAMGIWIAEMKKGGLPVQYKTDERINSDGCNDGCIVAHYQEPR
HMLCMWEKLLRTNQATCCN

ATGTGGGTGACAAAGCGACTCGGAATTACGGTTCTAATCGTCCTCTTCCCTCTGCTGATTGTGCATCATC
TGATAGTGAATAGCCCAGTGTCAGGGCCATCTCGATACCAAGTCATACATTCTAATCTCCTTGGTTGGTT
GAGCGACTCATTGGGAAATTCAGTGGCTCAAAATCCAGATAATACACCAGTAGAAGTGATACCAGCAGAT
GCTTCAGCTTCAAACAGCTCGGATTCCGGAAATTCCTCTCTTGAAGGATTTCAGTGGTTAAATACATGGA
ATCACATGAAGCAGTTGACCAACATCTCCGATGGCCTTCCTCACGCAAATGAGGCTATAGACAATGCAAG
AACTGCATGGGAAAACCTGACGATATCAGTTCACAATTCAACCTCTAAACAGATTAAGAAAGAGAGGCAG
TGTCCATATTCTATCCACAGAATGAATGCCTCCAAGCCAGACACTGGAGATTTTACCATCGATATACCTT
GTGGGCTGATTGTAGGTTCTTCAGTAACTATTATAGGCACTCCAGGTAGCCTCTCCGGTAACTTCAGGAT
TGATCTTGTTGGGACAGAATTGCCAGGGGGATCTGGAAACCCATCGTACTGCACTATGATGTTCGTCTA
ACAAGTGATGAACTTACTGGAGGTCCAGTCATAGTGCAGAATGCCTTCACTGCAAGTAATGGCTGGGGTT
ATGAGGATAGATGTCCCTGCAGTAACTGTAATAATGCAACTCAAGTGGATGATTTGGAGCGATGTAATTC
CATGGTGGGTAGAGAAGAGAAGAGAGCTATAAACTCAAAACAGCACCTGAATGCCAAGAAAGATGAACAC
CCAAGCACATATTTTCCATTTAAGCAGGGACACCTTGCTATCTCAACTCTTCGGATAGGGCTAGAAGGGA
TTCACATGACAGTTGATGGAAAACATGTTACCTCATTTCCATATAAAGCGGGCTTGGAAGCATGGTTCGT
TACTGAAGTAGGAGTATCTGGTGATTTCAAGCTAGTAAGTGCAATTGCAAGTGGCCTGCCCACATCAGAA
GACCTGGAGAACAGCTTCGATCTTGCAATGTTGAAGTCGTCACCTATTCCAGAGGGAAAAGATGTGGACC
TTTTGATTGGTATCTTTTCAACAGCAAACAACTTCAAACGAAGGATGGCGATCCGACGGACATGGATGCA
ATATGATGCGGTGCGCGAAGGAGCAGTAGTGGTCCGTTTTTTCGTTGGCCTGCATACAAACCTCATTGTA
AACAAGGAACTCTGGAATGAAGCACGCACATATGGCGACATTCAAGTGCTGCCCTTTGTTGATTACTACA
GTCTGATAACATGGAAGACACTGGCAATATGCATCTACGGGACTGGTGCTGTATCAGCGAAATATCTGAT
GAAAACAGACGATGATGCTTTTGTCCGTGTGGACGAGATACACTCCTCAGTAAAACAACTGAATGTCAGT
CACGGCCTACTTTATGGTCGTATCAATTCTGACTCAGGTCCTCACAGAAATCCTGAGAGCAAATGGTACA
TAAGCCCAGAGGAATGGCCTGAAGAGAAATACCCACCATGGGCACACGGACCAGGATATGTGGTATCACA

AGACATTGCAAAGGAAATCAACAGTTGGTATGAAACAAGTCATTTGAAGATGTTCAAACTAGAAGATGTA
GCAATGGGTATATGGATTGCTGAAATGAAGAAGGGTGGGTTACCTGTCCAATACAAAAACAGATGAAAGAA
TTAACAGTGATGGCTGCAATGATGGGTGCATCGTTGCTCACTACCAAGAACCAAGGCACATGCTATGCAT
GTGGGAGAAACTCCTAAGGACAAATCAAGCAACGTGCTGCAACTAA

>Poplar (Populus trichocarpa)
MKKWYGGVLVASLFMLLILRYGLLKNPIGGNILLNPSSNASKPLEWVHPAVPPAVQNPETSSQVFSTDTI
VSSLFAPRNISNEEHKSLQTWNLLKHLIDHAQVFANGVEAIKEAGNAWSSLMASAEEERLSYTNESSSRK
VKEKQCPHFLNKMNATELDNSGYKLWLPCGLTQGSSITIISIPDGLLGNFRIDLTGEALPGEPDPPIILH
YNVRLHGDKITEDPVIVQNTWTVAHDWGEEERCPSPSPEKIKKVDELDQCNKMVGRNDTRVTSMHSDHSR
RSSLQEGTKARRYFPFKQGQLSVATLRVGMEGIQMTVDGKHITSFAYRETLEPWLVSEVRISGDLNLISV
VASGLPTSEDSEHAVDLEVLKSAPLSPKKTLDLFIGVFSTANNFKRRMAVRRTWMQYAAVRSGAVAVRFF
VGLHKNQIVNEELWNEARTYGDIQLMPFVDYYNLITFKTLAICIFGTEVASAKYVMKTDDDAFVRVDEVL
ASLKRIKVSHGLLYGLINSDSRPHRSTESKWYISPEEWSEETYPPWAHGPGYVVSRDIAKAVYKRYKEGR
LKMFKLEDVAMGIWIAEMKKEGLEVKYEMEGRVHNEGCRDGYVVAHYQAPREMLCLWQKLQEGNVARCCG
DR

ATGAAGAAGTGGTACGGTGGTGTTCTGGTTGCATCCTTGTTCATGTTACTGATCCTTAGATATGGTCTCCTGAAAAATCC
AATCGGAGGAAATATTCTGTTGAATCCCTCCTCCAATGCAAGCAAGCCTCTTGAATGGGTGCATCCTGCTGTTCCACCTG
CAGTTCAAAATCCAGAGACTTCTTCTCAAGTTTTTTCTACAGATACCATAGTCTCCAGTCTGTTTGCTCCGAGAAATATT
TCCAATGAAGAGCACAAGTCTCTGCAGACATGGAATCTACTGAAACACTTGATTGATCATGCTCAGGTTTTCGCAAATGG
AGTAGAAGCTATTAAGGAAGCTGGAAATGCGTGGAGCAGCCTGATGGCTTCAGCAGAAGAGGAAAGACTTAGTTATACAA
ATGAAAGTTCAAGTAGGAAAGTGAAAGAGAAACAATGTCCTCACTTTCTTAACAAAATGAATGCCACAGAGCTTGATAAT
AGTGGTTATAAATTGTGGCTTCCTTGTGGCTTGACTCAGGGTTCGTCCATCACAATTATCAGCATTCCAGATGGTCTTCT
CGGTAATTTTCGGATCGACTTAACCGGGGAAGCACTTCCAGGGGAGCCTGATCCACCCATCATTTTACATTACAATGTTA
GGCTTCATGGTGATAAGATTACAGAGGATCCAGTAATAGTCCAAAACACCTGGACTGTGGCTCATGACTGGGGTGAAGAG
GAGCGTTGTCCTTCTCCATCTCCTGAAAAAATTAAGAAAGTGGACGAGTTGGATCAATGTAATAAGATGGTGGGCAGAAA
CGATACTCGGGTGACTAGCATGCATTCTGATCATTCGAGAAGGTCATCACTTCAGGAAGGAACTAAAGCAAGAAGATATT
TTCCATTTAAGCAAGGTCAGCTATCTGTCGCAACGCTTAGAGTGGGAATGGAAGGAATCCAGATGACTGTTGATGGAAAG
CACATAACATCCTTTGCATATCGTGAAACTTTGGAGCCATGGCTAGTTAGTGAAGTTAGGATTTCTGGGGACCTAAATCT
AATTTCAGTTGTGGCTAGTGGTCTACCTACATCAGAGGATTCAGAGCATGCAGTTGATCTAGAAGTACTCAAATCAGCTC
CTCTCTCTCCAAAAAAAACACTGGATCTCTTTATTGGTGTCTTTTCTACGGCAAACAATTTTAAGCGGAGGATGGCTGTT
CGAAGAACATGGATGCAGTATGCTGCAGTGCGATCAGGGGCAGTTGCAGTGCGCTTTTTTGTTGGTCTGCATAAAAATCA
AATAGTGAATGAGGAACTCTGGAATGAAGCACGGACATATGGAGACATACAGCTGATGCCTTTTGTTGACTACTATAACC
TCATCACCTTTAAAACTTTAGCTATCTGCATATTTGGGACAGAGGTTGCATCAGCTAAGTATGTCATGAAGACAGATGAC
GATGCGTTTGTTCGTGTGGATGAAGTGTTAGCTTCTTTAAAGAGGATCAAAGTGAGTCATGGGTTGCTCTATGGATTAAT
CAATTCAGACTCTCGGCCTCATAGGAGTACTGAAAGCAAGTGGTATATTAGCCCTGAGGAATGGTCTGAAGAGACCTATC
CCCCCTGGGCACATGGTCCTGGTTACGTTGTGTCTCGTGACATTGCCAAGGCAGTTTACAAGAGATACAAAGAAGGCCGT
TTAAAGATGTTTAAGCTAGAGGATGTAGCAATGGGTATCTGGATTGCTGAGATGAAAAAGGAGGGTTTAGAAGTCAAATA
TGAGATGGAAGGGAGGGTCCATAATGAAGGGTGCAGGGATGGCTATGTTGTAGCCCACTACCAAGCTCCTAGGGAAATGC

TCTGTCTGTGGCAGAAACTTCAAGAAGGGAATGTTGCTAGATGCTGCGGTGATCGATAA

>Maize1

MKKWHGGFVIVSLLIIILMLRYVILDRLLAEKSLQYVFQQNRTAELHWLDVPNPPAIQNPQSSSEVISTRLLASNLSITRN

LSDTELQSLHSWNHLKHLLTHAHILPDGVEAIKEAGVAWRELNTALAYDDSVVSVNGSIQQKDKGKQCPYSVRRMNATRL

GDRFALKLPCGLIQGSSITIIGTPGGLLANFKIELTGAAVPGEPDPPIVLHYNVRLLGDKLTEDPVIVQNTWTIADDWGS

ENRCPSPDSDAKDSAKVDDLEKCSSMVGEDQKKILPSKLHSHVLTMPPARKKKAESRKYFPFRQGYLAVAILRIGAHGIH

MTVDGKHITSFAFREDLEPGFVGEVRIAGDIKLLSVIASGLPTTEYFEHVTDLEILKAPPVAMDKSVDLFIGVFSTANNF

KRRMAVRRTWMQYDAVRSGKVAVRFFVGLHKNEVVNEELWNEARTYGDIQLMPFVDYYSQILWKTIAICIYGTNVLSAKY

VMKTDDDAFVRVDEILSSLDRTNISHGLLYGRVNSDSQPHRDPYSKWYITPEEWPEENYPPWAHGPGYIVSQDIAKEVYR

KHKNGELKMFKLEDVAMGIWINEMKKDGLDVKYENDGRILVEGCEDGYVIAHYQEPRDMMCLWDKFQKTKRGTCCKE

ATGAAGAAATGGCATGGAGGCTTTGTCATTGTATCTTTGCTCATCATCTTGATGTTAAGATATGTGATATTGGATAGGCT

ACTTGCAGAAAAATCGCTTCAGTATGTTTTCCAGCAAAACAGGACAGCAGAATTACATTGGTTGGATGTTCCAAACCCAC

CTGCAATCCAGAATCCACAGAGTTCTTCTGAAGTAATATCAACCAGACTCTTGGCATCCAACCTTTCCATTACCAGAAAT

CTCTCTGATACAGAACTTCAATCTTTACATTCTTGGAATCATTTGAAACACCTTTTAACGCATGCCCATATCCTACCAGA

TGGAGTAGAGGCAATCAAGGAAGCTGGAGTTGCATGGAGGGAACTCAATACAGCCCTTGCATATGATGATTCAGTTGTTT

CTGTTAATGGCAGCATTCAGCAGAAGGACAAAGGAAAGCAATGCCCTTATTCTGTCCGACGGATGAATGCCACAAGATTA

GGGGATAGGTTTGCTTTAAAACTTCCATGTGGATTGATTCAGGGCTCTTCAATAACAATTATTGGTACTCCTGGTGGTCT

TCTGGCTAATTTTAAGATAGAGTTAACTGGAGCTGCAGTTCCTGGTGAACCAGACCCTCCAATTGTCCTTCATTATAATG

TCCGCCTTCTTGGTGATAAACTCACAGAAGATCCTGTAATAGTCCAAAATACATGGACGATAGCTGATGATTGGGGTTCT

GAAAACCGTTGCCCATCTCCTGATTCAGATGCTAAGGACAGTGCAAAAGTGGATGATCTAGAAAAGTGTAGTAGCATGGT

AGGTGAGGACCAGAAGAAAATCCTTCCCTCTAAGTTACATTCACATGTTTTGACCATGCCACCTGCAAGGAAGAAGAAAG

CAGAATCTAGAAAGTATTTTCCATTCAGGCAGGGATATCTTGCTGTAGCAATTCTTCGTATCGGAGCACATGGGATCCAT

ATGACTGTAGATGGCAAACATATCACTTCGTTTGCATTTCGAGAAGATCTGGAACCAGGGTTTGTTGGTGAAGTAAGAAT

TGCAGGAGACATTAAATTGCTATCTGTGATAGCAAGTGGCCTGCCAACAACAGAGTACTTCGAGCATGTCACTGATTTGG

AAATTTTGAAGGCTCCACCTGTTGCCATGGATAAATCTGTTGATCTTTTCATTGGGGTCTTCTCTACAGCAAATAATTTC

AAACGCCGAATGGCAGTTCGCAGAACTTGGATGCAGTATGATGCTGTGCGCTCAGGAAAAGTTGCAGTTCGATTCTTTGT

TGGCCTGCACAAAAACGAGGTGGTCAATGAGGAACTCTGGAATGAAGCACGAACATATGGAGACATCCAGTTGATGCCGT

TTGTGGATTACTATAGCCAGATTCTTTGGAAGACCATAGCGATCTGCATCTATGGGACAAATGTCCTGTCAGCTAAGTAT

GTGATGAAAACTGATGATGATGCTTTTGTTCGAGTAGATGAAATACTTTCTTCTCTCGACCGAACCAATATCAGCCATGG

ACTGTTGTATGGTCGTGTCAATTCTGATTCCCAGCCTCATCGAGATCCCTATAGCAAGTGGTACATAACCCCTGAGGAAT

GGCCTGAGGAGAATTATCCTCCATGGGCGCATGGTCCAGGATATATTGTTTCACAGGACATAGCTAAAGAAGTTTACAGG

AAACACAAGAATGGGGAATTAAAGATGTTCAAGCTGGAGGATGTCGCGATGGGAATATGGATCAATGAGATGAAGAAGGA

TGGCTTGGATGTCAAGTATGAGAATGATGGGAGGATCTTGGTTGAAGGATGCGAGGATGGGTATGTGATTGCTCACTACC

AAGAACCAAGGGATATGATGTGCCTCTGGGATAAGTTTCAGAAAACAAAACGAGGAACATGTTGCAAAGAATAA

>Maize2

MKKWHGGFVIVSLFIIILMLRYVILDRLLAEKSLQYVFQQNRTAELHWLDVPNPPAIQNPQSSSEVISTRLLASNLSITRN

LSDTELQSLHSWNHLKHLLTHAHILPDGVEAIKEAGVAWRELNTALAYDDSVVSVNGSIQQKDKGKQCPYSVRRMNATRL
GDRFALKLPCGLIQGSSITIIGTPGGLLANFKIELTGAAVPGEPDPPIVLHYNVRLLGDKLTEDPVIVQNTWTIADDWGS
ENRCPSPDSDAKDSAKVDDLEKCSSMVGEDQKEILPSKLHSHVLTMPPARKKKAESRKYFPFRQGYLAVAILRIGAHGIH
MTVDGKHITSFAFREDLEPGFVGEVRIAGDIKLLSVIAGGLPTTEYFEHVTDLEILKAPPVAMDKSVDLFIGVFSTANNF
KRRMAVRRTWMQYDAVRSGKVAVRFFVGLHKNEVVNEELWNEARTYGDIQLMPFVDYYSLILWKTIAICIYGTNVLSAKY
VMKTDDDAFVRVDEILSSLDRTNISHGLLYGRVNSDSQPHRDPYSKWYITPEEWPEENYPPWAHGPGYIVSQDIAKEVYR
KHKNGELKMFKLEDVAMGIWINEMKKDGLDVKYENDGRILVEGCEDGYVIAHYQEPRDMMCLWDKFQKTKRGTCCKE

ATGAAGAAATGGCATGGAGGCTTTGTCATTGTATCTTTGTTCATCATCTTGATGTTAAGATATGTGATATTGGATAGGCT
ACTTGCAGAAAAATCGCTTCAGTATGTTTTCCAGCAAAACAGGACAGCAGAATTACATTGGTTGGATGTTCCAAACCCAC
CTGCAATCCAGAATCCACAGAGTTCTTCTGAAGTAATATCAACCAGACTCTTGGCATCCAACCTTTCCATTACCAGAAAT
CTCTCTGATACAGAACTTCAATCTTTACATTCTTGGAATCATTTGAAACACCTTTTAACGCATGCCCATATCCTACCAGA
TGGAGTAGAGGCAATCAAGGAAGCTGGAGTTGCATGGAGGGAACTCAATACAGCCCTTGCATATGATGATTCAGTTGTTT
CTGTTAATGGCAGCATTCAGCAGAAGGACAAAGGAAAGCAATGCCCTTATTCTGTCCGACGGATGAATGCCACAAGATTG
GGGGATAGGTTTGCTTTAAAACTTCCATGTGGATTGATTCAGGGCTCTTCAATAACTATTATTGGTACTCCTGGCGGTCT
TCTGGCTAATTTTAAGATAGAGTTAACTGGAGCTGCAGTTCCTGGTGAACCAGACCCTCCAATTGTCCTTCATTATAATG
TCCGCCTTCTTGGTGATAAACTCACAGAAGATCCTGTAATAGTCCAAAATACATGGACGATAGCTGATGATTGGGGTTCT
GAAAATCGTTGCCCATCTCCTGATTCAGATGCTAAGGACAGTGCAAAAGTGGATGATCTAGAAAAGTGTAGTAGCATGGT
AGGTGAGGACCAGAAGGAAATCCTTCCCTCTAAGTTACATTCACATGTTTTGACCATGCCACCTGCAAGGAAGAAGAAAG
CAGAATCTAGAAAGTATTTTCCATTCAGGCAGGGATATCTTGCTGTAGCAATTCTTCGTATCGGAGCACATGGGATCCAT
ATGACTGTAGATGGCAAACATATCACTTCGTTTGCATTTCGAGAAGATCTGGAACCAGGGTTTGTTGGTGAAGTAAGAAT
TGCAGGAGACATTAAATTGCTATCTGTGATAGCAGGTGGCCTGCCAACAACAGAGTACTTCGAGCATGTCACTGATTTGG
AAATTTTGAAGGCTCCACCTGTTGCCATGGATAAATCTGTTGATCTTTTCATTGGGGTCTTCTCTACAGCAAATAATTTC
AAACGCCGAATGGCAGTTCGCAGAACTTGGATGCAGTATGATGCTGTGCGCTCAGGAAAAGTTGCAGTTCGATTCTTTGT
TGGCCTGCACAAAAACGAGGTGGTCAATGAGGAACTCTGGAATGAAGCACGAACATATGGAGACATCCAGTTGATGCCAT
TTGTGGATTACTATAGCCTGATTCTTTGGAAGACCATAGCGATCTGCATCTATGGGACAAATGTCCTGTCAGCTAAGTAT
GTGATGAAAACTGATGATGATGCTTTTGTTCGAGTAGATGAAATACTTTCTTCTCTCGACCGAACCAATATCAGCCATGG
ACTGTTGTATGGTCGTGTCAATTCTGATTCCCAGCCTCATCGAGATCCCTATAGCAAGTGGTACATAACCCCTGAGGAAT
GGCCTGAGGAGAATTATCCTCCATGGGCGCATGGTCCAGGATATATTGTTTCACAGGACATAGCTAAAGAAGTTTACAGG
AAACACAAGAATGGGGAATTAAAGATGTTCAAGCTGGAGGATGTCGCGATGGGAATATGGATCAATGAGATGAAGAAGGA
TGGCTTGGATGTCAAGTATGAGAATGATGGGAGGATCTTGGTTGAAGGATGCGAGGATGGGTATGTGATTGCTCACTACC
AAGAACCAAGGGATATGATGTGCCTCTGGGATAAGTTTCAGAAAACAAAACGAGGAACATGTTGCAAAGAATAA

>Maize3
MKKWHGGFVIVSLFIILMLRYVILDNPLVENSLQYVFQQNRTAELHWLDVPNPPAIQNLQISSEVISTGLLASNLSITRN
LSDRELQSLHSWNHLKQLLSHVHILPDGVEAIKEAGVAWRELNTALAYDDAVSVNGSIQQKDKGKQCPYSIRRMNATRLG
DRFALKLPCGLIQGSSITIIGTPGGLLGNFKIELTGAAVPGEPDPPIVLHYNVRLLGDKLTEDPVIVQNTWTISDDWGSE
NRCPSPDSDAKDTTKVDDLEKCSSMVGEDQKEILPSKFHSNVSAMPPASKKKAEPRKYFPFRQGYLAVAILRIGAHGIHM
TVDGKHITSFAFREDLEPGFVGEVRIAGDIKLLSVIASGLPTTEDFEHVTDLEILKAPPVPMDKPVDLFIGVFSTANNFK
RRMAVRRTWMQYDSVRSGKVTVRFFVGLHKNELVNEELWNEARTYGDIQLMPFVDYYSLILWKTIAICIYGTNVLSAKYV

MKTDDDAFVRVDEILSSLDRTNISHGLLYGRVNSDSQPHRDPYSKWYITPEEWPEENYPPWAHGPGYIVSKDIAKEVYRK
HKSGELKMFKLEDVAMGIWINEMKKDGLDVKYENDGRILVEGCEDGYVIAHYQEPRDMMCLWDQFQKTKRGTCCKE

ATGAAGAAATGGCATGGAGGCTTTGTCATTGTATCTTTGTTCATCATCTTGATGTTAAGATATGTGATATTGGATAACCC
ACTTGTAGAAAACTCGCTTCAGTATGTTTTCCAGCAAAACAGGACGGCAGAATTACATTGGTTGGATGTTCCAAACCCAC
CTGCAATCCAGAATCTACAGATTTCTTCTGAAGTAATATCAACTGGACTCTTGGCTTCCAACCTTTCCATTACCAGAAAT
CTCTCTGATAGAGAACTTCAATCTTTACATTCTTGGAATCATTTGAAACAGCTATTATCTCATGTTCATATCCTACCAGA
TGGAGTAGAGGCAATCAAGGAAGCTGGAGTTGCATGGAGGGAACTCAATACAGCCCTTGCATATGATGATGCAGTTTCTG
TTAATGGCAGCATTCAGCAGAAGGACAAAGGAAAGCAATGCCCTTATTCTATCAGAAGGATGAATGCCACAAGATTAGGG
GATAGGTTTGCTTTAAAACTTCCCTGTGGACTGATTCAGGGCTCTTCAATAACTATTATTGGTACACCTGGTGGTCTTCT
TGGTAATTTTAAGATAGAGTTAACTGGGGCTGCAGTTCCTGGTGAACCAGACCCTCCAATTGTCCTTCATTATAATGTCC
GCCTTCTTGGTGATAAACTCACAGAAGATCCTGTAATAGTCCAAAATACATGGACAATATCTGATGATTGGGGTTCTGAA
AACCGTTGCCCATCTCCTGATTCAGATGCTAAGGACACTACAAAAGTGGATGATCTAGAAAAATGTAGTAGCATGGTAGG
TGAGGACCAGAAAGAAATCCTTCCCTCTAAGTTTCATTCAAATGTTTCGGCCATGCCACCTGCAAGTAAGAAGAAAGCAG
AACCTAGAAAGTATTTTCCATTCAGACAGGGATATCTTGCCGTAGCAATTCTTCGGATCGGAGCACATGGGATCCATATG
ACTGTAGATGGCAAACATATCACTTCTTTTGCATTCCGAGAGGATTTGGAACCAGGGTTTGTAGGTGAAGTAAGAATTGC
AGGAGACATTAAATTGTTATCTGTGATAGCAAGTGGCCTGCCTACAACAGAGGACTTCGAGCATGTCACTGACTTAGAAA
TATTGAAGGCTCCACCTGTGCCCATGGATAAACCGGTTGATCTTTTCATTGGGGTCTTCTCTACAGCAAATAATTTCAAA
CGCCGAATGGCAGTTCGCAGAACTTGGATGCAGTATGATTCTGTTCGATCAGGAAAAGTTACAGTTCGATTCTTTGTTGG
CCTGCACAAAAATGAGTTGGTCAATGAGGAACTCTGGAATGAAGCACGAACATATGGAGACATCCAGTTGATGCCATTTG
TGGATTACTATAGCCTGATTCTTTGGAAGACCATAGCGATCTGCATCTATGGGACAAATGTCCTGTCAGCTAAGTATGTG
ATGAAAACTGATGATGATGCTTTTGTTCGAGTAGATGAAATACTTTCTTCTCTCGACCGAACCAATATCAGCCATGGACT
GTTGTATGGTCGTGTCAATTCTGATTCCCAGCCTCATCGAGATCCCTATAGCAAGTGGTACATAACCCCTGAGGAATGGC
CTGAGGAGAATTATCCTCCCTGGGCGCATGGACCAGGATATATTGTTTCAAAGGACATAGCTAAAGAAGTTTACAGGAAA
CACAAGAGTGGGGAGTTAAAGATGTTCAAGCTGGAGGATGTCGCAATGGGAATATGGATCAATGAGATGAAGAAGGATGG
CTTGGATGTCAAGTATGAGAATGATGGGAGGATTTTGGTTGAAGGATGCGAGGATGGCTATGTGATTGCTCATTACCAAG
AACCAAGGGATATGATGTGCCTCTGGGATCAGTTTCAGAAAACAAAACGAGGAACATGTTGCAAAGAATAA

>Nicotiana tabacum
VSDLFIKRNLSAQEQQSLSTWYQLKRLVTHDQVLPNAIEAVKEASVAWNNLMSAVEREKLDANDSSVKTGKQKQCPHFLS
KTNATEIDASGFKLRFPCGLTQGSSITIIGIPNGLLGNFRIDLTGEPLPGEPDPPVILHYNVRLHGDKITEDPVIVQNTW
TIAHDWGEEERCPLPSDEKSKKVDELEQCNEMVGNVITRHVIATNKSSLAQDGSKSRKYFPFKQGYLSVATLRVGSEGIQ
MAVDGKHITSFAFRETLEPWLVSEVRISGDIKLISVVASGLPTSEDSEHISDLEALKAVPLPPRKRLDLFVGVFSTANNF
KRRMAVRRTWMQYDAVRSGQVAVRFFVGLHKNQMVNEELWNEARTYMDIQLMPFVDYYSLIAWKTIAICVFGTEVISAKF
VMKTDDDAFVRVDEILSSMQRINVTRGLLYGLINSDSHPHRSPDSKWFISPEEWPEETYPPWAHGPGYVVSSDIAKTISS
KQRKGRLKMFKLEDVAMGIWISEIKKKGLEVKYEKEERIFNEGCRDGYVIAHYQGPREMLCLWQKIEEKKRALCCGE

GTCTCTGATCTCTTTATCAAGAGGAACCTGTCAGCTCAGGAGCAACAATCTTTGTCTACGTGGTATCAGTTGAAACGCTT
AGTTACTCATGATCAAGTCTTACCTAATGCTATAGAGGCAGTTAAGGAAGCCAGTGTTGCATGGAACAACCTCATGAGTG

CAGTAGAGAGGGAAAAACTTGATGCAAATGATAGTTCAGTTAAGACCGGGAAACAGAAACAATGCCCTCATTTTCTCAGC
AAAACCAATGCTACAGAAATTGATGCCAGTGGCTTTAAGTTGCGGTTTCCTTGTGGTCTGACTCAGGGTTCTTCCATCAC
AATAATTGGCATTCCAAATGGTCTTCTTGGGAATTTTCGGATAGATTTGACTGGTGAACCACTTCCAGGTGAACCAGATC
CTCCTGTTATTCTGCATTACAATGTTAGGCTCCATGGTGATAAAATAACTGAGGATCCCGTAATTGTACAAAACACCTGG
ACAATTGCACATGACTGGGGTGAAGAGGAGCGCTGTCCCTTACCATCGGATGAAAAGAGTAAGAAAGTGGATGAATTGGA
GCAATGCAATGAGATGGTGGGAAATGTCATAACTCGACATGTCATTGCAACAAATAAATCTAGCCTGGCTCAGGACGGGT
CTAAATCAAGAAAATATTTTCCtTTCAAGCAAGGATATCTCTCTGTTGCCACTCTGAGAGTAGGATCTGAAGGAATTCAG
ATGGCAGTTGATGGAAAACACATAACATCTTTTGCTTTCCGTGAAACTTTGGAACCGTGGCTTGTAAGTGAAGTGAGGAT
ATCCGGAGACATAAAATTAATTTCTGTTGTCGCGAGTGGTTTGCCAACATCTGAGGATTCAGAGCATATAAGTGACTTGG
AAGCTCTAAAAGCAGTTCCTCTTCCTCCTCGGAAAAGACTAGATCTCTTTGTTGGTGTATTTTCTACCGCAAATAATTTT
AAGCGCAGAATGGCTGTCCGTAGAACTTGGATGCAATATGATGCAGTGCGATCTGGACAAGTTGCGGTGCGGTTTTTTGT
TGGCTTGCATAAAAACCAAATGGTGAATGAAGAGCTCTGGAATGAGGCTAGGACATATATGGACATCCAGCTGATGCCTT
TTGTTGATTACTATAGTCTTATCGCTTGGAAGACCATTGCCATATGTGTTTTTGGGACGGAGGTCATTTCGGCAAAGTTT
GTCATGAAGACAGATGATGACGCATTTGTTCGAGTGGATGAAATCTTGTCTTCTATGCAGAGGATTAATGTGACTCGCGG
ATTGCTCTATGGTCTTATCAACTCAGATTCCCATCCTCATAGGAGCCCAGACAGCAAGTGGTTTATCAGTCCAGAGGAAT
GGCCTGAAGAAACTTACCCTCCTTGGGCACATGGACCGGGTTATGTTGTGTCCAGTGATATAGCAAAAACAATCAGCTCA
AAACAGAGAAAAGGCCGCCTAAAGATGTTTAAGCTGGAAGATGTTGCTATGGGCATCTGGATTTCAGAAATAAAGAAGAA
AGGGTTGGAAGTGAAGTATGAAAAAGAAGAGAGGATTTTTAATGAAGGTTGCCGAGATGGTTATGTTATTGCACATTACC
AAGGCCCTAGAGAGATGCTCTGTCTTTGGCAAAAGATTGAAGAGAAAAAACGGGCTTTATGTTGTGGCGAGTAA

>Nicotiana benthamiana
SAQEQQSLSTWYQLKRLVTHDRVLPNAIEAVKEASIAWNNLMSAVEREKLDANDTSVKTGKQKQCPHFLSKTNATEIDAS
GFKLRFPCGLTQGSSITIIGIPNGLLGNFRIDLTGEPLPGEPDPPVILHYNVRLHGDKITEDPVIVQNTWTIAHDWGEEE
RCPLPSDEKSKKVDELEQCNEMVGNIMATRHVIATNKSSLAQDRSKSRKYFPFKQGYLSVATLRVGSEGIQMTVDGKHIT
SFSFRETLEPWLVSEVRISGDIKLISVVASGLPTSEDSEHISDLEALKAAPLPPRKRLDLFVGVFSTANNFKRRMAVRRT
WMQYDAVRSGQVAVRFFVGLHKNQMVNEELWNEARTYMDIQLMPFVDYYSLIAWKTIAICVFGTEVVSAKFVMKTDDDAF
VRVDEILSSMQRINVTRGLLYGLINSDSHPHRSPDSKWFISPEEWPEETYPPWAHGPGYVVSSDIAKQSAQNRKKGRLKM
FKLEDVAMGIWISEMNKKGLEVKYEKEERIFNEGCRDGYVIAHYQGPREMLCLWQKIEEKKRALCWGE.

TCAGCTCAGGAGCAACAATCTTTGTCTACGTGGTATCAGTTGAAACGCTTAGTTACTCATGATCGAGTCTTACCTAATGC
TATAGAGGCAGTTAAGGAAGCCAGTATCGCATGGAACAACCTCATGAGTGCAGTAGAGAGGGAAAAACTTGATGCAAATG
ATACTTCAGTTAAGACAGGGAAACAGAAACAATGCCCTCATTTTCTCAGCAAAACCAATGCTACAGAAATTGATGCCAGT
GGCTTTAAGTTGCGATTTCCTTGTGGTCTGACTCAGGGTTCTTCCATCACAATAATTGGCATTCCAAATGGTCTTCTTGG
GAATTTTCGGATAGATTTGACTGGTGAACCACTTCCAGGTGAACCAGATCCTCCTGTTATTCTGCACTACAATGTTAGAC
TCCATGGTGATAAAATAACTGAGGATCCCGTAATTGTACAAAACACCTGGACAATTGCACATGACTGGGGTGAAGAGGAG
CGCTGTCCCTTACCATCGGATGAAAAGAGTAAGAAAGTGGATGAATTGGAACAATGCAATGAGATGGTGGGAAATATCAT
GGCTACTCGACATGTCATTGCAACAAATAAATCTAGCCTGGCTCAGGACAGGTCTAAATCAAGAAAGTATTTTCCTTTCA
AGCAAGGATATCTCTCTGTCGCCACTCTGAGAGTAGGATCTGAAGGAATTCAGATGACAGTTGATGGAAAACACATAACA

TCTTTTTCTTTCCGTGAAACTTTGGAACCGTGGCTTGTAAGTGAAGTGAGGATATCCGGAGACATAAAATTAATTTCTGT
TGTCGCAAGTGGTTTGCCAACATCTGAGGATTCAGAGCATATAAGTGATTTGGAAGCTCTAAAAGCAGCTCCTCTTCCTC
CTCGGAAAAGACTAGATCTCTTTGTTGGTGTATTTCTACCGCAAATAATTTAAGCGCAGAATGGCTGTCCGTAGAACT
TGGATGCAATATGATGCAGTGCGATCTGGACAAGTTGCGGTGCGGTTTTTTGTCGGCTTGCATAAAAACCAAATGGTGAA
TGAAGAGCTCTGGAATGAGGCTAGGACATATATGGACATCCAGTTGATGCCTTTTGTTGATTACTATAGTCTTATCGCTT
GGAAGACCATTGCCATATGTGTTTTTGGGACCGAGGTCGTTTCGGCAAAGTTTGTCATGAAGACAGATGATGACGCATTT
GTTCGAGTGGATGAAATCTTGTCTTCTATGCAGAGGATTAACGTGACTCGCGGATTGCTCTATGGTCTTATCAACTCAGA
TTCCCATCCTCATAGGAGTCCAGACAGCAAGTGGTTTATCAGTCCAGAGGAATGGCCTGAAGAAACTTACCCTCCTTGGG
CACATGGACCGGGTTATGTTGTGTCCAGTGATATAGCAAAACAATCAGCTCAAAACAGAAAAAAAGGCCGCCTAAAGATG
TTTAAGCTGGAAGATGTTGCTATGGGCATCTGGATTTCAGAAATGAATAAGAAAGGGTTGGAAGTGAAGTATGAAAAGGA
AGAGAGGATTTTTAATGAAGGTTGCCGAGATGGTTATGTTATTGCACATTACCAAGGCCCTAGAGAGATGCTGTGTCTTT
GGCAAAAGATTGAAGAGAAAAAACGGGCTTTATGTTGGGGCGAGTAA

>Apple (Malus domestica)
GGLKLISVLASGLPTSEDSEHIINLQELKSAPLSPHKQMDLFIGVFSTANNFKRRMAVRRTWMQYAAVRSGAVSVRFFVGLHKNQI
VNEELWKEAQTYGDIQLMPFVDYYGLITWKTLAICIFGTEVVSAKFVMKTDDDAFVRVDEVLASLHRIKVNRGLLYGLINSDSRPH
RSPDSKWYISNEEWPEATYPPWAHGPGYVVSNDIAKAVSQRYKKGRLKMFKLEDVAMGIWIADMKKEGLNVRYEKEEKVYNEGCKD
GYVVAHYQGPREMLCLRQKIPKGQVA

GGAGGCCTGAAATTAATTTCTGTCCTAGCCAGCGGTTTACCCACGTCAGAGGATTCAGAGCACATAATTAATTTACAAGA
ACTCAAATCTGCTCCTCTCTCGCCTCACAAACAAATGGATCTCTTTATTGGTGTTTTCTCTACCGCAAACAATTTTAAAC
GACGGATGGCTGTTCGAAGAACATGGATGCAGTATGCTGCTGTACGATCAGGGGCAGTTTCAGTGCGCTTTTTTGTTGGT
TTGCATAAGAACCAAATAGTGAATGAGGAACTTTGGAAAGAGGCACAAACCTATGGAGACATACAGCTGATGCCTTTTGT
TGATTACTATGGCCTCATTACCTGGAAAACTCTGGCCATCTGCATCTTTGGGACGGAGGTTGTTTCAGCGAAGTTCGTTA
TGAAGACGGATGACGATGCATTTGTTCGTGTGGATGAAGTGCTAGCTTCCTTACACAGGATCAAAGTGAATCGTGGCTTG
CTCTATGGCCTCATTAACTCAGATTCTCGACCTCATCGAAGTCCAGATAGCAAGTGGTATATCAGCAATGAGGAATGGCC
TGAAGCAACTTACCCACCTTGGGCGCACGGTCCTGGTTATGTGGTATCGAATGACATAGCAAAGGCAGTTTCCCAGCGAT
ATAAAAAGGGCAGATTAAAGATGTTTAAGCTGGAAGACGTGGCGATGGGTATCTGGATCGCGGATATGAAAAAGGAGGGT
CTAAACGTACGGTATGAGAAGGAAGAGAAGGTCTACAACGAAGGATGCAAGGACGGGTATGTCGTTGCACATTACCAAGG
TCCCAGGGAGATGCTGTGTCTCCGGCAGAAAATTCCTAAAGGGCAAGTTGCTTGA

>Cotton
DHTVDIEALLSVPLSLQRPVDLFIGVFSTANNFKRRMAVRRTWMQYPEVRSGTVVVRFFVGLHKSHIVNDELWNEARTYG
DIQLMPFVDYYSLITWKTLAICTFGTEVVSAKFVMKTDDDAFVRLDEVMASLSRINVTHGLLYGLINSDSQPHRSTDSKW
FISPEEWSEEKYPPWAHGPGYVVSHDIAKAVYKRFNEVRLKMFKLEDVAMGIWIADMQKDGLEVHYEKEERIFNEGCKDG
YVIAHYQGPREMMCLWQKLPGDQKS

GACCATACGGTTGATATAGAAGCACTCCTTTCAGTTCCTCTTTCTCTTCAAAGACCAGTGGATCTCTTTATTGGTGTTTT
CTCTACTGCCAATAATTTTAAAAGAAGAATGGCTGTGAGAAGAACATGGATGCAGTATCCTGAAGTCCGATCTGGGACAG

TTGTAGTGCGCTTTTTTGTTGGTTTGCATAAAAGCCATATAGTGAATGATGAACTCTGGAATGAAGCACGGACATATGGA
GACATACAGCTGATGCCTTTTGTTGATTACTACAGCCTGATCACCTGGAAAACCTTGGCTATTTGCACCTTTGGGACAGA
AGTTGTTTCTGCAAAGTTTGTCATGAAGACAGATGATGATGCGTTTGTTCGTTTGGATGAAGTCATGGCTTCATTAAGCA
GGATAAATGTAACTCATGGATTGCTTTATGGACTCATTAACTCAGATTCCCAACCTCATCGCAGTACAGATAGCAAGTGG
TTTATTAGCCCTGAGGAATGGTCTGAAGAGAAGTACCCACCTTGGGCGCATGGTCCTGGTTACGTGGTGTCCCATGACAT
AGCAAAGGCTGTTTACAAGAGGTTCAATGAAGTGCGCTTAAAGATGTTTAAACTCGAAGATGTGGCAATGGGCATTTGGA
TCGCGGATATGCAAAAAGATGGTTTAGAAGTCCATTATGAGAAGGAAGAGAGGATCTTTAACGAGGGTTGTAAGGATGGT
TATGTTATTGCTCACTATCAAGGTCCAAGAGAGATGATGTGTTTGTGGCAGAAACTCCCAGGAGACCAAAAGAGCTAG

>Common bean (Phaseolus vulgaris)
PFVDYYSLITWKTLAICLFGTQVSAKFVMKTDDDAFVRVDEVLGSLHRINVDHGLLYGLINSDSRPHRNTDSKWYISPEE
WSEGTYPPWAHGPGYVVSHDIARTVAKKFRENHLKMFKLEDVAMGIWIADMKKEGVDVRCENEDRVYPEGCKDGYVVAHY
QGPREMLCLWQKLEEGKGAKCCGDRK

CCATTTGTTGACTACTACAGCCTCATCACGTGGAAAACTTTGGCAATCTGCCTTTTTGGGACACAAGTTTCGGCAAAGTT
TGTCATGAAGACGGATGATGATGCGTTTGTCAGGGTAGATGAAGTTCTAGGCTCTTTGCACAGGATCAATGTTGATCATG
GGTTACTGTATGGACTCATCAATTCGGATTCCAGACCTCATCGAAATACAGATAGCAAATGGTACATCAGCCCAGAGGAA
TGGAGCGAAGGAACTTATCCTCCTTGGGCACATGGTCCCGGCTATGTGGTCTCACACGATATAGCAAGAACGGTTGCCAA
GAAATTCAGAGAGAACCATTTGAAGATGTTTAAATTAGAAGACGTTGCAATGGGAATTTGGATTGCAGACATGAAGAAGG
AAGGTGTAGATGTTCGGTGTGAGAATGAGGACAGAGTGTATCCTGAGGGTTGTAAGGATGGTTATGTGGTTGCCCATTAC
CAGGGCCCTAGGGAGATGCTCTGTTTGTGGCAGAAACTTGAAGAAGGAAAAGGTGCAAAATGCTGTGGTGATAGGAAATG
A

>Leafy spurge (Euphorbia esula)
TIDGKHITSFAYRETLEPWLVSEVRISGDMNLMSIVASGLPTSEDSEHAIDLELLKSVPLPSKKTPPPHLFIGVFSTANN
FKRRMAVRRTWMQYPHVRSGTVAVRFFVGLHKNQLVNKELWNEASTYGDIQLMPFVDYYNLITWKTLAICIFGTEVASAK
YVMKTDDDAFVRVDQVLASLKRIKVNRGLLYGLINSDSQPHRSTDSKWYISLEEWPEEKYPPWAHGPGYVVSQDI

ACCATTGATGGAAAACACATCACATCTTTTGCGTATCGCGAGACATTGGAGCCATGGCTGGTTAGCGAAGTTAGAATATC
TGGAGACATGAATTTAATGTCTATAGTGGCAAGTGGTCTACCTACATCAGAGGATTCAGAACATGCAATTGATCTAGAGC
TACTCAAATCGGTCCCTCTCCCCTCGAAGAAAACACCACCACCTCATCTCTTTATTGGAGTATTCTCCACAGCCAACAAT
TTTAAACGTAGGATGGCTGTTCGAAGAACTTGGATGCAGTATCCTCATGTTCGCTCTGGGACTGTTGCTGTGCGCTTTTT
TGTTGGGTTGCACAAAAATCAATTAGTTAACAAGGAACTTTGGAATGAAGCATCAACATATGGAGACATACAGCTTATGC
CTTTTGTGGACTATTATAACCTCATCACCTGGAAGACTTTAGCTATCTGCATCTTTGGGACTGAGGTTGCATCAGCAAAA
TATGTCATGAAGACAGATGATGATGCATTTGTTCGAGTGGATCAAGTTTTAGCTTCTTTAAAACGGATCAAAGTGAATCG
CGGCCTGCTTTATGGACTCATAAATTCAGATTCCCAGCCTCATAGGAGTACTGATAGCAAATGGTATATTAGCCTTGAGG
AATGGCCTGAAGAGAAATATCCGCCATGGGCACATGGTCCTGGTTATGTGGTGTCTCAAGACATAGC

>Lettuce (Lactuca sativa)

MKKWYIYVLSASLLTLTILGYLIIQNPIREAYISSPPYFNTTNPLEWINSSSPPVQNQTPNPQIVSTDEIVSTLFIHRNI

SFNEQQPLHTWNHLRHLISHDRVLPNTVEAIKDGGIAWNNLMTTVEDDKRDMNRSSGGKKKEKQCPHFLNKMNATELNDT

ATGAAGAAATGGTATATCTATGTCCTATCTGCATCTCTATTAACATTAACAATACTCGGGTACTTAATCATTCAAAATCC

AATAAGAGAAGCTTACATTTCATCACCTCCATACTTCAACACAACAAACCCTCTTGAATGGATAAATTCCAGTTCCCCCC

CTGTTCAAAACCAAACCCCCAACCCTCAAATTGTCTCCACTGATGAAATAGTCTCCACCCTCTTTATCCACAGAAACATC

TCATTCAATGAACAACAACCATTACACACATGGAACCATTTAAGACACTTAATAAGCCATGATCGTGTTCTTCCTAACAC

AGTTGAAGCAATCAAAGATGGTGGAATCGCATGGAATAACCTCATGACTACAGTTGAAGATGACAAACGTGACATGAATA

GAAGCTCAGGGGGGAAAAAGAAAGAGAAACAATGTCCCCATTTTCTTAACAAGATGAATGCCACAGAGCTTAATGATACT

>Tomato

TMLCVFHETLEPWLISEVRISGDIKLISVVASGLPTSEDSEHISDLEALKAAPLPPRKRLDLFIGVFSTANNFKRRMAVR

RTWMQYDAVRSGQVAVRFFVGLHKNQMVNEELWNEARTYRDIQLMPFVDYYSLITWKTIAICVFGTEVVSAKFVMKTDDD

AFVRVDEILSSMERVNTTRGLLYGLINADSQPYRSPDSQWFISPEEWSEET

ACGATGTTATGTGTCTTCCATGAAACTTTGGAACCATGGCTCATAAGTGAAGTGAGGATATCGGGAGACA

TAAAATTAATTTCTGTTGTCGCAAGTGGTTTACCAACATCAGAGGATTCAGAGCATATAAGCGACTTGGA

AGCCTTAAAAGCAGCTCCTCTTCCTCCACGAAAAAGACTAGATCTCTTTATTGGTGTATTTTCTACAGCA

AATAATTTTAAGCGTAGAATGGCTGTCCGTAGAACTTGGATGCAATATGATGCAGTGCGGTCTGGACAAG

TTGCGGTGCGGTTTTTTGTTGGCTTGCATAAAAACCAAATGGTGAATGAAGAGCTCTGGAATGAGGCTCG

GACATACAGAGACATCCAGCTGATGCCTTTTGTTGATTACTACAGTCTTATCACTTGGAAAACCATTGCC

ATCTGTGTTTTTGGGACGGAGGTTGTTTCTGCAAAGTTTGTCATGAAGACAGATGATGATGCATTTGTTC

GAGTGGATGAAATCTTATCTTCTATGGAGAGGGTTAACACGACTCGTGGATTGTTATATGGACTTATTAA

CGCAGATTCCCAACCTTACAGGAGTCCAGACAGCCAGTGGTTTATCAGTCCAGAGGAATGGTCTGAAGAA

ACTTA

>Grapewine (Vitis vinifera)

GAVAVRFFVGLHKNQMVNEELWKEVQTYGDIQLMPFVDYYSLITWKTIAICIFGTEAVSAKYVMKTDDDSFVRVDEVLAS

LKKTKVTHGLLYGLINSDARPHRSSDSKWYISPEEWAGETYPPWAHGPGYVVSNDIAKTVYKRHKEGHLKMFKLEDVAMG

IWIAEMKKGGMEVSYIKEERVYNEGCNDGYVV

GGGGCAGTTGCCGTGCGCTTTTTTGTTGGTCTGCATAAAAATCAGATGGTGAATGAGGAACTCTGGAA

GGAGGTGCAGACATATGGAGACATTCAGCTGATGCCTTTTGTTGACTATTACAGTCTTATAACCTGGAAG

ACCATAGCAATTTGCATCTTTGGGACTGAGGCTGTTTCGGCCAAGTATGTGATGAAAACAGATGATGATT

CATTTGTTCGTGTGGATGAAGTGCTTGCTTCTTTAAAGAAGACCAAAGTGACTCATGGGTTGCTGTATGG

TCTTATTAACTCTGATGCTCGACCTCATCGTAGTTCTGACAGTAAATGGTACATCAGCCCTGAGGAATGG

GCAGGAGAAACTTATCCACCTTGGGCACATGGTCCTGGTTACGTGGTGTCAAATGACATAGCAAAAACAG

TGTACAAGAGACACAAGGAAGGTCATTTAAAGATGTTCAAGCTGGAAGATGTAGCAATGGGGATCTGGAT

AGCAGAGATGAAGAAGGGGGGAATGGAGGTTAGTTACATAAAAGAAGAGAGGGTCTATAATGAGGGGTGC
AACGATGGGTACGTTGTTG

>Wheat
AEPKKYYPFKQGNLAIAILRVGAEGIHMTVDGKHVTSFAFREDLEPGFVGEVRIEGDIKLLSVLASGLPTTEDFEHVTDL
EILKAPPVPKDKSIDLFIGIFSTANNFKRRMAVRRTWMQYDAVRSGKVAVRFFVGLHKNDVVNKELWNEARTYGDIQLMP
FVDYYSLILWKTIAICIYGTNVLSAKYVMKTDDDAFVRVDEILSSLHQVNIXHGLLYGRVNSDSQPHRDPY

GCAGAACCTAAAAAATATTATCCCTTCAAACAAGGAAATCTTGCTATAGCAATTCTTCGTGTTGGAGCAGAGGGGATCCA
TATGACTGTAGATGGGAAACATGTCACTTCCTTTGCATTTCGAGAGGATTTGGAGCCTGGGTTTGTTGGTGAAGTAAGGA
TTGAAGGAGATATTAAATTGCTGTCTGTGCTAGCAAGTGGCTTGCCTACAACAGAGGACTTTGAGCATGTCACTGACTTG
GAAATATTGAAGGCTCCACCTGTCCCTAAGGATAAATCCATTGATCTTTTTATTGGGATATTCTCTACAGCAAATAATTT
TAAACGCAGAATGGCGGTTCGAAGAACCTGGATGCAGTACGATGCTGTCCGCTCAGGAAAAGTCGCTGTTCGGTTCTTTG
TTGGCCTGCATAAAAATGATGTGGTGAACAAGGAGCTCTGGAACGAAGCACGGACATATGGAGACATCCAATTGATGCCA
TTTGTTGATTATTACAGCTTGATTCTTTGGAAGACCATTGCCATTTGCATATATGGAACAAATGTACTTTCAGCCAAGTA
TGTTATGAAAACCGACGATGACGCTTTCGTTCGTGTAGATGAGATACTTTCGTCCCTACATCAAGTAAATATCANCCATG
GACTGCTGTATGGTCGCGTCAATTCTGATTCTCAGCCTCACCGAGATCCGTAT

>Potato
NNQVIPAEIVVSDLFIKRNLSAQEQQSLSTWYQLKRLITHDLVLPNAIEAVKEATVAWNN
LMSAVEMDKLDTNDSSIKTGKQKQCPHFLSKTNATELDASGFKLRLPCGLTQGSSITIIG
IPNGLLGNFRIDLTGEPLPGEPDPPVILHYNVRLHGDKITEDPVIVQNTWTIAHDWGEKE
RCPLPSDEKSKKV

AATAATCAAGTAATACCTGCTGAAATTGTTGTCTCTGATCTCTTTATCAAGAGGAACCTGTCAGCTCA
GGAGCAACAATCTTTGTCTACGTGGTATCAATTGAAAAGGTTAATCACTCATGATCTAGTTTTACCTAAT
GCTATAGAGGCGGTTAAGGAAGCCACTGTTGCATGGAACAACCTCATGAGTGCAGTAGAGATGGACAAAC
TCGATACAAATGATAGTTCAATTAAGACAGGGAAACAGAAACAATGTCCTCATTTTCTTAGCAAAACCAA
TGCTACAGAACTTGATGCTAGCGGCTTTAAGTTGCGGCTTCCTTGTGGTCTGACTCAGGGTTCTTCCATC
ACAATAATTGGCATTCCAAATGGTCTTCTTGGGAATTTTCGGATAGATTTGACTGGTGAACCACTACCAG
GTGAACCAGACCCTCCTGTTATTCTGCACTACAATGTTAGGCTCCATGGTGATAAAATAACCGAGGATCC
TGTAATCGTACAAAACACCTGGACTATTGCACATGACTGGGGTGAAAAGGAGCGCTGTCCATTACCATCC
GATGAAAAGAGTAAGAAAGTGG

>Medicago

TLEPWLVSEVRIAGDLKLLSILASGLPTSEESEHVVDLEALKAPPLSPLRPLDLVIGVFSTANNFKR-
RMAVRRTWMQYDDVRSGRVAVRFFVGLHKSPIVNLELWNEARTYGDVQLMPFVDYYSLISWKTLAICIFG

ACACTGGAGCCATGGCTTGTTAGTGAAGTACGGATTGCAGGTGATTTGAAGTTACTATCCATTCTCGCTA
GCGGTTTACCCACATCAGAAGAATCAGAGCATGTTGTTGATCTAGAGGCACTGAAGGCACCTCCCCTTTC
ACCATTAAGGCCATTAGATCTTGTCATTGGCGTTTTCTCCACAGCAAACAATTTTAAACGACGGATGGCT
GTGAGAAGAACTTGGATGCAGTATGATGATGTAAGATCTGGAAGGGTTGCAGTACGCTTTTTTGTTGGCC
TTCACAAGAGTCCTATTGTTAACTTGGAACTATGGAACGAGGCTCGGACTTACGGTGACGTTCAGCTAAT
GCCCTTTGTTGATTACTACAGTCTCATCAGTTGGAAAACACTAGCAATCTGCATCTTCGGG

## Claims

1. The method of expressing a plant Lewis-type β1,3-galactosyltransferase in an organism comprising the step of providing the organism with a nucleic acid molecule comprising a sequence A which is defined as being selected from
a) a sequence according to SEQ ID NO: 1 with an open reading frame from base pair 1-1932,
f) a sequence which is at least 50% identical with SEQ ID NO: 1,
g) a sequence which hybridizes with SEQ ID NO: 1 under stringent conditions, or
h) a sequence which has degenerated to SEQ ID NO: 1 due to the genetic code,
wherein the sequences a) to d) encode a plant protein having Lewis-type β1,3-galactosyltransferase activity, or
i) a sequence which is complementary to one of the sequences a) to d),
and expressing a protein encoded by sequence A or if the organism comprises a sequence A in its wildtype form overexpressing a protein encoded by sequence A.

2. The method of claim 1, **characterized in that** the protein has a sequence of SEQ ID NO: 2 or is at least 50% identical with SEQ ID NO: 2.

3. The method of claim 1 or 2, **characterized in that** the protein comprises a galactosyltransferase domain according to SEQ ID NO: 4, or a galactosyltransferase domain which is at least 50% identical with SEQ ID NO: 4.

4. The method of any one of claims 1 to 3, **characterized in that** expression and/or overexpression is facilitated by transfecting the organism with a nucleic acid comprising a sequence A or by upregulation of the expression of an endogenous sequence A.

5. The method of any one of claims 1 to 4, **characterized in that** the nucleic acid molecule is a functional vector.

6. Use of a nucleic acid molecule comprising sequence A as defined in claims 1 to 3 for expressing a plant Lewis-type β1,3-galactosyltransferase.

7. A nucleic acid molecule comprising a sequence derived from a sequence A further comprising a disruption, deletion, insertion and/or substitution mutation, preferably in the galactosyltransferase domain as defined in claim 3, encoding an inactive Lewis-type β1,3-galactosyltransferase.

8. Nucleic acid molecule of claim 7 which hybridizes to sequence A, preferably to SEQ ID NO: 1, under stringent conditions.

9. Nucleic acid molecule of claim 7 or 8 comprising a disrupted Lewis-type β1,3-galactosyltransferase gene, wherein the disruption is by insertion of an exogenous sequence into said gene such that the disruption prevents expression of functional β1,3-galactosyltransferase, wherein the gene, prior to disruption, codes for a plant Lewis-type β1,3-galactosyltransferase with an amino acid sequence of SEQ ID NO: 2 or with at least 50% identity to SEQ ID NO: 2.

10. A biologically functional vector comprising a nucleic acid molecule of any one of claims 7 to 9 suitable to transfect an organism, preferably suitable to transfect a plant or a plant cell.

11. A biologically functional vector comprising a DNA molecule as defined in any one of claims 1 to 3, wherein the sequence is inversely oriented with respect to the promoter.

12. A double-stranded siRNA molecule or microRNA, or artificial microRNAs, comprising a base sequence of 10 to 100 bases, preferably 15 to 40 bases, more preferred 18 to 30 bases or a double stranded hairpin-RNA comprising a base sequence of 10 to 1000, preferably 50-500, especially 100-300, bases of SEQ ID NO: 1 or sequence A being complementary to SEQ ID NO: 1 or sequence A.

13. The method of reducing or preventing the expression of an endogenous plant Lewis-type β1,3-galactosyltransferase in an organism comprising the step of at least transiently transfecting the organism with a nucleic acid molecule of claims 7 to 9 or a vector of claims 10 or 11 or treating the organism with a RNA of claim 12 or transfecting the organism with a nucleic acid molecule expressing the RNA of claim 12 or a nucleic acid to any sequence A.

14. The method of any one of claims 1 to 4 or 13, **characterized in that** the organism is a plant or a plant cell.

15. The method of producing a plant or plant cells having blocked or reduced expression of an endogenous Lewis-type β1,3-galactosyltransferase present in the wildtype plant or plant cell at the transcription or translation level, **characterized in that** the plant or plant cell is stably or transiently transfected by a method of claim 13, wherein the reduction is preferably by at least 10% as compared to the wildtype plant or plant cell.

16. The method of claim 15, **characterized in that** the plant or plant cell is capable of producing complex N-glycans after the transfection and an expression of a Lewis-A epitope is prevented by the transfection.

17. The method of claim 15 or 16, **characterized in that** a normal expression of the Lewis-A epitope is prevented in at least one organ or tissue, and the plant or plant cell comprises a dysfunctional β1,2-xylosyltransferase and/or a dysfunctional α1,3-fucosyltransferase.

18. The method of any one of claims 13 to 15, **characterized in that** the plant or plant cell, respectively produce glycoproteins, which lack β1,2-xylose and α1,3-fucose linked to complex N-glycans.

19. A transformed plant or plant cell with an increased expression of a Lewis-type β1,3-galactosyltransferase, comprising a nucleic acid molecule as defined in any one of claims 1 to 4.

20. A transformed plant or plant cell with a reduced or no expression of a Lewis-type β1,3-galactosyltransferase, comprising a nucleic acid molecule of claims 7 to 9 or a vector of claims 10 or 11 or a RNA of claim 12 or a nucleic acid expressing the RNA of claim 12 or an antisense nucleic acid to any sequence A.

21. A transformed plant or plant cell of claim 19 or 20 comprising a nucleotide sequence linked to an exogenous promoter that drives expression in said plant or plant cell, respectively, wherein said nucleotide sequence encodes a functional mammalian galactosyltransferase that is expressed in the plant or plant cell, respectively.

22. A transformed plant or plant cell according to claim 21, **characterized in that** the mammalian galactosyltransferase is a β 1,4-galactosyltransferase, preferably a human β1,4-galactosyltransferase.

23. A transformed plant or plant cell according to any one of claims 19 to 22 comprising a nucleotide sequence linked to an exogenous promoter that drives expression in said plant or plant cell, respectively, wherein said nucleotide sequence encodes a functional mammalian N-acetylglucosaminyltransferase that is expressed in the plant or plant cell, respectively.

24. A transformed plant or plant cell according to claim 23, **characterized in that** the mammalian N-acetylglucosaminyltransferase is a β1,2-N-acetylglucosaminyltransferase I or II, preferably a human β1,2-N-acetylglucosaminyltransferase I or II, or a β 1,4-N-acetylglucosaminyltransferase III or IV, preferably a human β1,4-N-acetylglucosaminyltransferase III or IV, or a β1,6-N-acetylglucosaminyltransferase V, preferably a human β1,6-N-acetylglucosaminyltransferase V.

25. A transformed plant or plant cell according to any one of claims 19 to 24 comprising a nucleotide sequence linked to an exogenous promoter that drives expression in said plant or plant cell, respectively, wherein said nucleotide sequence encodes a functional mammalian sialyltransferase that is expressed in the plant or plant cell, respectively.

26. A transformed plant or plant cell according to claim 25, **characterized in that** the mammalian sialyltransferase is a human or rat α2,6-sialyltransferase or a human or rat α2,3-sialyltransferase.

27. A transformed plant or plant cell according to claims 19 to 26 selected from the group of alfaalfa, Arabidopsis thaliana, maize, mung bean, potato, rice, soybean, tobacco, tomato plant, wheat, barley, Nicotiana benthamiana, lemna, lettuce, spinach, banana, sugarcane, poples, apple, cotton, common bean, leafy spurge, carrot, cowpea, papaya, Brassica and grapewine.

28. A method of producing recombinant mammalian glycoproteins or glycosylated polypeptides, **characterized in that** a transformed plant according to any one of claims 19 to 26 expresses the glycoprotein or polypeptide and a plant is transfected with a nucleic acid that encodes the glycoprotein or polypeptide.

29. The method of claim 28, **characterized in that** the glycoprotein is a human antibody or a fragment thereof, preferably a human immunoglobulin G (IgG) or a glycosylated fragment thereof.

30. The method of claim 28, **characterized in that** the glycoprotein or glycosylated polypeptide is selected from the group consisting of human insulin, preproinsulin, proinsulin, glucagon, interferons such as alpha-interferon, beta-interferon, gamma-interferon, blood-clotting factors selected from Factor VII, VIII, IX, X, XI, and XII, fertility hormones including luteinising hormone, follicle stimulating hormone growth factors including epidermal growth factor, platelet-derived growth factor, granulocyte colony stimulating, prolactin, oxytocin, thyroid stimulating hormone, adrenocorticotropic hormone, calcitonin, parathyroid hormone, somatostatin, erythropoietin (EPO), haemoglobin, serum albumin, collagen and enzymes such as beta-glucocerebrosidase and human and non-human proteins selected from amidases, amylases, carbohydrases, cellulase, dextranase, esterases, glucanases, glucoamylase, lactase, lipases, pepsin, peptidases, phytases, proteases, pectinases, casein, whey proteins, soya proteins, gluten and egg albumin.

31. A method of producing at least a plant or a plant cell wherein the plant Lewis-type β1,3-galactosyltransferase activity is increased that comprises introducing into the plant or plant cell a nucleic acid sequence encoding a plant Lewis-type β1,3-galactosyltransferase nucleotide sequence, mammalian β1,3-galactosyltransferase nucleotide sequence, or bryophyte Lewis-type β 1,3-galactosyltransferase nucleotide sequence.

32. A transformed plant or plant cell with increased expression of the Lewis-A epitope, **characterized in that** the plant or plant cell comprises a mammalian β1,3-galactosyltransferase nucleotide sequence, preferably selected from β1,3-galactosyltransferase 1, β1,3-galactosyltransferase 2 or β1,3-galactosyltransferase 5, a bryophyte Lewis-type β1,3-galactosyltransferase nucleotide sequence or a plant Lewis-type β1,3-galactosyltransferase nucleotide sequence.
